# EUROPEAN PATENT SPECIFICATION

(11) **EP 2 444 481 B1**
(45) Date of publication and mention of the grant of the patent: **28.11.2018**
(21) Application number: 11185852.8
(22) Date of filing: 19.10.2011
(51) Int. Cl.: C12N 1/20, C12N 1/28

(54) **Microorganism producing O-phosphoserine and method of producing L-cysteine or derivatives thereof from O-phosphoserine using the same**
O-phosphoserin-herstellender Mikroorganismus und Verfahren zur Herstellung von L-cystein oder Derivaten davon aus O-phosphoserin damit
Micro-organisme de production d'O-phosphosérine et procédé de production de L-cystéine ou dérivés correspondants d'O-phosphosérine l'utilisant

(30) Priority: 20.10.2010 KR 20100102664; 26.08.2011 KR 20110086081
(43) Date of publication of application: 25.04.2012
(73) Proprietor: CJ CheilJedang Corporation, Seoul, 100-400 (KR)
(72) Inventor: Shin, Soo An, Seoul 100-400 (KR); Shin, Yong Uk, Seoul 100-400 (KR); Kim, Hye Won, Seoul 100-400 (KR); Kim, Sol, Seoul 100-400 (KR); Um, Hye Won, Seoul 100-400 (KR); Chang, Jin Sook, Seoul 100-400 (KR); Bae, Hyun Ae, Seoul 100-400 (KR); Jhon, Sung Hoo, Seoul 100-400 (KR); Jo, Jae Hyun, Seoul 100-400 (KR); Song, Byeong Cheol, Seoul 100-400 (KR); Lee, Kyoung Min, Seoul 100-400 (KR); Yang, Eun Bin, Seoul 100-400 (KR)
(74) Representative: Vossius & Partner Patentanwälte Rechtsanwälte mbB

(56) References cited:
- WO-A2-2006/138689
- DE-A1- 19 539 952
- GARNANT MK ET AL.: "Construction and analysis of plasmids containing the Escherichia coli serB gene", MOLECULAR AND GENERAL GENETICS, vol. 193, no. 1, 1984, - 1984, XP002669260,

## Description

### [Technical Field]

The present invention relates to a method for production of cysteine or its derivatives N-acetylcysteine or S-carboxymethylcysteine using O-phosphoserine as an intermediate and recombinant microorganism for use in production of O-phosphoserine.

### [Background Art]

L-cysteine is an amino acid that plays an important role in sulfur metabolism of all living organisms. It is used in the biosynthesis of proteins, such as hair keratin, glutathione, biotin, methionine and other sulfur-containing metabolites as well as serving as a precursor of coenzyme A. In addition, the biosynthesis of cysteine is known to be closely associated with the biosynthesis of other amino acids including L-serine, L-glycine, and L-methionine. Industrially, L-cysteine and its derivatives find applications in a variety of fields including the pharmaceutical industry (for treatment of bronchial diseases), the cosmetics industry (in hair shampoo, compositions for permanent waves), and the food industry (antioxidants, flavorant enhancers, dough aids, etc.).

L-cysteine was once obtained industrially by acid hydrolysis of human hairs or animal feathers (Biotechnology of the Amino Acids Production edited by Ko Aida, p 217-223, 1986). However, not only does the production of cysteine from hairs or feathers ensure a yield of as low as 7∼8 %, but also the use of hydrochloric acid or sulfuric acid produces a lot of waste resulting in environmental pollution. Further, extraction from hairs or feathers may induce the user to have a strong adversion thereto. These problems have caused a push for the development of environmentally friendly production processes of L-cysteine. The main contemporary route involves fermentation utilizing microorganisms.

Representative among the microbial production of L-cysteine is 1) the biological conversion of D, L-ATC using a microorganism (Ryu OH, Ju JY and Shin CS, Process Biochem., 32:201-209, 1997). This conversion process is, however, difficult to apply industrialy due to the low solubility of the precursor D, L-ATC. 2) Another method of L-cysteine production is direct fermentation using *E. coli* (Patent No. EP0885962B; Wada M and Takagi H, Appl. Microbiol. Biochem., 73:48-54, 2006). Excessive accumulation of L-cysteine within microorganisms incurs intracellular toxicity, exhibiting a limitation in the production of L-cysteine at a high concentration. To overcome this drawback, L-cysteine-exporting proteins are employed, but there have been no significant improvements in productivity.

Referring to the biosynthesis pathway of L-cysteine in microorganisms and plants, O-acetyl-serine (OAS) acts as an intermediate precursor providing the carbon backbone of L-cysteine (Kredich NM and Tomkins GM, J. Biol. Chem., 241: 4955-4965, 1966). The enzyme O-acetylserine sulfhydrylase (OASS), using hydrogen sulfide as a sulfur donor, catalyses the conversion of O-acetylserine to cysteine. Alternatively, SO₄ may be reduced to thiosulfate for use as a sulfur donor in cysteine production (Nakamura T, Kon Y, Iwahashi H and Eguchi Y, J. Bacteriol., 156: 656-662, 1983). Therefore, cystein may be produced using microorganisms accumulating OAS and OASS using various sulfur donors (US6, 579, 705). The cysteine biosynthesis pathway via OAS uses the two enzymes of serine acetyltransferase (CysE), which catalyzes the conversion of OAS from serine, and cysteine synthase (CysK), which catalyzes the conversion of OAS to cysteine. Among them, serine acetyltransferase (CysE) is highly sensitive to feedback inhibition by the final product cysteine (Wada M and Takagi H, Appl. Microbiol. Biochem., 73:48-54, 2006).

### [Disclosure]

### [Technical Problem]

Leading to the present invention, the present inventors found out the existence of O-phosphoserine sulfhydrylase (OPSS) in *Aeropyrum pernix, Mycobacterium tuberculosis*, and *Trichomonas vaginalis* that takes an O-phospho-L-serine (OPS)-specific pathway, rather than the OAS-specific pathway, to synthesize L-cysteine through intensive research (Mino K and Ishikawa K, FEBS letters, 551: 133-138, 2003; Burns KE, Baumgart S, Dorrestein PC, Zhai H, McLafferty FW and Begley TP, J. Am. Chem. Soc., 127: 11602-11603, 2005; Westrop GD, Goodall G, Mottram JC and Coombs GH, J. Biol. Chem., 281: 25062-25075, 2006) and that the OPSS of *M. tuberculosis,* can use Na₂S as a sulfur donor in converting OPS to cysteine even in the absence of the additional enzymes when five C-terminal amino acid residues are removed therefrom (Argen D, Schnell R and Schneider G, FEBS letters, 583: 330-336, 2009). In the present invention, a microorganism is mutated to accumulate OPS therein, following incubation to convert OPS into cysteine in the presence of the OPSS enzyme. Nowhere has this method been previously described.

### [Technical Solution]

It is an object of the present invention to provide a method for producing cysteine or a derivative thereof, i.e., N-acetylcysteine or S-carboxymethylcysteine.

It is another object of the present invention to provide a recombinant microorganism for the production of O-phosphoserine.

### [Advantageous Effects]

The method of the present invention in which O-phosphoserine is produced at high yield by a recombinant microorganism and is used for conversion into cysteine, as it is, is more friendly to the environment and ensures higher efficiency in the production of cysteine than do chemical synthesis methods. The cysteine and its derivatives produced by the fermentation and bioconversion of the present invention can be widely used in the production of animal and human foods and food additives.

### [Description of Drawings]

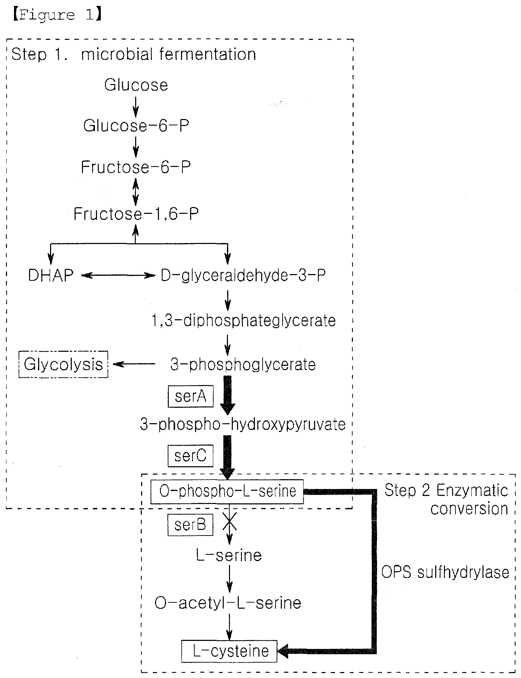

FIG. 1 is a schematic diagram showing the accumulation of O-phosphoserine by microbial fermentation and the enzymatic conversion of the accumulated O-phosphoserine into L-cysteine.
FIG. 2 is a graph showing the activity of OPS sulfhydrylase according to temperatures.
FIG. 3 is a set of graphs showing pH sensitivity of OPS sulfhydrylase.
FIG. 4 is a photograph showing the expression level of Msm-T in a pET system and a pCL-Pcjl system as analyzed by SDS PAGE.
FIG. 5 is a graph showing the enzymatic activity of OPS sulfhydrylase to convert purified OPS fermentation broth into cysteine.
FIG. 6 is a graph showing the enzymatic activity of OPS sulfhydrylase to convert OPS fermentation broth into cysteine.

### [Best Mode]

As used herein, the term "cysteine conversion" is intended to refer to the catalytic reaction of O-phosphoserine sulfhydrylase (OPSS) which results in the conversion of the substrate O-phosphoserine. (OPS) into the product cysteine, that is, it refers to the catalytic reaction of converting OPS into cyteine.

As used herein, the term "cysteine conversion rate" refers to the percentage of the amount of the product cysteine to the amount of the starting material OPS. Under optimal reaction conditions, 1 mole of OPS is conveted into 1 mole of cysteine. For example, if 100 moles of OPS is converted into 100 moles of cysteine, the cysteine conversion rate is 100 %.

In accordance with an aspect thereof, the present invention provides a method for producing cysteine , comprising:
1) culturing a recombinant microorganism which is modified to have decreased endogeneous phosphorerine phosphatase (SerB) activity to produce O-sphosphoserine (OPS); and 2) reacting the OPS of step 1) with a sulfide in the presence of O-phosphoserine sulfhydrylase (OPSS) or with a sulfide in presence of a microorganism expressing OPSS, to produce cysteine,
   wherein the level of SerB enzyme activity is reduced by using a technique selected from the group consisting of deletion of the chromosomal gene encoding the SerB enzyme, the introduction of mutation into the chromosomal gene encoding the SerB enzyme to reduce the endogenous gene activity, the substitution of the chromosomal gene encoding the SerB enzyme with a gene mutated to reduce the endogenous enzyme activity, the introduction of mutation into a regulatory region for the gene encoding the SerB enzyme to reduce endogenous gene activity, and the introduction of an antisense oligonucleotide complementary to a transcript of the gene encoding the SerB enzyme to inhibit the translation of the mRNA.

Moreover, in another aspect, the present invention provides a method for producing a cysteine derivative N-acetylcysteine or S-carboxymethylcysteine, comprising:
1) culturing a recombinant microorganism in which the activity of endogenous phosphoserine phosphatase (SerB) is reduced, to produce O-phosphoserine (OPS);
2) reacting the OPS of step 1) with a sulfide in presence of O-phosphoserine sulfhydrylase (OPSS) or with a sulfide in presence of a microorganism expressing OPSS, to produce cysteine, and
3) synthesizing the cysteine derivative N-acetylcysteine or S-carboxymethylcysteine from the cysteine produced in step 2), wherein the level of SerB enzyme activity is reduced by using a technique selected from the group consisting of deletion of the chromosomal gene encoding the SerB enzyme, the introduction of mutation into the chromosomal gene encoding the SerB enzyme to reduce the endogenous gene activity, the substitution of the chromosomal gene encoding the SerB enzyme with a gene mutated to reduce the endogenous enzyme activity, the introduction of mutation into a regulatory region for the gene encoding the SerB enzyme to reduce endogenous gene activity, and the introduction of an antisense oligonucleotide complementary to a transcript of the gene encoding the SerB enzyme to inhibit the translation of the mRNA.

In the method, step 1) is related to culturing a recombinant microorganism which is the activity of endogenous phosphoserine phosphatase (SerB) is reduced.

The SerB is a protein that has the activity of hydrolyzing OPS into L-serine. Thus, a microorganism which has reduced endogeneous SerBactivity is characterized by the accumulation of OPS therein. The SerB is not limited to, may comprise any amino acid sequences, which exhibits SerB activity, and may have preferably the amino acid sequence of SEQ ID NO: 1 or 2. However, as long as it exhibits SerB activity, any amino acid sequence is used, which preferably has a homology of 90% or higher, more preferaby 96% or higher, far more preferably 98% or higher, and most preferably 99% or higher with that of SEQ ID NO: 1 or 2. The reduced SerB activity means a decrease in SerB activity, compared to that of a prior-modified strain, and encompasses the disrupting of SerB. Various techniques for reduction of SerB activity are well known in the art. Illustrative examples include the deletion of a chromosomal serB, the introduction of mutation into the chromosomal *serB* to reduce endogenouse SerB activity, the introduction of mutation into a regulatory region for the serB to reduce endogenouse SerB activity, the substitution of the chromosomal *serB* with a gene mutated to reduce the endogenouse SerB activity and the introduction of an antisense oligonucleotide complementary to a transcript of the *serB* to inhibit the translation of the mRNA, but methods for reducing the SerB activity are not limited to these. These techniques may be applied to the reducing the activity of other enzymes in the present invention.

The disruption of endogenous SerB results in the introduction of serine auxotrophy into the recombinant microorganism so that the medium must be supplemented with glycine or serine. Glycine may be provided in the form of purified glycine, a glycine-containing yeast extract, or tryptone. Glycine is contained at a concentration of from 0.1 to 10 g/L, and preferably at a concentration of from 0.5 to 3 g/L. As for serine, it may be provided in the form of purified serine, a serine-containing yeast extract or tryptone. Its concentration in the culture medium ranges from 0.1 to 5 g/L, and preferably from 0.1 to 1 g/L.

As disclosed herein, when cultured in a glycine- or serine-containing medium, mutant *Corynebacterium glutamicum* or *E*. *coli* in which the activity of endogeneous SerB was distrupted was found to produce a higher amount of OPS, compared to the wild-type (see Tables 2, 3, 6 and 7).

In addition, the recombinant microorganism of the present invention may have enhanced phosphoglycerate dehydrogenase (SerA) or phosphoserine aminotransferase (SerC) activity. The SerA is a protein that has the activity of converting 3-phosphoglycerate to 3-phosphohydroxypyruvate. The SerA may have wild-type amino acids or a mutant amino acid sequence which shows resistance to feedback inhibition by serine, but is not limited to these. Preferably, the SerA may have one selected from the group consisting of amino acid sequences of SEQ ID NOS: 3 to 7. So long as it exhibits wild-type SerA activity or the mutant SerA activity resistant to serine feedback inhibition, any amino acid sequence may be used, although it preferably shares a homology of 90% or higher, more preferaby 96% or higher, far more preferably 98% or higher, and most preferably 99% or higher with that of one of SEQ ID NO: 3 to 7. A "mutant SerA resitant to feedback inhibition" means the mutant showing a maintained or enhanced SerA activity irrespective of the feedback inhibition by serine or glycine. The feedback-resistant mutants are well known in the art (Grant GA et al., J. Biol. Chem., 39: 5357-5361, 1999; Grant GA et al., Biochem., 39: 7316-7319, 2000; Grant GA et al., J. Biol. Chem., 276: 17844-17850, 2001; Peters-Wendisch P et al., Appl. Microbiol. Biotechnol., 60: 437-441, 2002; EP0943687B). In one embodiment of the present invention, when a feedback-resistant *serA* was further introduced thereinto, *Corynebacterium glutamicum* or *E. coli* having a disrupted serB was found to produce a higher amount of OPS, as compared to the wild-type (see Tables 4 and 9).

The SerC is a protein that has the activity of converting 3-phosphohydroxypyruvate to O-phosphoserine. The SerC is not limited to, may comprise the sequences which exhibits SerC activity, and may have preferably the amino acid sequence of SEQ ID NO: 8. However, as long as it exhibits SerC activity, any amino acid sequence may be employed, but it should preferably share a homology of 90% or higher, more preferaby 96% or higher, far more preferably 98% or higher, and most preferably 99% or higher with that of SEQ ID NO: 8. Furthermore, a mutation may be introduced into the *serC* so that the enzyme activity can be increased. In one embodiment of the present invention, when an *serC* was further introduced thereinto, *Corynebacterium glutamicum* or *E. coli* having a disrupted *serB* and a feeback-resistant *serA*was found to produce a higher amount of OPS, compared to the wild-type (see Table 9).

Further, the capacity of the recombinant microorganism of the present invention to perform the intracellular uptake of or the degradation of O-phosphoserine may be decreased. In detail, the recombinant microorganism may be modified to reduce the activity of PhnC/PhnD/PhnE alkylphosphonate ABC transporter (PhnCDE operon, that is, ATP-binding component of phosphonate transport (PhnC; EG 10713)-periplasmic binding protein component of Pn transporter (PhnD; EG 10714)-integral membrane component of the alkylphosphonate ABC transporter (PhnE; EG 11283)), alkaline phosphatase (PhoA) or acid phosphatase (AphA).

In one embodiment of the present invention, the further deletion of the phnCDE operon from the recombinant mutant was observed to lead to an increase in OPS production (Table 10). In the recombinant microorganism which was further disrupted of PhoA or AphA activity, OPS degradation started to decrease at the time when the concentration of phosphoric acid in the culture medium is decreased (Table 12). Moreover, the introduction of a feedback-resistant *serA* or a *serC* raised OPS production (Tables 14 to 16).

Also, the recombinant microorganism of the present invention may be further characterized by the enhancement of pyrimidine nucleotide transhydrogenase (PntAB; EC 1.6.1.1) activity. As described previously (Sauer U P et al., J Biol Chem. 20; 279(8):6613-9. Epub 2003), PntAB participates in NADPH metabolism to regulate intracelluar redox balance. In one embodiment of the present invention, the recombinant microorganism in which PntAB activity was further enhance by overexpression of *pntAB* was found to increase OPS production (Table 17).

Moreover, the recombinant microorganism of the present invention may be characterized by the enhancement of O-acetylserine/cysteine efflux permease (YfiK), homoserine/homoserine lactone efflux protein (RhtB; EG 11469) or threonine/homoserine lactone efflux protein (RhtC; EG11468). The YfiK is known as an exporter for exporting cysteine and OAS extracellularly (Franke I et al., J. Bacteriology, 185: 1161-1166, 2003) and RhtB is reported to act as an extracellular exporter of homoserine/homoserine lactone, a threonine precursor. Further, the RhtC is known as an exporter of threonine and homoserine. The enhancement of the activity of YfiK, RhtC and RhtB showed an increase in the growth and OPS production of the OPS accumulation strain (Table 18).

The enhancement of the enzyme activity may be achieved using various well-known methods, including, but not being limited to, increasing the copy number of a gene encoding an enzyme of interest, introducing a mutation into a regulatory region for the gene to enhance the enzyme activity, substituting the chromosomal gene with a gene mutated to enhance the enzyme activity, and intoroducing a mutation into the chromosomal gene to enhance the enzyme activity.

In addition, the recombinant microorganism of the present invention is further characterized by the reduced activity of phosphoglycerate mutase. The phosphoglycerate mutase exists as three isozymes: GpmI, GpmA and GpmB and is responsible for the conversion of 3-phosphoglycerate into 2-phosphoglycerate in the glycolysis process. For the use of 3-phosphoglycerate as a substrate, these enzymes are in competition with SerA that catalyzes the synthesis of 3-phosphohydroxypyruvate. Therefore, the decreasing activity of each of the enzymes was observed to cause an abundance of 3-phosphoglycerate, a precursor of OPS, resulting in the production of an increased level of OPS (Table 19).

In the recombinant microorganism of the present invention, L-serine dehydratase I (SdaA; EC 4.3.1.17) or 2-amino-3-ketobutyrate coenzyme A ligase (Kbl) may be also reduced. Thus, the recombinant microorganism is characterized by the OPS production maintained or increased even when it is cultured in a medium containing a low concentration of glycine or serine (Table 20).

Further, the recombinant microorganism of the present invention may be further characterized by the reduced activity of IclR. IclR is a transcription factor that functions to repress the expression of aceBAK, a glyoxylate bypass operon (L Gui et al., J. Bacteriol., Vol 178, No. 1, 321-324, 1996). When it was deleted, the production of OPS was observed to increase (Table 21).

Also, the recombinant microorganism of the present invention may be further characterized by the enhancement of an enzyme activity selected from the group consisting of i) acetyl-CoA synthetase (Acs), ii) acetic acid kinase (AckA)-phosphotransacetylase (Pta), iii) malate synthase G (GlcB), iv) malate dehydrogenase (MaeB), v) glutamate dehydrogenase (GdhA), vi) glyoxylate carboligase (Glc), vii) tartronate semialdehyde reductase 2 (GlxR), viii) glycerate kinase II (GlxK), and a combination thereof.

In a concrete embodiment of the present invention, when i) Acs (EC No.6.2.1.1; J Bacteriol. 1995 May; 177(10):2878-86) or pyruvate oxidase monomer (PoxB; EC 1.2.2.2) or ii) AckA and Pta (EC 2,3,1,8), all of which aim to effectively reuse accumulated acetate with the concomitant consumption of produced NADH, were further enhanced, the recombinant microorganism of the present invention was found to increase production of OPS (Table 22). Functioning to catalyze the synthesis of malate from glyoxylate and the conversion of malate into pyruvate, iii) GlcB (EC No.2.3.3.9) and iv) MaeB (EC 1.1.137) can weaken the TCA cycle and thus be used to increase the glucose comsumption and the production of O-phosphoserine (Table 23). According to one embodiment of the present invention, the enhancementof v) GdhA; (EC 1.4.1.2), which catalyzes the synthesis of glutamate, a substrate of SerC, from 2-oxoglutarate and NADPH, bestowed a much higher potential for producing OPS on the microorganism (Table 17). All of vi) Glc(EC 4.1.1.47), vii) GlxR(EC 1.1.1.60) and viii) GlxK(EC 2.7.1.31) are known to convert glycoxylate into 3-phosphoglycerate, that is, to increase the level of the substrate of phosphoglycerate dehydrogenase (Kim HJ et al., J. Bacteriol., 186(11), 3453-3460, 2004; Eva Cusa et al., J. Bacteriol., 181(24), 7479-7484, 1999; Chnag YY et al., J. Biol. Chem. 268(6): 3911-3919, 1993). The recombinant microorganism of the present invention, when further enhanced in the activity of Glc, GlxR and GlxK, was improved in sugar consumption and growth (Table 24).

The recombinant microorganism of the present invention refers to any microorganism in which there is the reduction of SerB activity, thus producing OPS at an elevated level. If this condition is satisfied, any microorganism, whether prokaryotic or eukaryotic, falls within the scope of the present invention. Representative among them are enterobacteria or coryneform bacteria. Examples of the microorganisms useful in the present invention include *Escherichia* sp., *Erwinia* sp., *Serratia* sp., *Providencia* sp., *Corynebacterium* sp., and *Brevibacterium* sp. Preferable are *Escherichia* sp. and *Corynebacterium* sp, with more preference given for *Escherichia* sp. and with the highest preference being for *E*. *coli.*

In an embodiment, the recombinant strain capable of producing OPS was named *E. coli* CA07-0012, and deposited with the Korean Culture Center of Microorganisms, located at 361-221, Hongje 1, Seodaemun, Seoul, Korea, on October. 12, 2011 under accession number KCCM11212P.

In addition, in an embodiment, the recombinant strain capable of producing OPS was named *E. coli* CA07-0022/pCL-prmf-serA*(G336V)-serC, and deposited with the Korean Culture Center of Microorganisms, located at 361-221, Hongje 1, Seodaemun, Seoul, Korea, on September. 28, 2010 under accession number KCCM11103P. Herein, the term "CA07-0022/pCL-prmf-serA*(G336V)-serC" is used interchangerably with CA07-0022 serA*(G336V)/pCL-prmf-serA*(G336V)-serC.

After the strain was cultured for 80 hours in a 1 L fermenter, O-phosphoserine was produced at a concentration of 19.5 g/L (Example 35).

As used herein, the term "culturing" is intended to mean growing microorganisms under artificially controlled conditions. A culturing procedure may be conducted using a suitable medium and culturing conditions well known in the art. Those skilled in the art can readily control the culturing procedure to correspond to the strains employed. For example, it may be performed in a batch type, in a continuous type, or in a fed-batch type, but is not limited thereto.

In addition, the culture medium contains a carbon source. Examples of the carbon source include saccharides and carbohydrates such as glucose, sucrose, lactose, fructose, maltose, starch and cellulose, oils and fats such as soybean oil, sunflower oil, castor oil and coconut oil, fatty acis such as palmitic acid, stearic acid and linoleic acid, alcohols such as glycerol and ethanol, and organic acids such as acetic acid. These carbon sources may be present solely or in combination in the culture medium. As a nitrogen source, an organic material such as peptone, yeast extract, meat juice, malt extract, corn steep liquor, soybean, and wheat protein, or an inorganic nitrogen compound such as urea, ammonium sulfate, ammonium chloride, ammonium phosphate, ammonium carbonate and ammonium nitrate may be contained in the culture medium. These nitrogen sources may be used solely or in combination. The medium may contain potassium dihydrogen phosphate, potassium phosphate, or corresponding sodium salts as a phosphorous source. The medium may contain metallic salts such as magnesium sulfate or iron sulfate. The culture medium may also contain amino acids, vitamins and suitable precursors. The nutrients may be added in a batch manner or a continous manner to the medium.

A compound such as ammonium hydroxide, potassium hydroxide, ammonia, phosphoric acid and sulfuric acid may be added in a suitable manner to the culture medium during culturing to adjust the pH of the culture. In addition, during culturing, an anti-foaming agent such as fatty acid polyglycol ester is used to suppress the formation of foam. Further, in order to maintain the culture medium in an aerobic condition, oxygen or oxygen-containing gas can be injected into the culture medium. For an anaerobic or microaerobic condition, nitrogen, hydrogen, or carbon dioxide is provided without aeration. The culture medium may be typically maintained at a temperature of from 27°C to 37°C and preferably at a temperature of from 30°C to 35°C. As for the culture period, it may be maintained until the product of interest is obtained in a desired amount, and preferably it ranges from 10 to 100 hours.

For further collection and recovery of the OPS produced during the culturing step from the culture medium, a suitable method well known in the art may be selected depending on the type of culture, be it a batch, continuous or fed-batch culture.

In the method of the present invention, step 2) addresses the reaction of the OPS of step 1) with a sulfide in the presence of O-phosphoserine sulfhydrylase (OPSS) or a microorganism expressing OPSS, to induce the conversion of O-phosphoserine into cysteine or its derivatives.

The sulfide may be provided in a liquid or gas form as well as in a solid form typically used in the art, because of the difference in pH, pressure and/or solubility. So long as it may be converted to a thiol group (SH), any sulfur compound such as sulfide (S²⁻) or thiosulfate (S₂O₃²⁻) may be used in the present invention. Preferably, Na₂S, NaSH, H₂S, (NH₄)₂S, NaSH and Na₂S₂O₃, all of which can provide a thiol group for OPS, may be used. In the reaction, one thiol group is supplied to one OPS molecule to afford one molecule of cysteine or a derivative thereof. In this enzymatic conversion, a sulfide may be preferably added at a molar concentration 0.1 to 3 times and more preferably 1 to 2 times as high as that of OPS used. In light of the economy, a thiol group-providing sulfide and OPS are most preferably used at a molar ratio of 1:1. In one embodiment of the present invention, Na₂S was used as the source of sulfur. Na₂S was added at a molar concentration 1 to 3 times as high as that of OPS used in the conversion reaction. Preferably, it is fed at a molar concentration twice as high as that of OPS to effectively convert OPS into cysteine (Table 34).

As used herein, the term "O-phosphoserine sulfhydrylase (OPSS)" refers to an enzyme that catalyzes the transfer of a thiol group (SH) to OPS (O-phosphoserine) to convert OPS into cysteine. The enzyme was first found in *Aeropyrum pernix, Mycobacterium tuberculosis,* and *Trichomonas vaginalis* (Mino K and Ishikawa K, FEBS letters, 551: 133-138, 2003; Burns KE et al., J. Am. Chem. Soc., 127: 11602-11603, 2005).

As used herein, the term "mutant" refers to a culture or an individual that shows an inheritable or non-heritable alteration in phenotype. When used in conjunction with OPSS, the term "mutant" is intended to mean an OPSS enzyme which is genetically altered such that its activity can be effectively enhanced, compared to the wild-type.

In the present invention, the OPSS mutant can be constructed by deleting, substituting or adding a part of a nucleotide sequence encoding OPSS. According to one embodiment of the present invention, an OPSS enzyme with enhanced activity was prepared by deleting five C-terminal amino acid residues of the OPSS enzyme of *Mycobacterium smegmatis.*

The OPSS mutant can be obtained in *E. coli,* widely used for enzyme expression, using gene synthesis techniques based on codon optimization by which enzymes of interest can be obtained in high yield. Alternatively, screening methods of useful enzyme resources based on the bioinformatics of massive amounts of genetic information about microorganisms may be used to obtain the OPSS mutant. In one embodiment of the present invention, OPSS enzymes that utilize OPS as a substrate to synthesize cysteine were selected from various microbes by screening the homology of amino acid sequences. In this regard, cell pellets obtained using a medium and culture conditions that were suitable in the art were lyzed, followed by the purification of the supernatant containing the enzyme to afford the OPSS enzyme (Table 26).

In addition, a high-yield expression system was developed for obtaining the OPSS enzyme economically. A pET vector employing a T7 promoter is well known in the art. However, the present inventors developed an enzyme expression system, named the CJ1 system (Korean Patent 10-0620092 B1), instead of employing the typical system. In one embodiment of the present invention, the expression levels of OPSS between a pET system comprising a T7 promoter and the CJ1 system comprising a CJ1 promoter were compared given the same conditions. As a result, the CJ1 system showed a higher expression level of OPSS than the pET system. In addition, the overexpression of OPSS required a low temperature (18°C) and a long period of time in the pET system, but a high temperature (37°C) and a short period of time in the pCL-pCJ1 system. Preferably, the pCL-pCJ1 system is used to obtain OPSS (Example 46).

The enhancement of the enzyme activity may be achieved using various well-known methods. For example, it can be performed by increasing the number of copies of a gene encoding OPSS, using a strong promoter, or introducing a genetic mutation.

Optimization of the enzymatic conversion of OPSS may be achieved using various methods known in the art. For example, the optimization may be based on a full understanding of the characteristics of OPSS, such as the optimal temperature and pH, inhibition against substrates, substrate concentration, heat stability, etc. In addition, the optimization may be determined by optimal conditions for the enzymatic conversion, such as the optimal OPSS concentration, the optimal balances of the substrates used in terms of concentrations, a preference for sulfur compounds providing SH for the enzymatic conversion, a preference for certain buffers, the influence of ions generated, and cofactors and their optimal concentrations.

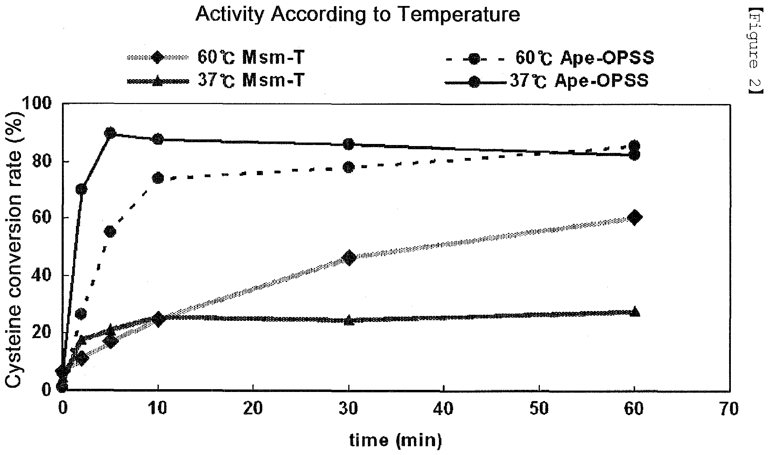

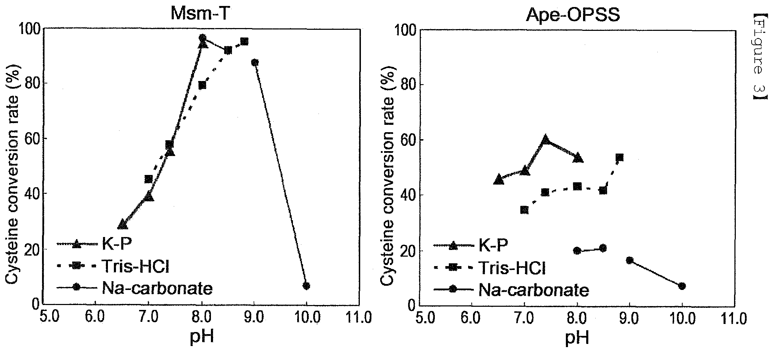

In one embodiment of the present invention, the OPSS enzyme obtained using the above-mentioned method was characterized and on the basis of the determined characteristics, an economically beneficial enzymatic conversion process that has a high conversion rate of cysteine from OPS, with the guarantee of enzyme stability, was developed. In the enzymatic conversion process, the reaction temperature can be set from 37°C to 80°C. In detail, Ape-OPSS (SEQ ID NO: 12), belonging to Archea, exhibits increased enzymatic activity at 60°C compared to 37°C, and the enzyme itself is highly stable to heat, with optimal reactivity at 60°C. On the other hand, Msm-T (SEQ ID NO: 10) exhibits optimal activity at 37°C and is relieved the activity to heat treatment at 60°C. The OPSS enzyme was observed to have enzymatic activity over a pH range of 6.0 to 10.0. Ape-OPSS showed optimal activity at pH 7.4. With the appearance of optimal activity at a pH of from 8.0 to 9.0, Msm-T showed stability over a wider pH range, compared to Ape-OPSS (Tables 28 and 31, and FIGS. 2 and 3).

As a cofactor, 0.001 - 2 mM PLP (pyridoxal-5'-phosphate) or 0.001 - 100 mM DTT may be used in the enzymatic conversion. In one embodiment of the present invention, the cysteine conversion rate was 2.3-fold increased in the presence of 25 mM DTT or 0.2 mM PLP. As such, treatment with DTT or PLP brought about an improvement in the cysteine conversion rate of step 2). The addition of the cofactor was set to a reasonable level in consideration of the increased production cost and the increased conversion rate (Table 30).

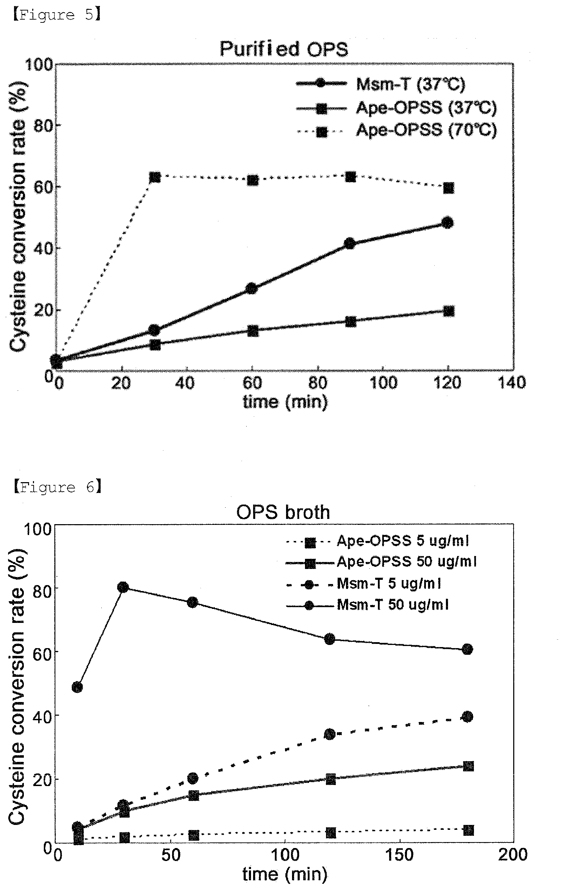

The reaction conditions for OPSS may vary depending on the kinds and concentration of the OPS used. In one embodiment of the present invention, pure OPS (commercially available), OPS purified from the culture prepared in step 1), and the OPS-containing culture of step 1) were used under various conditions to provide the optimal conversion rates. As a result, the cysteine conversion rate varied depending on the kind and concentration of OPSS and the reaction temperature and the kind and concentration of OPS (FIGS. 5 and 6, and Table 35).

The method of the present invention may further comprise isolating and purifying the cysteine produced in step 2). After the enzymatic conversion, cysteine can be isolated and purified from the culture medium using a method well known in the art.

Those skilled in the art may chemically synthesize cysteine derivatives from cysteine using a well known method. Cysteine may be readily reacted with an acetylation agent to give NAC (N-acetylcysteine) and with haloacetic acid under basic conditions to give SCMC (S-carboxymetylcysteine). These cysteine derivatives are used as materials in medicines that treat coughs, bronchitis, bronchial asthma, and sore throat.

In the present invention, the OPS broth obtained through microbial fermentation is used as a substrate for synthesizing cysteine. The OPS broth obtained by microbial fermentation has economical advantages over commercially available pure OPS in that the OPS broth can be used without having to be additionally purified and the cofactor PLP necessary for the conversion can be obtained from the fermented culture.

In one embodiment of the present invention, a conversion process was developed which ensures a cysteine conversion rate of as high as 80% when 50 µg/ml Msm-T was used under reaction conditions of a 50 mM OPS broth or a 60 mM purified OPS broth, 100 mM Na₂S or 120 mM Na₂S, and 0.2 mM PLP. It should be appreciated to those skilled in the art that the enzymatic conversion using highly active enzymes can easily be optimized and scaled up.

In accordance with another aspect thereof, the present invention provides a recombinant microorganism which is reduced the activity of SerB for the production of OPS. In one embodiment, the recombinant microorganism shows an enhancement of serine feedback-resistant serA or serC or deletion of at least one selected from among PhnC/PhnD/PhnE alkylphosphonate ABC transporter (phnCDE operon), alkaline phosphatase (phoA) and acid phosphatase (aphA). Preferably, the recombinant microorganisms for the production of OPS are the microorganism deposited under accession No. KCCM11103P or KCCM11212P. More preferably, the recombinant microorganism for the production of OPS is the microorganism deposited under accession No. KCCM11103P.

### [Mode for Invention]

A better understanding of the present invention may be obtained through the following examples which are set forth to illustrate, but are not to be construed to limit the present invention.

### <Preparation of O-phosphoserine producing Corynebacterium and Production of O-Phosphoserine Using the Same>

### EXAMPLE 1: Preparation of Phosphoserine Phosphatase (serB) deficient Corynebacterium Strain

*Corynebacterium glutamicum* 13032 was modified by deleting the serB gene (SEQ ID NO: 13, EC 3.1.3.3) encoding phosphoserine phosphatase, which catalyses the synthesis of L-serine from O-phosphoserine, therefrom. To this end, a fragment for inactivation of serB was constructed. In this regard, primers were designed for the preparation of the recombinant strain 13032-ΔserB of the present invention. First, the serB sequence of *Corynebacterium glutamicum* 13032 was obtained with reference to the data of the NIH GenBank, and primers SEQ ID NOS: 22 to 27 were synthesized on the basis of the serB sequence. For the site- specific gene disruption, a pDC vector which cannot replicate in *Corynebacterium glutamicum* was employed. A pDC-ΔserB plasmid in which the open reading frame of serB was internally disrupted was constructed and adopted for the preparation of a site-specific serB gene deletion in *Corynebacterium glutamicum* mutant strain. The internal gene distruption of the pDC-ΔserB was generated by crossover PCR using primer pairs of SEQ ID NOS: 22 and 23 and SEQ ID NOS: 24 and 25, with the genomic DNA of *Corynebacterium glutamicum* ATCC13032 serving as a template, and introducing the PCR product into a pDC vector. The resulting recombinant plasmid was transformed into wild-type *Corynebacterium glutamicum* by electroporation (van der Rest et al. 1999). The plasmid was introduced into the chromosome by primary recombination (crossing over), followed by secondary recombination (crossing over) to excise the original serB from the chromosome.

After completion of the secondary recombination, the *Corynebacterium glutamicum* transformants containing the deletion mutation of serB was analyzed by diagnostic PCR using a pair of gene-specific primers SEQ ID NOS: 26 and 27. The recombinant strain was named CB01-0047.

### EXAMPLE 2: Assay for O-Phosphoserine Productivity in the Phosphoserine Phosphatase deficient Corynebacterium Strain

The mutant strain CB01-0047, resulting from the deletion of serB from *Corynebacterium glutamicum* 13032, which was anticipated to accumulate O-phosphoserine, was spread over BHIS plates and incubated overnight in a 30 °C incubator. Afterwards, the colonies appearing on the BHIS plates were inoculated in 25 mL of a titer medium shown in Table 1 using a platinum loop and then incubated at 30°C for 48 hours with shaking at 200 rpm. The results are summarized in Table 2, below.

**[Table 1]**

| Composition of Main Medium | |
|---|---|
| Composition | Amount (per liter) |
| Glucose | 100 g |
| KH₂PO₄ | 1.1 g |
| (NH₄)₂SO₄ | 45 g |
| MgSO₄ 7H₂O | 1.2 g |
| HSM | 20 g |
| Trace elements | 20 ml |
| Calcium carbonate | 30 g |
| pH | 7.2 |
| Trace elements | |

| Composition | Amount (per liter) |
|---|---|
| Biotin | 0.09 g |
| Thiamine | 0.45 g |
| Ca-Panthenate | 0.45 g |
| NCA | 3 g |
| FeSO_{4.} 7H₂O | 9 g |
| MnSO_{4.} 4H₂O | 9 g |
| ZnSO_{4.} 7H₂O | 0.045 g |
| CuSO4_{.} 5H₂O | 0.045 g |

**[Table 2]**

| Strain | OD 562 nm | Sugar consumed (g/L) | O-phosphoserine(g/L) |
|---|---|---|---|
| *C.glutamicum* 13032 | 25 | 100 | 0.02 |
| CB01-0047 | 6.5 | 23 | 0.07 |

The CB01-0047 strain was observed to grow very slowly in the titer medium. This growth retardation was not improved even upon the addition of an L-glycine supplement. However, the growth was increased in the presence of L-serine, but a slight increase in the production of O-phosphoserine compared to the wild-type was observed. The results are summarized in Table 3, below.

**[Table 3]**

| Strain | A.A. (amino acids) added | OD 562nm | Sugar consumed (g/L) | O-phosphoserine(g/L) |
|---|---|---|---|---|
| CB01-0047 | - | 6.3 | 21 | 0.09 |
| | L-Glycine | 6.9 | 22 | 0.09 |
| | L-Serine | 24.5 | 100 | 0.05 |

### EXAMPLE 3: Construction of mutated Phosphoglycerate Dehydrogenase (SerA*) Gene derived from Corynebacterium

The *Corynebacterium glutamicum*-derived genes serA*(E235K) (SEQ ID NO: 14) and serA*(197Δ) (SEQ ID NO: 15) were constructed, which encode respective mutants of 3-phosphoglycerate dehydrogenase, an enzyme catalyzing the synthesis of 3-phosphohydroxypyruvate from 3-phosphoglycerate. The mutants were reported to be feedback resistant (FBR) to serine (Peters-Wendisch P et al., Appl. Microbiol. Biotechnol., 60: 437-441, 2002; EP0943687B). serA*(E235K) was obtained by sewing PCR on the genomic DNA of ATCC13032 using primers of SEQ ID NOS: 28 to 31 while serA*(197Δ) was constructed by PCR using pairs of primers of SEQ ID NOS: 28 to 32. The PCR products thus obtained were inserted into respective T vectors to construct recombinant vectors called Tblunt-serA*(E235K) and Tblunt-serA*(197Δ). Subsequently, the two vectors were treated with restriction enzymes EcoRV and XbaI to give two DNA fragments serA*(E235K) and serA*(197Δ). These fragments were inserted to respective pECCG117-Pcj7-GFP-terminator vectors which had been disgested with the same restriction enzymes. As a result, two recombinant vectors pECCG117-Pcj7-serA*(E235K), and pECCG117-Pcj7- serA*(197Δ) were obtained.

### EXAMPLE 4: Preparation of serA* overexpressing Corynebacterium Strain and Assay for O-Phosphoserine Productivity

The two *Corynebacterium*-derived FBR-serA* plasmids constructed in Example 3 were introduced into *Corynebacterium glutamicum* CB01-0047. To evaluate O-phosphoserine productivity, the transformants were spread over BHIS plates and incubated overnight at 30°C. Afterwards, the colonies appearing on the BHIS plates were inoculated in 25 mL of a titer medium shown in Table 1 additionally contained 2 g/L L-serine using a platinum loop and then incubated at 30°C for 48 hours with shaking at 200 rpm. The results are summarized in Table 4, below.

**[Table 4]**

| Strain | OD 562nm | Sugar consumed (g/L) | O-phosphoserine(g/L) |
|---|---|---|---|
| CB01-0047/pECCG117 | 24.5 | 100 | 0.03 |
| CB01-0047/pECCG117-Pcj7-serA* (E235K) | 25.3 | 100 | 0.3 |
| CB01-0047/pECCG117-Pcj7-serA* (197Δ) | 24.3 | 100 | 0.28 |

In the *Corynebacterium glutamicum* strains transformed with the corynebacterium-derived FBR-serA*, as shown in Table 4, The accumulations of O-phosphoserine at a concentration of from 0.1 to 0.3 g/L were observed.

### <Preparation of O-Phosphoserine producing E. coli and Production of O-Phosphoserine Using the Same>

### EXAMPLE 5: Preparation of E. coli Strain having the reduced activity of Phosphoserine Phosphatase (SerB)

*E. coli* was modified by deleting the serB gene (SEQ ID NO: 16) encoding phosphoserine phosphatase, which catalyses the synthesis of L-serine from O-phosphoserine, therefrom. The deletion mutant *E. coli* K12 was prepared using the one-step inactivation method (Datsenko KA and Wanner BL, Proc. Natl. Acad. Sci., 97: 6640-6645, 2000) to delete an antibiotic-resistant maker gene. To prepare the serB deletion strain, first, PCR was performed on a pKD3 plasmid (Datsenko KA and Wanner BL, Proc. Natl. Acad. Sci., 97: 6640-6645, 2000; GenBank No. AY048742) using a pair of primers of SEQ ID NOS: 33 and 34. The PCR product was (indroduced into competent cells of pKD46 containing *E. coli* K12 (Datsenko KA and Wanner BL, Proc. Natl. Acad. Sci., 97: 6640-6645, 2000; GenBank No. AY048746) by electroporation. Thereafter, strains that showed resistance to chloramphenicol were subjected to PCR to confirm the deletion of serB, and then transformed with pCP20 (Datsenko KA and Wanner BL, Proc. Natl. Acad. Sci., 97: 6640-6645, 2000) to remove the antibiotic-resistant marker. The resulting mutant strain was named CA07-0012.

In addition, the initiation codon of serB was modified to lower phosphoserine phosphatase activity as follows. The wild-type serB gene with ATG as an initiation codon was obtained by PCR with the genomic DNA of *E. coli* W3110 serving as a template. A mutant serB with CTG as an initiation codon was constructed by sewing PCR. A pair of primes of SEQ ID NOS: 35 and 36 was used in the PCR for amplifying the wild-type serB while pairs of primers of SEQ ID NOS: 37 to 38 were employed for PCR amplification of the mutant serB. The PCR products was treated with *HindIII* and cloned into pccBAC1 (Epicentre) at the *HindIII* restriction site to construct pccBAC1-Pself-ATG-serB, and pccBAC1-Pself-CTG-serB respectively. The wild-type and the mutant serB vector was introduced into CA07-0012 to compare the phosphoserine phosphatase activity.

### EXAMPLE 6: Assay of Strain having the reduced activity of SerB for O-Phosphoserine Productivity

The phosphoserine phosphatase deficient mutant strain CA07-0012 that was anticipated to accumulate O-phosphoserine, was spread over LB plates and incubated overnight in a 33°C incubator. Afterwards, the colonies appearing on the LB plates were inoculated in 25 mL of a titer medium shown in Table 5 using a platinum loop and then incubated at 33°C for 48 hours with shaking at 200 rpm. The results are summarized in Table 6, below.

**[Table 5]**

| Composition | Amount (per liter) |
|---|---|
| Glucose | 40 g |
| KH₂PO₄ | 2 g |
| (NH₄) ₂SO₄ | 17 g |
| MgSO_{4.}7H₂O | 1 g |
| FeSO_{4.}7H₂O | 10 mg |
| MnSO_{4.}4H₂O | 10 mg |
| ZnSO_{4.}7H₂O | 10 mg |
| Yeast extract | 2 g |
| Calcium carbonate | 30 g |
| pH | 6.8 |

**[Table 6]**

| Strain | OD 562nm | Sugar consumed (g/L) | O-phosphoserine (g/L) |
|---|---|---|---|
| *E.coli* W3110 | 16 | 40 | 0.03 |
| CA07-0012 | 9.8 | 16 | 0.5 |
| CA07-0012 / pccBAC1-Pself-ATG-serB | 20 | 40 | 0 |
| CA07-0012 / pccBAC-Pself-CTG-serB | 15 | 40 | 0.7 |

To enhance the growth and O-phosphoserine productivity thereof, CA07-0012 was cultured for 48 hours in the titer medium of Table 5 additionally contained 1 g/L L-glycine. The results are summarized in Table 7, below.

**[Table 7]**

| Strain | OD 562nm | Sugar consumed (g/L) | O-phosphoserine(g/L) |
|---|---|---|---|
| *E.coli* W3110 | 16 | 40 | 0.03 |
| CA07-0012 | 18 | 40 | 1.5 |

As shown in Table 7, the addition of L-glycine to the culture medium allowed the strain to increase the growth rate and the O-phosphoserine productivity.

### EXAMPLE 7: Construction of the Vector Harvoring the Mutated Phosphoglycerate Dehydrogenase (SerA*) Gene derived from E. coli

The *E. coli*-derived genes serA* (G336V) (SEQ ID NO: 18), serA* (G336V, G337V) (SEQ ID NO: 19), and serA* (G336V, R338G) (SEQ ID NO: 20) encoding respective mutants of 3-phosphoglycerate dehydrogenase, an enzyme catalyzing the synthesis of 3-phosphohydroxypyruvate from 3-phosphoglycerate were constructed. The mutants were reported to be feedback resistant (FBR) to serine (Grant GA, Xu XL and Hu Z, Biochem., 39: 7316-7319, 2000; Grant GA, Hu Z and Xu XL, J. Biol. Chem., 276: 17844-17850, 2001). The introduction of the mutant genes into the chromosome of *E. coli* was carried out using the sewing PCR method. The DNA fragments containing mutations were prepared using following primers.

Primers of SEQ ID NOS: 39 and 41 were used commonly in SerA* gene. To introduce mutations into the serA gene, PCR was performed with a pair of primers of SEQ ID NOS: 42 and 43 for serA* (G336V), with a pair of primers of SEQ ID NOS: 44 and 45 for serA*(G336V, G337V), and with a pair of primers of SEQ ID NOS: 46 and 47 for serA* (G336V, R338G) . The primers were synthesized on the basis of information about the K12 W3110 gene (GenBank accession number AP 003471) and its neighboring nucleotide sequences, registered in the NIH GenBank.

### EXAMPLE 8: Cloning of E. coli-Derived serA Gene, serA* Gene, and 3-Phosphoserine Aminotransferase (serC) Gene

serA(SEQ ID NO: 17, EC 1.1.1.95), serC(SEQ ID NO: 21, EC 2.6.1.52), serA*(G336V), serA*(G336V, G337V) and serA*(G336V, R338G) were cloned as follows. serA and serC were obtained by performing PCR on the genomic DNA of E. coli W3110 while serA*(G336V), serA*(G336V, G337V), and serA*(G336V, R338G) were constructed by PCR with the DNA fragments of Example 7 serving as templates. PCR primers were SEQ ID NOS: 48 and 49 for serA and SEQ ID NOS: 50 and 51 for serC. After treatment with EcoRV and HindII, the PCR products were cloned into the recombinant vector pCL-Prmf, constructed by inserting the *E. coli* rmf promoter into the pCL1920 vector (GenBank No AB236930) to produce respective recombinant vectors named pCL-Prmf-serA, pCL-Prmf-serC, pCL-Prmf-serA*(G336V), pCL-Prmf-serA*(G336V, G337V), and pCL-Prmf-serA*(G336V, R338V) respectively.

In addition, plasmids in which serA, one of the three serA mutants, and/or serC form an operon, that is, pCL-Prmf-serA-(RBS)serC, pCL-Prmf-serA*(G336V)-(RBS)serC, pCL-Prmf-serA*(G336V, G337V)-(RBS)serC, and pCL-Prmf-serA*(G336V, R338V)-(RBS)serC were constructed. In this regard, an (RBS)serC fragment was obtained using primers of SEQ ID NOS: 51 and 52 and cloned at a HindIII site into pCL-Prmf-serA, pCL-Prmf-serA*(G336V), pCL-Prmf-serA*(G336V, G337V), and pCL-Prmf-serA*(G336V, R338V).

### EXAMPLE 9: Preparation of E. coli-Derived serA, serA* and serC enhanced Strains and Assay for O-Phosphoserine Productivity

The eight plasmids constructed in Example 8 were transformed into CA07-0012 and the resulting recombinant strains were assayed for the productivity of O-phosphoserine. Each strain was spread over LB plates and incubated overnight at 33°C. Afterwards, colonies appearing on the LB plates were inoculated into 25 mL of titer media of Table 8 and cultured at 33°C for 48 hours with shaking at 200 rpm. The results are summarized in Table 9, below.

**[Table 8]**

| Composition | Amount (per liter) |
|---|---|
| Glucose | 40 g |
| KH₂PO₄ | 4 g |
| (NH₄)₂SO₄ | 17 g |
| MgSO_{4.}7H₂O | 1 g |
| FeSO_{4.}7H₂O | 10 mg |
| MnSO_{4.}4H₂O | 10 mg |
| ZnSO_{4.}7H₂O | 10 mg |
| L-Glycine | 2.5 g |
| Tryptone | 2 g |
| Yeast extract | 2 g |
| Calcium carbonate | 30 g |
| pH | 6.8 |

**[Table 9]**

| Strain | OD 562nm | Sugar consumed (g /L) | O-phosphoserine (g/L) |
|---|---|---|---|
| CA07-0012 | 23 | 40 | 1.7 |
| CA07-0012 / pCL-Prmf-serA | 25 | 40 | 1.8 |
| CA07-0012 / pCL-Prmf-serA* (G336V) | 23 | 37 | 2.2 |
| CA07-0012 / pCL-Prmf-serA*(G336V,G337V) | 21 | 36 | 2.1 |
| CA07-0012/pCL-Prmf-serA*(G336V, R338V) | 22 | 37 | 2.2 |
| CA07-0012/pCL-Prmf-serA-(RBS) serC | 20 | 35 | 2.1 |
| CA07-0012/pCL-Prmf-serA* (G336V)-(RBS) serC | 18 | 31 | 2.5 |
| CA07-0012 / pCL-Prmf-serA*(G336V,G337V) - (RBS)serC | -17 | 32 | 2.5 |
| CA07-0012/pCL-Prmf-serA*(G336V, R338V)-(RBS)serC | 16 | 30 | 2.6 |

As apparent from the data of Table 9, the *E. coli* CA07-0012 strain increased in the productivity of O-phosphoserine when it was transformed with serA, and the productivity of O-phosphoserine was increased to a greater extent upon the introduction of one of the three serA* mutants. The strains in which serA, or one of three serA* mutants and serC that were activated simultaneously showed higher productivity of O-phosphoserine than did those in which there was the sole activation of serA or serA*. The highest productivity of O-phosphoserine was detected in a strain in which the mutant serA* and serC were activated simultaneously.

### EXAMPLE 10: Preparation of PhnC/PhnD/PhnE Alkylphosphonate ABC Transporter (phnCDE operon) deficient E. coli Strain

In *E. coli*, PhnC/PhnD/PhnE alkylphosphonate ABC transporter is reported to translocate O-phosphoserine into the cytoplasm (Wanner BL and Metcalf WW. FEMS Microbiol. Lett., 15:133-139, 1992). The phnCDE operon encoding a PhnC/PhnD/PhnE alkylphosphonate ABC transporter protein was deleted from a serB deletion strain to prepare the CA07-0016 strain. For the deletion of phnCDE, a pair of primers of SEQ ID NOS: 53 and 54 were employed. The deletion was performed in a manner similar to that of Example 5.

In addition, pCL-Prmf-serA*(G336V)-(RBS)serC, constructed in Example 8, was introduced into CA07-0016.

### EXAMPLE 11: Assay of phnCDE operon deficient E. coli Strain for O-Phosphoserine Productivity

The strains CA07-0016 and CA07-0016/pCL-Prmf-serA*(G336V)-(RBS)serC, prepared in Example 10, were evaluated for O-phosphoserine productivity. Each strain was spread over LB plates or LB(spectinomycine) plates and incubated overnight at 33°C. Afterwards, colonies appearing on the LB plates or the LB (spectinomycine) plates were inoculated into 25 mL of titer media of Table 8 using a platinum loop and cultured at 33°C for 48 hours with shaking at 200 rpm. The results are summarized in Table 10, below.

**[Table 10]**

| Strain | OD 562nm | Sugar consumed(g/ L) | O-phosphoserine(g/L ) |
|---|---|---|---|
| CA07-0012 | 22 | 40 | 1.8 |
| CA07-0016 | 23 | 38 | 2.0 |
| CA07-0012/pCL-Prmf-serA*(G336V)-(RBS)serC | 21 | 35 | 2.1 |
| CA07-0016/pCL-Prmf-serA* (G336V) - (RBS) serC | 20 | 40 | 2.4 |

As seen in Table 10, the phnCDE operon deletion strain showed only a slight increase in O-phosphoserine productivity.

### EXAMPLE 12: Preparation of Alkaline Phosphatase (phoA), Acid Phosphatase (aphA) deficient E. coli Strain

The phosphoserine phosphatase deletion *E. coli* strain was additionally deleted the phoA gene coding for alkaline phosphatase and the aphA gene coding for acid phosphatase. A DNA fragment for use in deleting phoA was obtained by performing PCR on a pkD3 plasmid with a pair of primers of SEQ ID NOS: 55 and 56. On the other hand, a DNA fragment for use in deleting aphA was obtained using a pair of primers of SEQ ID NOS: 57 and 58 in the same manner. Each deletion strain was prepared in the same manner as in Example 5. The strain which deleted both phoA and aphA was prepared by electroporating the DNA fragment for aphA deletion into a competent cell of the phoA deletion strain which had been transformed again with pKD46. Thereafter, the transformants which were resistant to chloramphenicol were subjected to PCR to confirm the deletion of aphA, and then transformed with pCP20 to remove the antibiotic-ressitant marker. The resulting mutant strains and their genotypes are summarized in-Table 11, below.

**[Table 11]**

| Strain | Genotype |
|---|---|
| CA07-0013 | W3110 ΔserB ΔphoA |
| CA07-0015 | W3110 ΔserB ΔaphA |
| CA07-0018 | W3110 ΔserB ΔphoA ΔaphA |

To each deletion strain, pCL-Prmf-serA*(G336V)-(RBS)serC, constructed in Example 8, was introduced in the same manner as in Example 10.

### EXAMPLE 13: Assay of Alkaline Phosphatase (phoA), Acid Phosphatase (aphA) deficient E. coli Strain for Ability to Degrade O-phosphoserine

The strains prepared in Example 12 were assayed for the productivity of OPS and the incapability of degrading OPS. Each strain was spread over LB plates or LB (spectinomycine) plates and incubated overnight at 33°C. Afterwards, colonies appearing on the LB plates or the LB (spectinomycine) plates were inoculated into 25 mL of titer media of Table 8 using a platinum loop and cultured at 33°C for 72 hours with shaking at 200 rpm. The results are summarized in Table 12, below. Incapability of degrading OPS was evaluated by a change in phosphate ion level as determined by phosphate ion analysis.

**[Table 12]**

| Strain | OD 562nm | Sugar consumed(g/L) | O-phosphoserine (g/L) | PO₄ (ppm) |
|---|---|---|---|---|
| CA07-0012 | 23 | 40 | 0.3 | 692 |
| CA07-0013 | 22 | 40 | 1.6 | 459 |
| CA07-0015 | 7.4 | 25 | 0 | 1098 |
| CA07-0018 | 19 | 40 | 1.7 | 487 |
| CA07-0012/pCL-Prmf-serA*(G336V)-(RBS)serC | 20 | 40 | 0.1 | 714 |
| CA07-0013/pCL-Prmf-serA*(G336V)-(RBS) serC | 16 | 40 | 1.8 | 385 |
| CA07-0018/pCL-Prmf-serA* (G336V) - (RBS)serC | 17 | 40 | 1.6 | 593 |

As seen in Table 12, the aphA deletion strain showed an abnormal growth phenomenon whereas the strains which lacked phoA or both phoA and aphA somewhat increased in O-phosphoserine productivity and decreased in the capability of degrading O-phosphoserine. On the other hand, the strain in which neither phoA nor aphA was deleted degraded the O-phosphoserine accumulated for 72 hours, with the concomitant increase of the PO₄ level.

### EXAMPLE 14: Preparation of phnCDE operon, phoA and aphA deficient Strains

The serB deficient strain (CA07-0012) was modified to further delete phnC/phnD/phnE alkylphosphonate ABC transporter-encoding phnCDE, alkaline phosphatase-encoding phoA, and acid phosphatase-encoding aphA. The strains thus prepared are given in Table 13, below. The one-step inactivation method described in Example 5 was employed to prepare the deletion mutants.

**[Table 13]**

| Strain | Genotype |
|---|---|
| CA07-0020 | W3110 ΔserB ΔphoA ΔphnCDE |
| CA07-0022 | W3110 ΔserB ΔphoA ΔaphA ΔphnCDE |

Into each of the deletion strains, the pCL-Prmf-serA*(G336V)-(RBS)serC, constructed in Example 8, was introduced in the same manner as in Example 10.

### EXAMPLE 15: Assay of phnCDE operon, phoA and aphA deficient E. coli Strains for O-Phosphoserine Productivity

The strains prepared in Example 14 were assayed for OPS productivity. Each strain was spread over LB plates or LB (spectinomycine) plates and incubated overnight at 33°C. Afterwards, colonies appearing on the LB plates or the LB (spectinomycine) plates were inoculated into 25 mL of titer media of Table 8 using a platinum loop and cultured at 33°C for 72 hours with shaking at 200 rpm. The results are summarized in Table 14, below.

**[Table 14]**

| Strain | OD 562nm | Sugar consumed (g/L) | O-phosphoserine (g/L) | PO₄ (ppm) |
|---|---|---|---|---|
| CA07-0012 | 23 | 40 | 0.3 | 692 |
| CA07-0020 | 18.3 | 40 | 1.9 | 262 |
| CA07-0022 | 19.1 | 40 | 2 | 263 |
| CA07-0012/pCL-Prmf-serA*(G336V)-(RBS) serC | 20 | 40 | 0.1 | 714 |
| CA07-0020/pCL-Prmf-serA* (G336V) - (RBS)serC | 17.6 | 40 | 2.5 | 174 |
| CA07-0022/pCL-Prmf-serA*(G336V)-(RBS)serC | 17 | 40 | 2.6 | 218 |

CA07-0020 and CA07-0022 were found to have increased OPS productivity and decreased ability to degrade O-phosphoserine, compared to CA07-0012. This property was also detected in the strains transformed further with pCL-Prmf-serA* (G336V) -(RBS)serC.

### EXAMPLE 16: Preparation of E. coli Mutants deficient of phnCDE operon, phoa, and aphA Genes and Having Substitutition of Phosphoglycerate Dehydrogenase (serA*)

In CA07-0022, 3-phosphoglycerate dehydrogenase-encoding serA was substituted with serA*(G336V), serA*(G336V, G337V), or serA*(G336V, R338G), all being reported to have feedback resistance to serine, on the chromosome, as follows. To introduce mutations into the serA gene on the chromosome, vectors were constructed as follows. PCR was performed with a pair of primers of SEQ ID NOS: 40 and 41 on serA* (G336V), serA*(G336V, G337V), and serA*(G336V, R338G), prepared in Example 7. After treatment with both SacI and BamHI, the PCR products thus obtained were cloned into pSG76C at the SacI and BamHI site. The resulting recombinant vector was transformed into *E. coli* BW which was then spread over LB plates. The colonies appearing on the plates were subjected to base sequencing, and the transformants into which mutations were introduced were selected. From them, plasmids were prepared using a typical miniprep method. According to the introduced mutations, the plasmids were named pSG76C-serA*(G336V), pSG76C-serA*(G336V, G337V) and pSG76C-serA*(G336V, R338G).

Each of the *E. coli* mutants was prepared as described previously (Posfai G, Kolisnychenko V, Bereczki Z and Blattner FR, Nucleic Acids Res. 27: 4409-4415, 1999), and the antibiotic-resistant marker gene was removed from them. To prepare the serA*(G336V) mutant, pSG76C-serA*(G336V) was introduced into a competent cell of CA07-0022 by electroporation. The strains resistant to chlorampenicol were subjected to PCR to confirm the introduction of serA* (G336V) . The strain was transformed with pST76-ASceP (Posfai G, Kolisnychenko V, Bereczki Z and Blattner FR, Nucleic Acids Res. 27: 4409-4415, 1999) to remove the antibiotic-resistant marker gene. The resulting strain was named CA07-0022 serA*(G336V). The CA07-0022 serA*(G336V) strain was transformed with pSG76C-serA*(G336V, G337V) and pSG76C-serA*(G336V, R338G) in a similar manner to give serA*(G336V, G337V) and serA*(G336V, R338G) mutants, named CA07-0022 serA*(G336V, G337V) and serA* (G336V, R338G), respectively.

### EXAMPLE 17: Assay of E. coli Mutants deficient of phnCDE operon, aphA, and aphA Genes and Having Substitutition of Phosphoglycerate Dehydrogenase (serA*) for O-Phosphoserine Productivity

The strains prepared in Example 16 were assayed for O-phosphoserine productivity. Each strain was spread over LB plates or LB (spectinomycine) plates and incubated overnight at 33°C. Afterwards, colonies appearing on the LB plates or the LB (spectinomycine) plates were inoculated into 25 mL of titer media of Table 8 using a platinum loop and cultured at 33°C for 48 hours with shaking at 200 rpm. The results are summarized in Table 15, below.

**[Table 15]**

| Strain | OD 562nm | Sugar consumed (g/L) | O-phosphoserine (g/L) |
|---|---|---|---|
| CA07-0022 | 22 | 40 | 2.2 |
| CA07-0022 serA*(G336V) | 21 | 35 | 2.7 |
| CA07-0022 serA*(G336V, G337V) | 20 | 36 | 2.8 |
| CA07-0022 serA*(G336V, R338G) | 20 | 38 | 2.7 |

The strains in which serA had been altered to sereine feedback-resistant genes showed somewhat decreased growth rates, but an increase in O-phosphoserine productivity.

### EXAMPLE 18: Preparation of Mutant E. coli Strains deficient of phnCDE operon, phoA and aphA and HavingSubstituted Phosphoglycerate Dehydrogenase (serA*) and enhanced 3-Phosphoserine Aminotransferase and Assay for O-Phosphoserine Productivity

Into the strains prepared in Example 16, that is, CA07-0022 serA*(G336V), CA07-0022 serA* (G336V, G337V), and CA07-0022 serA* (G336V, R338G) was introduced in the plasmid prepared in Example 8, that is, pCL-Prmf-serC. The resulting mutants were evaluated for O-phosphoserine productivity in the same manner as in Example 9. The results are summarized in Table 16, below.

**[Table 16]**

| Strain | OD562nm | Sugar consumed (g/L) | O-phosphoserine(g/L) |
|---|---|---|---|
| CA07-0022 / pCL-Prmf-serC | 20 | 38 | 2.9 |
| CA07-0022 serA*(G336V) / pCL-Prmf-serC | 19.5 | 34 | 3.45 |
| CA07-0022 serA*(G336V, G337V) / pCL-Prmf-serC | 20 | 33 | 3.55 |
| CA07-0022 serA*(G336V, R338G) / pCL-Prmf-serC | 19 | 35 | 3.6 |

As seen in Table 16, the serC-activated strains were found to be improved in O-phosphoserine productivity. This phenomenon was more apparent in the strain in which serA was modified into a serine feedback-resistant gene.

### EXAMPLE 19: Pyrimidine Nucleotide Transhydrogenase (PntAB)-enhanced Strain and construction of Glutamate Dehydrogenase (GdhA) containing vector

To prepare a strain in which pntAB encoding for pyrimidine nucleotide transhydrogenase is upregulated, the pntAB promoter was changed with a trc promoter using a mutant loxP system (Arakawa H et al., BMC Biotechnol. 1: 7, 2001). In this regard, PCR was performed on the pmlox-trc (ref) plasmid using a pair of primers of SEQ ID NOS: 59 and 60, and the PCR product thus obtained was introduced into a competent cell of CA07-0022 serA*(G336V) anchoring pKD46 by electroporation. The transformants which showed resistance to chloramphenicol were subjected to PCR to confirm the replacement of the promoter, followed by transformation with pJW168(Le Borgne S et al., Methods Mol Biol. 267: 135-43, 2004) to remove the antibiotic-resistant marker gene. The resulting strain was named CA07-0022 serA*(G336V) P(trc)-pntAB. The primers used for the PCR were designed on the basis of the information about the K12 W3110 gene (GenBank accession number AP002223, AP002224) and its neighboring nucleotide sequences, registered in the NHI GenBank.

The glutamate dehydrogenase-encoding gdhA gene was amplified using PCR with a pair of primers of SEQ ID NOS: 61 and 62 to give a single polynucleotide. Both the primers of SEQ ID NOS: 61 and 62 have the restriction enzyme site *HindIII*. The primers were designed on the basis of the information about the K12 W3110 gene (GenBank accession number AP 002380) and its neighboring nucleotide sequences, registered in the NHI GenBank.

PCR started with denaturation at 94°C for 3 min and proceeded with 25 cycles of denaturing at 94°C for 30 sec, annealing at 56°C for 30 sec and extending at 72°C for 2 min, followed by extending at 72°C for 7 min. As a result, a 1714 bp-long polynucleotide was obtained. After treatment with *HindIII,* the PCR product was cloned into pCC1BAC at the *HindIII* site and introduced into E. coli DH5α which was then spread over LB plates. Base sequencing allowed the selection of the developed colonies that had no mutations in their gdhA gene. The plasmid was isolated using a typical miniprep method and named pCC1BAC-P(native)-gdhA.

### EXAMPLE 20: Introduction of pntAB and gdhA into OPS-Producing Strain and Assay for OPS Productivity

To prepare an OPS-producing strain in which pntAB and gdhA were upregulated, the CA07-0022 serA*(G336V) strain or the CA07-0022 serA*(G336V) P(trc)-pntAB strain was transformed with pCL-P(trc)-serA*(G336V)-serC and pCC1BAC-P(native)-gdhA individually or in combination, as shown in the following table. Each transformant was incubated overnight at 33°C on LB plates. The colonies were inoculated into the 25 mL of titer media of Table 8 using a platinum loop and cultured at 33°C for 48 hours with shaking at 200 rpm.

**[Table 17]**

| Strain | OD562nm | Sugar consumed (g/L) | OPS(g/L) |
|---|---|---|---|
| CA07-0022 serA* (G336V) /pCL-P(trc)-serA*(G336V)-serC | 20 | 37 | 3.1 |
| CA07-0022 serA*(G336V) P(trc)-pntAB /pCL-P(trc)-serA*(G336V)-serC | 19 | 35 | 3.4 |
| CA07-0022 serA* (G336V) P(trc)-pntAB /pCC1BAC-P(native)-gdhA | 7.2 | 11 | 0.2 |
| CA07-0022 serA*(G336V) P (trc) -pntAB /pCC1BAC-P(native)-gdhA /pCL-P(trc)-serA*(G336V)-serC | 27.0 | 40 | 3.95 |

As seen in Table 17, the strain was improved in O-phosphoserine productivity when pntAB was upregulated therein. The upregulation of both pntAB and gdhA brought about a bigger increase in O-phosphoserine productivity, as compared to the control. Hence, pntAB and gdhA are understood to play an important role in the production of OPS.

### EXAMPLE 21: Construction of Vectors Carrying Genes Encoding E. coli O-Acetylserine/Cysteine Efflux Protein (ydeD), O-Acetylserine/Cysteine Efflux Permease (yfiK), Homoserine/Homoserine Lactone Efflux Protein (rhtB), Threonine/Homoserine Efflux Protein (rhtC), Arsenite/Antimonite Transporter (asrB), and Leucine/Isoleucine/Valine Transport Subunit (livHM)

The release of the produced O-phosphoserine out of the cell requires a suitable export factor none of which have, however, been reported previously. In this context, six genes, that is, O-acetylserine/cysteine efflux protein-encoding ydeD, O-acetylserine/cysteine efflux permease-encoding yfiK (Franke I, Resch A, Dassler T, Maier T and Bock A, J. Bacteriology, 185: 1161-166, 2003), homoserine/homoserine lactone efflux protein-encoding rhtB, threonine/homoserine efflux protein-encoding RhtC, arsenite/antimonite transporter-encoding asrB, and leucine/isoleucine/valine transport subunit-encoding livHM were selected from among the previously reported variety of transporter genes, and were cloned and evaluated.

Each gene was obtained by performing PCR on the genomic DNA of *E. coli* W3110, with a pair of primers of SEQ ID NOS: 63 and 64 for ydeD, with a pair of primers of SEQ ID NOS: 65 and 66 for yfiK, with a pair of primers of SEQ ID NOS: 67 and 68 for rhtB, with a pair of primers of SEQ ID NOS: 69 and 70 for rhtC, with a pair of primers of SEQ ID NOS: 71 and 72 for asrB, and with a pair of primers of SEQ ID NOS: 73 and 74 for livHM. After treatment with EcoRV and HindIII, each of the PCR products thus obtained was cloned at the EcoRV and HindIII site into the pCL-Prmf-GFP, to give recombinant vectors, named pCL-Prmf-ydeD, pCL-Prmf-yfiK, pCL-Prmf-rhtB, pCL-Prmf-rhtC, pCL-Prmf-arsB, and pCL-Prmf-livHM.

### EXAMPLE 22: introduction of Vectors Carrying Genes Encoding E. coli YdeD, YfiK, RhtB, RhtC, AsrB, livHM into O-Phosphoserine-Producing Strain and Assay for O-Phosphoserine Productivity

The CA07-0022 serA*(G336V) strain was transformed with the six plasmids constructed in Example 21 and evaluated for O-phosphoserine productivity in the same manner as in Example 9. The results are given in Table 18, below.

**[Table 18]**

| Strain | OD 562nm | Sugar consumed (g/L) | O-phosphoserine(g/L) |
|---|---|---|---|
| CA07-0022 serA* (G336V) | 22.1 | 37.7 | 2.5 |
| CA07-0022 serA* (G336V) /pCL-Prmf-ydeD | 7.4 | 22.3 | 0.69 |
| CA07-0022 serA* (G336V) /pCL-Prmf-yfiK | 21 | 40 | 2.55 |
| CA07-0022 serA* (G336V) /pCL-Prmf-rhtB | 23 | 40 | 2.8 |
| CA07-0022 serA* (G336V) /pCL-Prmf-rhtC | 22.5 | 40 | 2.75 |
| CA07-0022 serA* (G336V) /pCL-Prmf-arsB | 21 | 38 | 2.4 |
| CA07-0022 serA* (G336V) /pCL-Prmf-livHM | 8 | 23 | 0.8 |

As shown in Table 18, the strains transformed with ydeD, mdtG or livHM exhibited decreased growth rate and decreased OPS productivity whereas transformation with yfiK, rhtB or rhtC increased growth rate and OPS productivity (Table 18).

### EXAMPLE 23: Preparation of Phosphoglycerate Mutase (gpmI, gpmA and gpmB) deficient Strain

gpmI, gpmA, and gpmB, each encoding phosphoglycerate mutase, were deleted solely or in combination from CA07-0022 serA*(G336V) to produce the mutant strains named CA07-0022 serA*(G336V)ΔgpmI, CA07-0022 serA*(G336V)ΔgpmA, CA07-0022 serA*(G336V)ΔgpmB, CA07-0022 serA*(G336V)ΔgpmIΔgpmA, CA07-0022 serA*(G336V)ΔgpmAΔgpmB, and CA07-0022 serA*(G336V)ΔgpmIΔgpmAΔgpmB, respectively. The gpmA- and gpmB-deletion strains were prepared in a manner similar to that of Example 5, using a pair of primers of SEQ ID NOS: 75 and 76 for gpmA and a pair of primers of SEQ ID NOS: 81 and 82 for gpmB. For the construction of a gpmI deletion strain, as described in Example 16, a gpmI mutation containing a stop codon was introduced using pSG76C. A gpmI mutant containing a stop codon was amplified by sewing PCR using primers of SEQ ID NOS: 77 to 81, with the genomic DNA of K12 W3110 serving as a template, and cloned into pSG76 at the *SacI*/*BamHI* site.

### EXAMPLE 24: Assay of gpmI, gpmA and gpmB deficient Strains for OPS Productivity

The strains prepared in Example 23 were evaluated for OPS productivity in the same manner as in Example 9. The results are summarized in Table 19, below.

**[Table19]**

| Strain | OD562nm | Sugar consumed (g/L) | OPS(g/L) |
|---|---|---|---|
| CA07-0022 serA* (G336V) | 23 | 40 | 2.4 |
| CA07-0022 serA* (G336V) ΔgpmI | 22 | 38 | 2.5 |
| CA07-0022 serA* (G336V)ΔgpmA | 20 | 34 | 2.8 |
| CA07-0022 serA* (G336V)ΔgpmB | 20 | 34 | 2.7 |
| CA07-0022 serA* (G336V)ΔgpmI ΔgpmA | 19 | 32 | 2.6 |
| CA07-0022 serA* (G336V)ΔgpmA ΔgpmB | 21 | 35 | 3.3 |

As can be seen in Table 19, when each of gpmI, gpmA and gpmB was deleted and the others not deleted, the sugar consumption of the mutant strains decreased, but their OPS productivity increased, compared to the mother strain. Particularly, the strain devoid of both gpmA and gpmB had similar sugar consumption, but increased OPS productivity, compared to the strains devoid of either gpmA or gpmB. Therefore, the deletion of gpmI, gpmA and gpmB is understood to produce an increased amount of 3-phosphoglycerate, a precursor of OPS, thus leading to increased OPS production.

### EXAMPLE 25: Preparation of 2-Amino-3-ketobutyrate CoA Ligase (kbl), L-Serine Deaminase I (sdaA) deficient Strains

The kbl gene coding for 2-amino-3-ketobutyrate CoA ligase and the sdaA gene coding for L-serine deaminase I were deleted from CA07-0022 serA*(G336V) to yield CA07-0022 serA*(G336V) Δkbl, and CA07-0022 serA*(G336V) ΔsdaA, respectively. The kbl- and the sdaA-deletion strain were prepared in a manner similar to that of Example 5, using a pair of primers of SEQ ID NOS: 83 and 84 for kbl and a pair of primers of SEQ ID NOS: 85 and 86 for sdaA.

### EXAMPLE 26: Assay for OD and Sugar Consumption of kbl/sdaA deficient Strains According to Glycine concentration

The strains prepared in Example 25 were evaluated for OD, sugar consumption, and O-phosphoserine productivity when they were incubated in the same medium condition as described in Table 8 of Example 9, with the exception that glycine was used in an amount of from 0 to 2.5 g/L.

**[Table 20]**

| Glycine Conc. (g/L) | Strain | OD 562nm | Sugar Consumed (g/L) | OPS (g/L) |
|---|---|---|---|---|
| 0 | CA07-0022 serA* (G336V) | 8 | 10 | 0.7 |
| | CA07-0022 serA* (G336V)Δkbl | 7 | 8 | 0.7 |
| | CA07-0022 serA* (G336V)Δsda A | 9 | 10 | 0.7 |
| 1 | CA07-0022 serA* (G336V) | 15 | 30 | 1.5 |
| | CA07-0022 serA* (G336V)Δkbl | 14 | 28 | 1.2 |
| | CA07-0022 serA* (G336V)Δsda A | 22 | 39 | 2.4 |
| 2 | CA07-0022 serA* (G336V) | 19 | 35 | 2.1 |
| | CA07-0022 serA* (G336V)Δkbl | 17 | 32 | 1.7 |
| | CA07-0022 serA* (G336V)Δsda A | 23 | 40 | 2.5 |
| 2.5 | CA07-0022 serA* (G336V) | 22 | 38 | 2.5 |
| | CA07-0022 serA* (G336V)Δkbl | 23 | 39 | 2.7 |
| | CA07-0022 serA* (G336V)Δsda A | 24 | 40 | 2.3 |

As can seen in Table 20, the OD and the rate of sugar consumption in all three of the strains increased when the glycine level in the medium was increased. Particularly, the sdaA-deletion strain showed a significant increase in OD and sugar consumption rate at a glycine concentration of 1 g/L. The OPS productivity of the kbl deletion strain greatly improved in the presence of 2.5 g/L glycine.

### EXAMPLE 27: Preparation of iclR deficient Strain

The transcription factor iclR was deleted from CA07-0022 serA*(G336V) to produce CA07-0022 serA*(G336V) ΔiclR. The deletion mutant strain was prepared using the one-step inactivation method as in Example 5 and the antibiotic-resistant marker gene was removed. For the preparation of the iclR deletion strain, PCR was performed with a pair of primers of SEQ ID NOS: 87 and 88.

### EXAMPLE 28: Assay of the iclR deficient Strain for OPS Productivity

The strain prepared in Example 27 was evaluated for OPS productivity in the same manner as in Example 9.

**[Table 21]**

| Strain | OD562nm | Sugar consumed(g/L) | OPS (g/L) |
|---|---|---|---|
| CA07-0022 serA* (G336V) | 22 | 38 | 2.5 |
| CA07-0022 serA* (G336V)ΔiclR | 22 | 40 | 2.7 |

As is apparent from the data of Table 21, the OPS productivity of the iclR deletion strain was found to increase.

### EXAMPLE 29: Construction of Vectors Carrying E. coli Acetyl CoA Synthetase (acs), Pyruvate Oxidase Monomer (poxB), Acetate Kinase (ackA) and Phosphate Acetyltransferase (pta)

To enhance the production and reuse of acetate in the O-phosphoserine-producing strain, expression plasmids carrying acetyl CoA synthetase-encoding acs, pyruvate oxidase monomer-encoding poxB, acetate kinase-encoding ackA and phosphate acetyltransferase-encoding pts, respectively, were constructed.

Each gene was obtained by performing pfu PCR on the genomic DNA of *E*. *coli* W3110 with a pair of primers of SEQ ID NOS: 89 and 90 for acs, with a pair of primers of SEQ ID NOS: 91 and 92 for poxB, and with a pair of primers of SEQ ID NOS: 93 and 94 for ackA and pta. After treatment with HindIII, each of the PCR products thus obtained was cloned at the EcoRV and HindIII site into the pCL-Prmf-GFP vector constructed by inserting an *E. coli* rmf promoter into pCL1920, so as to give pCL-Prmf-acs, pCL-Prmf-poxB, and pCL-Prmf-ackA-pta. Subsequently, these plasmids were treated with EcoRI to obtain DNA inserts, that is, Prmf-acs, Prmf-poxB, and Prmf-ackA-pta, which were then introduced into pCC1BAC (EcoRI) (CopyControl™ pcc1BAC™ Vector, Epicentre. Cat. Nos. CBAC311) to construct pCC1BAC-Prmf-acs, pCC1BAC-Prmf-poxB, and pCC1BAC-Prmf-ackA-pta, respectively.

### EXAMPLE 30: Preparation of E. coli acs, poxB, ackA, pta-enhanced OPS-Producing Strain and Assay for OPS Productivity

The CA07-0022 serA*(G336V) strain was transformed with the three vectors prepared in Example 29 and assayed for OPS productivity in the same manner as in Example 9.

**[Table 22]**

| Strain | OD562nm | Sugar consumed (g/L) | OPS (g/L) |
|---|---|---|---|
| CA07-0022 serA* (G336V) | 21.9 | 35.5 | 2.45 |
| CA07-0022 serA* (G336V) / pCC1BAC-Prmf-acs | 23.6 | 40 | 2.65 |
| CA07-0022 serA* (G336V) / pCC1BAC-Prmf-poxB | 18.3 | 36.8 | 1.86 |
| CA07-0022 serA* (G336V) / pCC1BAC-Prmf-ackA-pta | 21.8 | 40 | 2.65 |

As can be seen in Table 22, the growth rate of the strain transformed with poxB decreased whereas the introduction of acs or ackA-pta increased the growth rate and OPS productivity.

### EXAMPLE 31: Construction of Vectors Carrying E. coli malate synthase A (aceB), Isocitrate Lyase Monomer (aceA), Phosphoenolpyruvate Carboxykinase (pckA), Malate Synthase G (glcB), and Malate Dehydrogenase (maeB)

Plasmids which allow the expression of both malate synthase A-encoding aceB and isocitrate lyase monomer-encoding aceA, phosphoenolpyruvate carboxykinase-encoding pckA, malate synthase G-encoding glcB, and malate dehydrogenase-encoding maeB in *E. coli,* respectively, were constructed.

The genes were prepared by performing pfu PCR on the genomic DNA of *E. coli* W3110 with a pair of primers of SEQ ID NOS: 95 and 96 for aceBA, with a pair of primers of SEQ ID NOS: 97 and 98 for pckA, with a pair of primers of SEQ ID NOS: 99 and 100 for glcB, and with a pair of primers of SEQ ID NOS: 101 and 102 for maeB. After treatment with HindIII, each of the PCR products thus obtained was cloned at the EcoRV and HindIII site into the pCL-Prmf-GFP vector constructed by inserting an *E. coli* rmf promoter into pCL1920, so as to give pCL-Prmf-aceBA, pCL-Prmf-pckA, pCL-Prmf-glcB, and pCL-Prmf-maeB.

### EXAMPLE 32: Preparation of E. coli aceB, aceA, pckA, glcB and maeB-enhanced OPS-Producing Strain and Assay for O-phosphoserine Productivity

The CA07-0022 serA*(G336V) strain was transformed with the four vectors prepared in Example 31 and assayed for O-phosphoserine productivity in the same manner as in Example 9.

**[Table 23]**

| Strain | OD 562nm | Sugar consumed (g/L) | OPS (g/L) |
|---|---|---|---|
| CA07-0022 serA* (G336V) | 23 | 40 | 2.4 |
| CA07-0022 serA*(G336V) / pCL-Prmf-aceBA | 20 | 36 | 1.9 |
| CA07-0022 serA*(G336V) / pCL-Prmf-pckA | 10 | 11 | 0 |
| CA07-0022 serA*(G336V) / pCL-Prmf-glcB | 21 | 40 | 2.8 |
| CA07-0022 serA*(G336V) / pCL-Prmf-maeB | 21 | 40 | 2.9 |

As can be seen in Table 23, the sugar consumption rate and OPS productivity of the strain somewhat decreased when transformed with aceBA and the growth rate significantly decreased when transformed with pckA whereas the introduction of glcB or maeB increased OPS productivity.

### EXAMPLE 33: Construction of Vectors Carrying Glyoxylate Carboligase (glc), Tartronate Semialdehyde Reductase 2 (glxR), and Glycerate Kinase II (glxK)

Glyoxylate carboligase-encoding gcl, tartronate semialdehyde reductase 2-encoding glxR, and glycerate kinase II-encoding glxK, all of which are involved in the conversion of glyoxylate into 3-phosphoglycerate, were cloned as follows. The genes were obtained by performing PCR on the genomic DNA of *E. coli* W3110 with a pair of primers of SEQ ID NOS: 103 and 104 for gcl, and with pairs of primers of SEQ ID NOS: 105 to 108 for glxR-glxK. After digestion with EcoRV and HindIII, each of the PCR products was cloned at the EcoRV and HindIII sites into the pCL-Prmf-GFP vector constructed by inserting an E. *coli* rmf promoter into pCL1920 to afford recombinant plasmids, named pCL-Prmf-gcl, pCL-Prmf-glxR-glxK, and pCL-Prmf-glxR-glxK-Prmf-gcl, respectively.

### EXAMPLE 34: Introduction of Vectors Carrying glc, glxR, glxK into O-Phosphoserine-Producing Strain and Assay for O-Phosphoserine Productivity

The three plasmids constructed in Example 33 were introduced into CA07-0022 serA*(G336V) which were then evaluated for O-phosphoserine productivity in the same manner as in Example 9. The results are summarized in Table 24, below.

**[Table 24]**

| Time | Strain | OD 562nm | Sugar consumed (g/L) | O-phosphoserine (g/L) |
|---|---|---|---|---|
| 24h | CA07-0022 serA*(G336V) | 15.6 | 24 | 1.25 |
| | CA07-0022 serA*(G336V) / pCL-Prmf-gcl | 19.6 | 29.7 | 1.2 |
| | CA07-0022 serA*(G336V) / pCL-Prmf-glxR-glxK | 21 | 33 | 1.3 |
| | CA07-0022 serA*(G336V) / pCL-Prmf-glxR-glxK-Prmf-gcl | 18.4 | 29.7 | 1.03 |
| 48h | CA07-0022 serA* (G336V) | 23 | 40 | 2.4 |
| | CA07-0022 serA* (G336V) / pCL-Prmf-gcl | 31.5 | 40 | 1.67 |
| | CA07-0022 serA* (G336V) / pCL-Prmf-glxR-glxK | 26.2 | 40 | 1.5 |
| | CA07-0022 serA* (G336V) / pCL-Prmf-glxR-glxK-Prmf-gcl | 22 | 40 | 1.61 |

As can be seen in Table 24, the final O-phosphoserine productivity of the strains transformed respectively with gcl, glxR-glxK and glxR-glxK-gcl was decreased, but growth rate and sugar consumption rate were increased, compared to the CA07-0022 serA*(G336V) strain itself. Particularly, the introduction of glxR-glxK was found to have the greatest increase on growth rate and sugar consumption rate.

### EXAMPLE 35: Evaluation of O-phosphoserine-Producing Strain in a Fermentor

CA07-0022 serA*(G336V)/pCL-Prmf-serA*(G336V)-serC strains were incubated at 33°C for 24 hours on MMYE agar plates (2 g/L glucose, 2 mM magnesium sulfate, 0.1 mM calcium chloride, 6 g/L sodium pyrophosphate, 0.5 g/L sodium chloride, 3 g/L potassium dihydrogen phosphate, 10 g/L yeast extract, 18 g/L agar) containing 50 µg/mL spectinomycine. The resulting colonies were scraped from 1/10 of the area of each agar plate, inoculated into a 50 µg/mL spectinomycine-containing seed medium, 10 g/L glucose, 0.5 g/L magnesium sulfate, 3 g/L potassium dihydrogen phosphate, 10 g/L yeast extract, 0.5 g/L sodium chloride, 1.5 g/L ammonium chloride, 12.8 g/L sodium pyrophosphate, 1 g/L glycine) in a baffle flask, and incubated at 30°C for six hours while shaking at 200 rpm. To 300 mL of a main medium in a 1 L fermentor, the resulting seed culture in an amount as large as 16% of the volume of the main medium was added, followed by incubation at 33°C and pH 7.0. The main medium had the composition given in Table 25, below.

**[Table 25]**

| [Composition of Main Medium] | |
|---|---|
| Glucose | 20 g/L |
| Magnesium sulfate | 0.3 g/L |
| Potassium dihydrogen phosphate | 1.5 g/L |
| Yeast extract | 5 g/L |
| Ammonium sulfate | 5 g/L |
| Tryptone | 10 g/L |
| Glycine | 2 g/L |
| Sodium chloride | 0.5 g/L |
| Sodium citrate | 1 g/L |
| Iron sulfide | 75 mg/L |
| calcium chloride | 15 mg/L |
| *Trace elements* | 1 ml/L |

| [*Trace elements*] | |
|---|---|
| Cobalt chloride | 0.7 g/L |
| Zinc sulfate | 0.3 g/L |
| Molybdate | 0.15 g/L |
| Boric acid | 1.2 g/L |
| Manganese sulfate | 1.6 g/L |
| Copper sulfate | 0.25 g/L |

During incubation, the pH of the culture medium was adjusted to 7.0 with ammonia water. Upon the depletion of glucose from the culture medium, fed-batch-type fermentation was conduced by adding a 520 g/L glucose solution. Following fermentation for 80 hours, O-phosphoserine was produced at a concentration of 19.5 g/L as measured by HPLC.

### <Development and Characterization of O-Phosphoserina (OPS) Sulfhydrylase (OPSS)>

### EXAMPLE 36: Development of OPS Sulfhydrylase (OPSS)

*Aeropyrum pernix, Mycobacterium tuberculosis,* and *Trichomonas vaginalis* are reported to have O-phosphoserine sulfhydrylase (OPSS), an enzyme that employs O-phospho-L-serine (OPS), instead of O-acetyl serine (OAS) in E. *coli,* as a substrate for the synthesis of cysteine (Mino K and Ishikawa K, FEBS letters, 551: 133-138, 2003; Burns KE, Baumgart S, Dorrestein PC, Zhai H, McLafferty FW and Begley TP, J. Am. Chem. Soc., 127: 11602-11603, 2005; Westrop GD, Goodall G, Mottram JC and Coombs GH, J. Biol. Chem., 281: 25062-25075, 2006). Based on the report, the present inventors found two types of OPS sulfhydrylase, which converts OPS into cysteine, from *Aeropyrum pernix* and *Mycobacterium tuberculosis* H37Rv. Of them, the *Mycobacterium tuberculosis* H37Rv-derived OPSS enzyme was used for screening amino acid homology. As a result, three types of OPSS were secured from *Mycobacterium smegmatis* str. MC2 155, *Rhodococcus jostii* RHA1, and *Nocardia farcinica* IFM 10152.

To obtain OPSS from each strain, a pET28a vector system (Novagen), which is typically used for enzyme expression, was constructed. Each templates and primers for use in cloning the five different OPS sulfhydrylase genes and the resulting recombinant plasmids are summarized in Table 26, below. Suitable combinations of the templates and the primers, as given in Table 26, were used for PCR for amplifying respective OPSS genes. The PCR products and the pET28a vector were digested with NdeI and HindIII (37°C for 3 hours) . Each of the gene fragments was ligated to the digested pET28a vector (Novagen). Base sequencing confirmed the construction of the expression vectors carrying the each OPSS genes. The enzyme expression vectors were introduced into *E. coli* (DE3) to produce strains capable of expressing five OPSS enzymes. Enzyme names are given in Table 26, below.

**[Table 26]**

| Enzyme | Vector | Template | Primer |
|---|---|---|---|
| Ape-OPSS | pET28a-Ape-OPSS | Synthetic DNA | SEQ ID NOS: 109 and 110 |
| Mtb-OPSS | pET28a-Mtb-OPSS | *Mtb* Genomic DNA | SEQ ID NOS: 111 and 112 |
| Msm-OPSS | pET28a-Msm-OPSS | *Msm* Genomic DNA | SEQ ID NOS: 113 and 114 |
| Rjo-OPSS | pET28a-Rjo-OPSS | *Rjo* Genomic DNA | ID NOS: 115 and 116 |
| Nfa-OPSS | pET28a-Nfa-OPSS | *Nfa* Genomic DNA | SEQ ID NOS: 117 and 118 |

Expression of the enzymes was conducted according to the instructions of the pET system manufacturer (Novagen). Single colonies of each strain from the LB plates were inoculated into 5 mL of LB broth and incubated at 37°C for 16 hours while shaking at 200 rpm. The cultures were transferred to 25 mL of fresh LB broth (in 250 mL flasks) and incubated to an OD₆₀₀ of 0.5 - 0.6 (for 2 - 3 hours) in the same condition, immediately after which 1 mM IPTG was added to the media to induce the enzymes to be expressed during incubation at 18°C for 18 hours while shaking at 120 rpm. The enzymes were purified using Ni-NTA columns for His-tag, with the aid of His SpinTrap (GE Healthcare). Of the five OPSS enzymes thus isolated, four were found to be in soluble forms, with one (Rjo-OPSS) being an inclusion body, as analyzed by 14% SDS-PAGE electrophoresis.

### EXAMPLE 37: Assay of OPS sulfhydrylase (OPSS) for Cysteine Synthesis Activity

The OPS sulfhydrylase enzymes obtained from the four microorganism strains were assayed for ability to catalyze the conversion of O-phosphoserine (OPS) to cysteine. With regard to assay conditions and methods (cysM enzyme assay), reference was made to previous reports (Mino K and Ishikawa K, FEBS letters, 551: 133-138, 2003; Burns KE, Baumgart S, Dorrestein PC, Zhai H, McLafferty FW and Begley TP, J. Am. Chem. Soc., 127: 11602-11603, 2005; Westrop GD, Goodall G, Mottram JC and Coombs GH, J. Biol. Chem., 281: 25062-25075, 2006). The amount of the substrate used is represented by a unit of mL. Assay conditions for enzyme activity are summarized in Table 27, below.

**[Table 27]**

| Stock soln | Final Conc. | Blank | OPS sulfhydrylase |
|---|---|---|---|
| 6x his-enzyme | | - | 40 (50mg) |
| 1 M HEPES(pH7.4) | 100 mM HEPES | 100 | 100 |
| 0.5 M Na₂S | 10 mM Na₂S | 20 | 20 |
| 10 mM PLP | 0.2 mM PLP | 20 | 20 |
| 100mM OPS | 5mM OPS | 0 | 50 |
| DW | | 790 | 750 |
| Total | | 1000 | 1000 |

Reaction solutions excepting of the enzymes were incubated at 37°C for 5 min, after which 50 mg of purified OPS sulfhydrylase was added to the reaction solution. At predetermined times during incubation at 37°C, 100 mL of the enzyme reactions was taken and mixed with 100 mL of 33.2% TCA to stop the enzymatic reaction. The cysteine concentrations of the enzyme reactions were quantitatively analyzed by measuring absorbance at OD₅₆₀ according to the Gaitonde method. Cysteine synthesis activities of the four different OPS sulfhydrylase enzymes are summarized in Table 28, below. The cysteine synthesis titers of the OPSS enzymes are expressed as cysteine conversion rates with reaction time.

**[Table 28]**

| | Cysteine Conversion Rate (%) | | |
|---|---|---|---|
| | 10min | 30min | 60min |
| Ape-OPSS | 63.4 | 89.7 | 97.4 |
| Mtb-OPSS | 1.7 | 4.8 | 10.1 |
| Msm-OPSS | 12.8 | 25 | 43.7 |
| Nfa-OPSS | 0.1 | 0.1 | 0.2 |

The OPS sulfhydrylase enzymes derived from *Aeropyrum pernix* and *Mycobacterium tuberculosis* H37Rv, which were previously reported (Mino K and Ishikawa K, FEBS letters, 551: 133-138, 2003; Westrop GD, Goodall G, Mottram JC and Coombs GH, J. Biol. Chem., 281: 25062-25075, 2006), were confirmed to have the activity of using OPS as a substrate to synthesize cysteine. The cysteine synthesis activity of the novel *Mycobacterium smegmatis* str. MC2 155-derived OPS sulfhydrylase, which was obtained by screening amino acid homology with the Mtb-OPSS enzyme, was first found. As seen in the data of Table 28, the conversion rate from OPS into cysteine of Ape-OPSS reached near 100% in one hour. The final conversion rate of the Msm-OPSS enzyme, which was newly selected through enzyme screening on the basis of previously reported *Mycobacterium tuberculosis* H37Rv-derived OPSS, was 43.7% that was 4.3 times as high as that of Mtb-OPSS. On the other hand, the novel Nocardia farcinica IFM 10152-derived OPS sulfhydrylase, obtained by the homology screening, exhibited insufficient activity of converting O-phosphoserine into cysteine.

### EXAMPLE 38: Preparation of Mtb-T and Msm-T that encode C-Terminally 5 Amino Acid Residues truncated Mtb-OPSS and Msm-OPSS

*Mycobacterium tuberculosis* H37Rv-derived OPSS (Mtb-OPSS), which catalyzes the conversion of OPS to cysteine with the aid of the additional enzymes mec+ and cysO, is reported to be able to use an S²⁻ containing sulfur source in converting OPS to cysteine even in the absence of the additional enzymes when five C-terminal amino acid residues are removed therefrom (Agren D, Schnell R and Schneider G, FEBS letters, 583: 330-336, 2009). On the basis of this report, Mtb-T (SEQ ID NO: 11), which can rapidly convert OPS in the presence of S²⁻ as a sulfur source, was obtained. Msm-T was also obtained from Msm-OPSS (SEQ ID NO: 9) that shares an amino acid homology with Mtb-OPSS. Expression vectors carrying the two enzyme mutants were constructed. In this regard, pfu PCR was performed on the genomic DNA of *Mycobacterium tuberculosis* H37Rv or *Mycobacterium smegmatis* in the presence of a pair of primers of SEQ ID NOS: 119, 120, 121 and 122. The OPSS gene fragments thus obtained were treated with NdeI and HindIII and were cloned into the pET28a vector digested with the same restriction enzymes to construct recombinant expression vectors named pET28a-Mtb-T and pET28a-Msm-T, respectively. The recombinant expression vectors were introduced into *E. coli* (DE3). The expression of the two mutant OPSS enzymes was confirmed by 14% SDS PAGE. The two mutant OPSS enzymes are purified and expressed in the same conditions as in Example 36. As a result, Mtb-T (SEQ ID NO: 11) and Msm-T (SEQ ID NO: 10) were obtained.

### EXAMPLE 39: Assay of Mtb-T and Msm-T for Cysteine Conversion Activity

On the basis of the report that *Mycobacterium tuberculosis* H37Rv-derived OPSS mutants devoid of five C-terminal amino acid residues show increased affinity for an S²⁻ group-containing sulfur source even in the absence of subsidiary enzymes (Agren D, Schnell R and Schneider G, FEBS letters, 583: 330-336, 2009), Mtb-T and Msm-T were obtained. They were evaluated for enzymatic activity by measuring final cysteine conversion rates. Enzymatic activity was assayed in the same condition and manner as in Example 37. The produced cysteine was quantitatively analyzed using the Gaitonde method.

**[Table 29]**

| | Cysteine Conversion Rate (%) | | |
|---|---|---|---|
| | 10min | 30min | 60min |
| Mtb-T | 9.5 | 18.6 | 37.1 |
| Msm-T | 20.3 | 54.6 | 100 |

As seen in Table 29, Msm-T, being devoid of the five C-terminal amino acid residues of *Mycobacterium smegmatis* str. MC2 155-derived OPSS allowed the conversion of cysteine from the substrate at a rate of 100% in one hour.

When its amino acid sequence was modified, the O-phosphoserine sulfhydrylase (OPSS) can more effectively catalyze the biosynthesis of L-cysteine.

### EXAMPLE 40: Requirement of Cofactor for OPS Sulfhydrylase Activity

To examine the effect of cofactors on the cysteine conversion of OPSS, the cysteine conversion rate of Msm-T was measured in the absence or presence of PLP (pyridoxal-5'-phosphate) and DTT (dithiothreitol) . In this regard, the substrates of 50 mM OPS broth and 100 mM Na₂S were reacted at 37°C for 30 min in the presence of 25 mM DTT or 0.2 mM PLP. The cysteine thus produced was quantitatively analyzed using the Gaitonde method. As seen in Table 30, the cysteine conversion rate in the presence of both PLP and DTT was 2.3 times as large as that in the absence of both PLP and DTT. Thus, both PLP and DTT were observed to have a positive influence on the conversion.

**[Table 30]**

| Msm-T | Cysteine Conversion Rate (%) |
|---|---|
| (-) PLP, (-) DTT | 23.62 |
| (+) PLP, (-) DTT | 33.21 |
| (-) PLP, (+) DTT | 40.08 |
| (+) PLP, (+) DTT | 54.65 |

### EXAMPLE 41: The Influence of Tenperature on the Activity of OPS Sulfhydrylase

The cysteine conversion rates of Ape-OPSS and Msm-T according to temperatures were examined. The enzymatic activity at 37°C and 60°C was measured 2, 5, 10, 30, and 60 min after reaction. The reaction was conducted under the condition of 100 mM HEPES (pH 7.4), 5 mM OPS, 10 mM Na₂S, 0.2 mM PLP, and CysM 50 µg/mL. The amount of produced cysteine was determined using the Gaitonde method. In the condition of a buffer, as shown in FIG. 2, Ape-OPSS showed a faster initial reaction rate at 37°C as well as higher reactivity at 60°C than did Msm-T.

### EXAMPLE 42: Heat Stability of OPS Sulfhydrylase

Ape-OPSS and Msm-T were analyzed for heat stability. Each of the enzymes was diluted to a concentration of 2 mg/mL in an OPS broth and thermally treated at 37°C and 60°C for 10, 30, 60, 120, and 240 min, followed by reaction at 37°C for 30 min under the condition of 5 mM OPS, 10 mM Na₂S, 0.2 mM PLP, and 100 mM HEPES (pH 7.4). For this reaction, 10 µg/mL Ape-OPSS and 50 µg/mL Msm-T were employed. The amounts of the produced cysteine were measured using the Gaitonde method. Ape-OPSS was observed to retain its intact activity in spite of heat treatment at 60°C for 4 hours while the activity of Msm-T was maintained at 37°C, but decreased by 50% upon heat treatment at 60°C for 30 min. The results are given in Table 31, below.

**[Table 31]**

| | Relative activity (%) | | | | | |
|---|---|---|---|---|---|---|
| Heating time (min) | (-) | 10 min | 30 min | 60 min | 120 min | 240 min |
| Ape-OPSS | 100 | 102 | 107 | 100 | 107 | 101 |
| Msm-T | 100 | 82 | 50 | 32 | 19 | 8 |

An examination was made of the retention of enzymatic activity at 37°C when Msm-T was used in an amount of 50 µg/mL, which is a practical concentration in OPS broth. In the absence of Na₂S, 50 µg/mL Msm-T was treated, together with 50 mM OPS broth and 0.2 mM PLP, at 37°C for 0.5, 1, 2, 4, and 6 hours, after which Na₂S was added to induce the enzymatic reaction. After the reaction for 30 min, the activity of Msm-T was measured. The amounts of the produced cysteine were determined using the Gaitonde method. As a result, the activity of Msm-T was decreased below 50% 2 hours after reaction at 37°C in OPS broth (Table 32).

**[Table 32]**

| Time | 0 | 30 min | 60 min | 120 min | 240 min | 360 min |
|---|---|---|---|---|---|---|
| Cysteine conversion rate (%) | 100 | 88 | 73 | 47 | 11 | 3 |

### EXAMPLE 43: The Infulence of pH on the OPS Sulfhydrylase

The cysteine conversion rates of Ape-OPSS and Msm-T according to pH were measured. In 100 mM buffer, Ape-OPSS and Msm-T, each having a concentration of 50 µg/mL, were subjected to reaction at 37°C for 10 min. In this regard, K-phosphate buffer with a pH of 6.4 / 7.0 / 7.4 / 8.0, Tris-HCl buffer with a pH of 7.0 / 7.4 / 8.0 / 8.5 / 8.8, and Na-carbonate buffer with a pH of 8.0 / 8.5 / 9.0 / 10.0 were used. The quantitative analysis of the produced cysteine was conducted using the Gaitonde method. As seen in FIG. 3, Msm-T exhibited the highest activity at a pH of from 8.0 to 9.0 irrespective of buffer. As for Ape-OPSS, its highest activity was detected in K-phosphate (pH 7.4), with an optimal pH differing from one buffer to another.

### EXAMPLE 44: Effect of Ions on the Activity of OPS Sulfhydrylase

Effects of ions on the activity of the OPSS enzymes were examined as follows. In a reaction mixture containing 5 mM OPS, 10 mM Na₂S, 0.2 mM PLP, and 100 mM HEPES (pH 7.4), the enzymes were subjected to reaction at 37°C for 30 min in the presence of (NH₄)₂SO₄ (1, 3, 5, 10, 20 g/L), KH₂PO₄ (0.5, 1, 2, 4, 8 g/L), or NH₄Cl (0.2, 0.5, 1, 2 g/L). Ape-OPSS and Msm-T were used at a concentration of 10 µg/mL and 50 µg/mL, respectively. The amounts of the produced cysteine were determined using the Gaitonde method.

No changes were detected in the cysteine conversion rate when (NH₄)₂SO₄ or KH₂PO₄ was added to the reaction mixture. On the other hand, as seen in Table 33, the cysteine conversion rate was decreased with an increase in NH₄Cl concentration. Particularly, the maximal enzyme activity was decreased by more than 70% when 2 g/L NH₄Cl was added. Therefore, NH₄Cl was observed to have a negative effect on the conversion activity of OPS sulfhydrylase.

**[Table 33]**

| Relative | Relative activity (%) | |
|---|---|---|
| NH₄Cl | Ape-OPSS | Msm-T |
| 0 | 100.00 | 100.00 |
| 0.2 | 86.26 | 91.49 |
| 0.5 | 73.35 | 91.30 |
| 1 | 49.11 | 67.11 |
| 2 | 27.72 | 47.12 |

### EXAMPLE 45: Effect of Sulfur Source on the Cysteine Synthesis Activity of OPS Sulfhydrylase

An experiment was conducted to examine the effect of sulfur sources on the cysteine synthesis activity of each enzyme. In a reaction mixture containing 5 mM OPS, 0.2 mM PLP, and 100 mM HEPES, each enzyme (50 µg/mL Ape-OPSS, 50 µg/mL Msm-T) was subjected to reaction at 37°C for 1 hour in the presence of 10 mM Na₂S, NaSH, or Na₂S₂O₃. The amounts of the produced cysteine were measured using the Gaitonde method. Ape-OPSS was observed to prefer Na₂S₂O₃ as a sulfur source, whereas Msm-T prefers Na₂S. The results are summarized in Table 34, below.

**[Table 34]**

| | Relative activity (%) | | |
|---|---|---|---|
| Enzyme | Na₂S | NaSH | Na₂s₂O₃ |
| Ape-OPSS | 100.0 | 95.2 | 142.3 |
| Msm-T | 106.7 | 98.3 | 66.2 |

### EXAMPLE 46: Construction of the Expression Vector Carrying OPS Sulfhydrylase (pCL-Pcj1 System) and Expression in E. coli

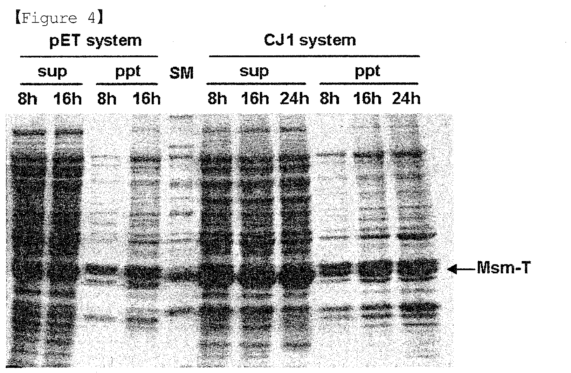

PCR was performed using primers of SEQ ID NOS: 123 and 124, with the pET28a-Msm-T vector serving as a template. The PCR product thus obtained was treated with *EcoR*V and *Hind*III and cloned into pCL-P(CJ1) to construct a recombinant vector named pCL-P(CJ1)-Msm-T. To examine a difference in the expression level of Msm-T between the pET system and the pCL-Pcj1 system, strains for expressing the enzyme were prepared. The pET system was introduced into Rosetta (DE3) while the pCL-Pcjl system used the K12G strain. Single colonies taken from LB plates were inoculated into 5 mL of LB broth and cultured at 37°C for 16 hours while shaking at 200 rpm. These cultures were transferred to 25 mL of fresh LB broth containing kanamycine or spectinomycine and 0.2% glucose (in 250 mL flasks) and incubated to an OD₆₀₀ of 0.5 - 0.6, immediately after which 1 mM IPTG was added to the media to induce the enzymes to be expressed. During incubation at 37°C while shaking at 200 rpm, the expression levels of the enzyme were measured at various culture times (8, 16, 24 hours). The enzyme expression levels of the two systems were analyzed on 14% SDS PAGE (FIG. 4).

### EXAMPLE 47: Cysteine Synthesis by OPS Sulfhydrylase with the Purified OPS Fermentation Broth

The conversion rates from purified OPS to cysteine of Msm-T and Ape-OPSS were determined. In the presence of 75 µg/mL of each of the enzymes and 0.2 mM PLP, 60 mM OPS purified from OPS fermentation broth was reacted with 120 mM Na₂S at 37°C or 70°C for 30, 60, 90, and 120 min. The reaction was conducted only at 37°C for Msm-T, but at both 37°C and 70°C for Ape-OPSS. The amounts of the produced cysteine were measured using the Gaitonde method. As seen in FIG. 5, a purified OPS fermentation broth served well as a substrate for the enzymatic conversion into cysteine. Particularly, the conversion rate of Ape-OPSS was increased at 70°C even upon the use of the purified OPS fermentation broth.

### EXAMPLE 48: Cysteine Synthesis by OPS Sulfhydrylase with the OPS Fermentation Broth

When an OPS fermentation broth was used as a substrate, the cysteine conversion rates of Msm-T and Ape-OPSS were measured according to the concentrations of the enzymes. In the presence of 100 mM Na₂S and 0.2 mM PLP, 50 mM of OPS fermentation broth was reacted with 5 µg/mL or 50 µg/mL of each of Msm-T and Ape-OPSS at 37°C. The amounts of the produced cysteine were measured using the Gaitonde method. As seen in FIG. 6, the highest conversion rate was detected in 50 µg/mL Msm-T. In addition, upon the use of OPS fermentation broth as a substrate, the activity of Msm-T was higher than that of Ape-OPSS.

### EXAMPLE 49: Cysteine Conversion Rate According to OPS Concentration

To examine the effect of OPS concentration on the conversion rate of Msm-T, predetermined amounts of purified OPS were added to OPS fermentation broth to induce the conversion reaction. The enzyme was used in an amount of 50 µg. The amounts of cysteine in the reaction solution were measured using the Gaitonde method. Msm-T exhibited a conversion rate of as high as 100% when the concentration of OPS was about 30 g/L.

When the concentration of OPS exceeded 50 g/L, both the conversion rate and the conversion percentage were found to decrease. From these results, it is understood that when OPS fermentation broth is used as a substrate, there is an optimal concentration ratio between OPS and the enzyme.

**[Table 35]**

| Cysteine Conversion Rate (Msm-T 50 ug) | | | | | | |
|---|---|---|---|---|---|---|
| Time | 0 min | 10 min | 30 min | 60 min | 120 min | 180 min |
| OPS measured 10.65g/l | 0 | 23.03 | 65.38 | 65.70 | 61.95 | 55.35 |
| OPS measured 36.09g/l | 0 | 1.15 | 10.23 | 28.07 | 97.84 | 100.34 |
| OPS measured 55.6g/l | 0 | 0 | 2.36 | 7.41 | 42.69 | 66.67 |

## Claims

1. A method for producing cysteine, comprising:
1) culturing a recombinant microorganism in which the activity of endogeneous phosphoserine phosphatase SerB is reduced, to produce O-phosphoserine (OPS); and
2) reacting the OPS of step 1) with a sulfide in presence of O-phosphoserine sulfhydrylase (OPSS) or with a sulfide in presence of a microorganism expressing OPSS, to produce cysteine, wherein the level of SerB enzymeactivity is reduced by using a technique selected from the group consisting of:
deletion of the chromosomal gene encoding the SerB enzyme,
the introduction of mutation into the chromosomal gene encoding the SerB enzyme to reduce the endogenous gene activity,
the substitution of the chromosomal gene encoding the SerB enzyme with a gene mutated to reduce the endogenous enzyme activity,
the introduction of mutation into a regulatory region for the gene encoding the SerB enzyme to reduce endogenous gene activity, and
the introduction of an antisense oligonucleotide complementary to a transcript of the gene encoding the SerB enzyme to inhibit the translation of the mRNA.

2. The method of claim 1, wherein the phosphoserine phosphatase has an amino acid sequence of SEQ ID NO: 1 or 2.

3. The method of claim 1, wherein the recombinant microorganism in which the activity of endogenous SerB is disrupted is cultured in a medium containing glycine or serine.

4. The method of claim 3, wherein the medium contains glycine in an amount of from 0.1 to 10 g/L.

5. The method of claim 3, wherein the medium contains serine in an amount of from 0.1 to 5 g/L.

6. The method of claim 1, wherein the recombinant microorganism is further modified to enhance the activity of phosphoglycerate dehydrogenase SerA or phosphoserine aminotransferase SerC.

7. The method of claim 6, wherein the SerA is a wild-type or a mutant resistant to serine feedback inhibition.

8. The method of claim 6, wherein:
i) the SerA has one selected from the group consisting of amino acid sequences of SEQ ID NOS: 3 to 7; and
ii) the SerC has an amino acid sequence of SEQ ID NO: 8.

9. The method of claim 1, wherein the recombinant microorganism is further modified to reduce the activity of a PhnCDE operon phnC (ATP-binding component of phosphonate transport, EG 10713)-phnD (periplasmic binding protein component of Pn transporter, EG 10714)-phnE (integral membrane component of the alkylphosphonate ABC transporter, EG 11283).

10. The method of claim 1, wherein the recombinant microorganism is further modified to reduce the activity of alkaline phosphatase PhoA or acid phosphatase AphA.

11. The method of claim 1, wherein the recombinant microorganism is further modified to enhance the activity of nucleotide transhydrogenase PntAB.

12. The method of claim 1, wherein the recombinant microorganism is further modified to enhance the activity of at least one enzyme selected from the group consisting of o-acetylserine/cysteine efflux permease YfiK, homoserine/homoserine lactone efflux protein RhtB, and threonine/homoserine efflux protein RhtC.

13. The method of claim 6, 11 or 12, wherein the the level of enzyme activity is enhanced by using a technique selected from the group consisting of increasing a copy number of a gene encoding the enzyme, introducing a mutation into a regulatory region for the gene to enhance the enzyme activity, substituting the chromosomal gene with a gene mutated to enhance the enzyme activity, and introducing a mutation into the chromosomal gene to enhance the enzyme activity.

14. The method of claim 1, wherein the recombinant microorganism is further modified to reduce the activity of at least one selected from the group consisting of phosphoglycerate mutase isozymes GpmA, GpmI or GpmB.

15. The method of claim 1, wherein the recombinant microorganism is further modified to reduce the activity of L-serine dehydratase I SdaA.

16. The method of claim 1, wherein the recombinant microorganism is further modified to reduce the activity of 2-amino-3-ketobutyrate coenzyme A ligase Kbl or a transcription factor IclR.

17. The method of claim 1, wherein the recombinant microorganism is further modified to enhance the activity of at least one enzyme selected from the group consisting of acetyl-CoA synthetase Acs, acetic acid kinase AckA-phosphotransacetylase Pta, malate synthase G GlcB, malate dehydrogenase MaeB, glutamate dehydrogenase GdhA, glyoxylate carboligase Glc, tartronate semialdehyde reductase 2 GlxR and glycerate kinase II GlxK.

18. The method of claim 17, wherein the recombinant microorganism is improved in sugar consumption and growth by enhancement of the acitivity of at least one enzyme selected from the group consisting of Glc, GlxR, and GlxK.

19. The method of claim 1, wherein the recombinant microorganism is *Escherichia sp.* or *Coryneform bacteria.*

20. The method of claim 1, wherein the sulfide of step 2) is selected from the group consisting of Na₂S, NaSH, (NH₄)₂S, H₂S, Na₂S₂O₃ and a combination thereof.

21. The method of claim 1, wherein the sulfide of step 2) is used at a molar concentration 0.1 to 3 times as high as that of OPS used in the reaction of step 2).

22. The method of claim 1, wherein the OPSS of step 2) is from at least one species selected from the group consisting of *Aeropyrum pernix, Mycobacterium tuberculosis, Mycobacterium smegmatis* and *Trichomonas vaginalis.*

23. The method of claim 22, wherein the OPSS is further modified to increase a conversion rate of step 2).

24. The method of claim 1, wherein the reaction of step 2) is carried out in presence of a cofactor selected from 0.001 ∼ 2 mM PLP (pyridoxal-5-phosphate), 0.001 ∼ 100 mM DTT (dithiothreitol), and a combination thereof.

25. The method of claim 1, further comprising isolating and purifying the cysteine produced in the reaction of step 2).

26. A recombinant microorganism in which the activity of endogenous phosphoserine phosphatase SerB is reduced and which is further modified to enhance the activity of phosphoglycerate dehydrogenase SerA or phosphoserine aminotransferase SerC, wherein the SerA is resistant to serine feedback inhibition.

27. The recombinant microorganism of claim 26, which is further modified to reduce the activity of at least one selected from among PhnCDE, PhoA, and AphA.

28. The recombinant microorganism of claim 26, which is deposited under accession No. KCCM11103P.

29. A method for producing N-acetylcysteine or S-carboxymethylcysteine, comprising:
1) culturing a recombinant microorganism in which the activity of endogeneous phosphoserine phosphatase SerB is reduced, to produce O-phosphoserine (OPS);
2) reacting the OPS of step 1) with a sulfide in presence of O-phosphoserine sulfhydrylase (OPSS) or with a sulfide in presence of a microorganism expressing OPSS, to produce cysteine,and
3.) synethesizing N-acetylcysteine or S-carboxymethylcysteine from the cysteine produced in step 2), wherein the level of SerB enzyme activity is reduced by using a technique selected from the group consisting of:
deletion of the chromosomal gene encoding the SerB enzyme,
the introduction of mutation into the chromosomal gene encoding the SerB enzyme to reduce the endogenous gene activity,
the substitution of the chromosomal gene encoding the SerB enzyme with a gene mutated to reduce the endogenous enzyme activity,
the introduction of mutation into a regulatory region for the gene encoding the SerB enzyme to reduce endogenous gene activity, and
the introduction of an antisense oligonucleotide complementary to a transcript of the gene encoding the SerB enzyme to inhibit the translation of the mRNA.

30. The method of claim 29, further comprising isolating and purifying said N-acetylcysteine or S-carboxymethylcysteine produced in the reaction of step 3).

## Patentansprüche

1. Verfahren zur Herstellung von Cystein, umfassend:
1) Züchten eines rekombinanten Mikroorganismus, in dem die Aktivität der endogenen Phosphoserin-Phosphatase SerB herabgesetzt ist, um O-Phosphoserin (OPS) herzustellen; und
2) Zur-Reaktion-Bringen von OPS aus Schritt 1) mit einem Sulfid in Anwesenheit von O-Phosphoserin-Sulfhydrylase (OPSS) oder mit einem Sulfid in Anwesenheit eines Mikroorganismus, der OPSS exprimiert, um Cystein herzustellen, wobei der Grad der Aktivität des Enzyms SerB herabgesetzt ist durch die Verwendung einer Technik, die ausgewählt ist aus der Gruppe bestehend aus:
Deletion des chromosomalen Gens, das das Enzym SerB codiert,
Einführen einer Mutation in das chromosomale Gen, das das Enzym SerB codiert, um die Aktivität des endogenen Gens herabzusetzen,
Substitution des chromosomalen Gens, das das Enzym SerB codiert, durch ein Gen, das mutiert ist, um die Aktivität des endogenen Gens herabzusetzen,
Einführen einer Mutation in eine für das Gen, das das Enzym SerB codiert, regulatorische Region, um die endogene Enzymaktivität herabzusetzen, und
Einführen eines Antisense-Oligonucleotids, das zum Transkript des Gens komplementär ist, das das Enzym SerB codiert, um die Translation der mRNA zu inhibieren.

2. Verfahren nach Anspruch 1, wobei die Phosphoserin-Phosphatase eine Aminosäuresequenz von SEQ ID NO:1 oder 2 aufweist.

3. Verfahren nach Anspruch 1, wobei der rekombinante Mirkoorganismus, in dem die Aktivität des exogenen SerB unterbrochen wird, in einem Medium, das Glycin oder Serin enthält, gezüchtet wird.

4. Verfahren nach Anspruch 3, wobei das Medium Glycin in einer Menge von 0,1 bis 10 g/l enthält.

5. Verfahren nach Anspruch 3, wobei das Medium Serin in einer Menge von 0,1 bis 5 g/l enthält.

6. Verfahren nach Anspruch 1, wobei der rekombinante Mikroorganismus des Weiteren modifiziert ist, um die Aktivität der Phosphoglycerat-Dehydrogenase SerA oder der Phosphoserin-Aminotransferase SerC zu verstärken.

7. Verfahren nach Anspruch 6, wobei SerA ein Wildtyp oder eine Mutante ist, die gegenüber Serin-Feedback-Inhibierung resistent ist.

8. Verfahren nach Anspruch 6, wobei:
i) SerA eine Aminosäuresequenz ausgewählt aus der Gruppe bestehend aus den Aminosäuresequenzen der SEQ ID NOs:3 bis 7 aufweist; und
ii) SerC eine Aminosäuresequenz der SEQ ID NO:8 aufweist.

9. Verfahren nach Anspruch 1, wobei der rekombinante Mikroorganismus des Weiteren modifiziert ist, um die Aktivität eines PhnCDE-Operons phnC (ATPbindende Komponente des Phosphonat-Transports, EG 10713)-phnD (periplasmatische Bindungsproteinkomponente des Pn-Transporters, EG 10714)-phnE (integrale Membrankomponente des Alkylphosphonat ABC-Transporters, EG 11283).

10. Verfahren nach Anspruch 1, wobei der rekombinante Mikroorganismus des Weiteren modifiziert ist, um die Aktivität der alkalischen Phosphatase PhoA oder der sauren Phosphatase AphA herabzusetzen.

11. Verfahren nach Anspruch 1, wobei der rekombinante Mikroorganismus des Weiteren modifiziert ist, um die Aktivität der Nucleotid-Transhydrogenase PntAB zu verstärken.

12. Verfahren nach Anspruch 1, wobei der rekombinante Mikroorganismus des Weiteren modifiziert ist, um die Aktivität mindestens eines Enzyms zu verstärken, das ausgewählt ist aus der Gruppe bestehend aus O-Acetylserin/Cystein-Efflux-Permease YfiK, Homoserin/Homoserin-Lacton-Efflux-Protein RhtB und Threonin/Homoserin-Efflux-Protein RhtC.

13. Verfahren nach Anspruch 6, 11 oder 12, wobei der Grad der Enzymaktivität verstärkt ist durch Verwendung einer Technik, die ausgewählt ist aus der Gruppe bestehend aus der Erhöhung der Kopienzahl eines das Enzym codierenden Gens, dem Einführen einer Mutation in eine für das Gen regulatorische Region zur Verstärkung der Enzymaktivität, Substituieren des chromosomalen Gens durch ein mutiertes Gen zur Verstärkung der Enzymaktivität und Einführen einer Mutation in das chromosomale Gen zur Verstärkung der Enzymaktivität.

14. Verfahren nach Anspruch 1, wobei der rekombinante Mikroorganismus des Weiteren modifiziert ist, um die Aktivität mindestens eines Isozyms herabzusetzen, das aus der Gruppe bestehend aus den Phosphoglyceratmutase-Isozymen GpmA, Gpml oder GpmB ausgewählt ist.

15. Verfahren nach Anspruch 1, wobei der rekombinante Mikroorganismus des Weiteren modifiziert ist, um die Aktivität der L-Serindehydratase I SdaA herabzusetzen.

16. Verfahren nach Anspruch 1, wobei der rekombinante Mikroorganismus des Weiteren modifiziert ist, um die Aktivität der 2-Amino-3-ketobutyrat-Coenzym A-Ligase Kbl oder eines Transkriptionsfaktors IcIR herabzusetzen.

17. Verfahren nach Anspruch 1, wobei der rekombinante Mikroorganismus des Weiteren modifiziert ist, um die Aktivität mindestens eines Enzyms zu verstärken, das ausgewählt ist aus der Gruppe bestehend aus Acetyl-CoA-Synthase Acs, Acetokinase AckA-Phosphotransacetylase Pta, Malat-Synthase G GIcB, Malat-Dehydrogenase MaeB, Glutamatdehydrogenase GdhA, Glyoxylat-Carboligase Glc, Tartronat-Semialdehyd-Reduktase 2 GlxR und Glyceratkinase II GlxK.

18. Verfahren nach Anspruch 17, wobei der rekombinante Mikroorganismus bezuüglich Zuckerverbrauch und Wachstum durch Verstärkung der Aktivität mindestens eines Enzyms ausgewählt aus der Gruppe bestehend aus Glc, GlxR und GlxK verbessert ist.

19. Verfahren nach Anspruch 1, wobei der rekombinante Mikroorganismus *Escherichia sp.* oder ein coryneformes Bakterium ist.

20. Verfahren nach Anspruch 1, wobei das Sulfid aus Schritt 2) ausgewählt ist aus der Gruppe bestehend aus Na₂S, NaSH, (NH₄)₂S, H₂S, Na₂S₂O₃ und einer Kombination davon.

21. Verfahren nach Anspruch 1, wobei das Sulfid aus Schritt 2) in einer molaren Konzentration von 0,1 bis 3-mal so hoch wie die von OPS, das in der Reaktion in Schritt 2) verwendet wird, ist.

22. Verfahren nach Anspruch 1, wobei OPSS aus Schritt 2) von mindestens einer Spezies stammt, die ausgewählt ist aus der Gruppe bestehend aus *Aeropyrum pernix, Mycobacterium tuberculosis, Mycobacterium smegmatis* und *Trichomonas vaginalis.*

23. Verfahren nach Anspruch 22, wobei OPSS des Weiteren modifiziert ist, um die Umsetzungsrate in Schritt 2) zu erhöhen.

24. Verfahren nach Anspruch 1, wobei die Reaktion in Schritt 2) in Anwesenheit eines Kofaktors durchgeführt wird, der ausgewählt ist aus 0,001 ∼ 2 mM PLP (Pyridoxal-5-phosphat), 0,001 ∼ 100 mM DTT (Dithiothreitol) und einer Kombination davon.

25. Verfahren nach Anspruch 1, das des Weiteren das Isolieren und Aufreinigen des in der Reaktion von Schritt 2) hergestellten Cysteins umfasst.

26. Rekombinanter Mikroorganismus, in dem die Aktivität der endogenen Phophoserinphosphatase SerB herabgesetzt ist und der des Weiteren modifiziert ist, um die Aktivität der Phosphoglycerat-Dehydrogenase SerA oder der Phosphoserin-Aminotransferase SerC zu verstärken, wobei SerA gegenüber Serin-Feedback-Inhibition resistent ist.

27. Rekombinanter Mikroorganismus nach Anspruch 26, der des Weiteren modifiziert ist, um die Aktivität mindestens eines ausgewählt aus PhnCDE, PhoA und AphA herabzusetzen.

28. Rekombinanter Mikroorganismus nach Anspruch 26, der unter der Hinterlegungsnummer KCCM11103P hinterlegt ist.

29. Verfahren zur Herstellung von N-Acetylcystein oder S-Carboxymethylcystein, umfassend:
1) Züchten eines rekombinanten Mikroorganismus, in dem die Aktivität endogener Phosphoserin-Phosphatase SerB herabgesetzt ist, um O-Phosphoserin (OPS) herzustellen;
2) Zur-Reaktion-Bringen von OPS aus Schritt 1) mit einem Sulfid in Anwesenheit von O-Phosphoserin-Sulfhydrylase (OPSS) oder mit einem Sulfid in Anwesenheit eines Mikroorganismus, der OPSS exprimiert, um Cystein herzustellen, und
3) Synthese von N-Acetylcystein oder S-Carboxymethyl-Cystein aus dem in Schritt 2) hergestellten Cystein, wobei der Grad der Aktivität des Enzyms SerB durch Verwendung einer Technik herabgesetzt wird, die ausgewählt ist aus der Gruppe bestehend aus:
Deletion des chromosomalen Gens, das das Enzym SerB codiert,
Einführen einer Mutation in das chromosomale Gen, das das Enzym SerB codiert, um die Aktivität des endogenen Gens herabzusetzen,
Substitution des chromosomalen Gens, das das Enzym SerB codiert, durch ein Gen, das mutiert ist, um die Aktivität des endogenen Gens herabzusetzen,
Einführen einer Mutation in eine für das Gen, das das Enzym SerB codiert, regulatorische Region, um die Aktivität des endogenen Gens herabzusetzen, und
Einführen eines Antisense-Oligonucleotids, das zu einem Transkript des Gens, das das Enzym SerB codiert, komplementär ist, um die Translation der mRNA zu inhibieren.

30. Verfahren nach Anspruch 29, das des Weiteren das Isolieren und das Aufreinigen des in der Reaktion in Schritt 3) hergestellten N-Acetylcysteins oder S-Carboxymethyl-Cysteins umfasst.

## Revendications

1. Méthode pour produire de la cystéine, comprenant :
1) la culture d'un micro-organisme recombinant dans lequel l'activité de la phosphosérine phosphatase endogène SerB est réduite, pour produire de la O-phosphosérine (OPS) ; et
2) la réaction de l'OPS de l'étape 1) avec un sulfure en présence de O-phosphosérine sulfhydrylase (OPSS) ou avec un sulfure en présence d'un micro-organisme exprimant l'OPSS, pour produire de la cystéine, dans laquelle le niveau d'activité de l'enzyme SerB est réduit en utilisant une technique sélectionnée parmi le groupe constitué par :
la délétion du gène chromosomique codant pour l'enzyme SerB,
l'introduction de mutation dans le gène chromosomique codant pour l'enzyme SerB pour réduire l'activité du gène endogène,
la substitution du gène chromosomique codant pour l'enzyme SerB par un gène muté pour réduire l'activité de l'enzyme endogène,
l'introduction de mutation dans une région régulatrice du gène codant pour l'enzyme SerB pour réduire l'activité du gène endogène, et
l'introduction d'un oligonucléotide antisens complémentaire d'un transcrit du gène codant pour l'enzyme SerB pour inhiber la traduction de l'ARNm.

2. Méthode selon la revendication 1, dans laquelle la phosphosérine phosphatase a une séquence d'acides aminés de SEQ ID No : 1 ou 2.

3. Méthode selon la revendication 1, dans laquelle le micro-organisme recombinant dans lequel l'activité de la SerB endogène est réduite est cultivé dans un milieu contenant de la glycine ou de la sérine.

4. Méthode selon la revendication 3, dans laquelle le milieu contient de la glycine en une quantité de 0,1 à 10 g/L.

5. Méthode selon la revendication 3, dans laquelle le milieu contient de la sérine en une quantité de 0,1 à 5 g/L.

6. Méthode selon la revendication 1, dans laquelle le micro-organisme recombinant est en outre modifié pour augmenter l'activité de la phosphoglycérate déshydrogénase SerA ou de la phosphosérine aminotransférase SerC.

7. Méthode selon la revendication 6, dans laquelle la SerA est de type sauvage ou un mutant résistant à la rétro-inhibition de la sérine.

8. Méthode selon la revendication 6, dans laquelle :
i) la SerA a une séquence d'acides aminés sélectionnée parmi le groupe constitué par les séquences d'acides aminés de SEQ ID Nos : 3 à 7 ; et
ii) la SerC a une séquence d'acides aminés de SEQ ID No : 8.

9. Méthode selon la revendication 1, dans laquelle le micro-organisme recombinant est en outre modifié pour réduire l'activité d'un opéron PhnCDE phnC (composant de transport du phosphonate se liant à l'ATP, EG 10713)-phnD (composant protéique du transporteur de Pn se liant au périplasme, EG 10714)-phnE (composant membranaire intégral du transporteur ABC d'alkylphosphonate, EG 11283).

10. Méthode selon la revendication 1, dans laquelle le micro-organisme recombinant est en outre modifié pour réduire l'activité de la phosphatase alcaline PhoA ou de la phosphatase acide AphA.

11. Méthode selon la revendication 1, dans laquelle le micro-organisme recombinant est en outre modifié pour augmenter l'activité de la nucléotide transhydrogénase PntAB.

12. Méthode selon la revendication 1, dans laquelle le micro-organisme recombinant est en outre modifié pour augmenter l'activité d'au moins une enzyme sélectionnée parmi le groupe constitué par la perméase d'efflux acétylsérine/cystéine YfiK, la protéine d'efflux homosérine/homosérine lactone RhtB, et la protéine d'efflux thréonine/homosérine RhtC.

13. Méthode selon la revendication 6, 11 ou 12, dans laquelle le niveau d'activité de l'enzyme est augmenter en utilisant une technique sélectionnée parmi le groupe constitué par l'accroissement du nombre de copies d'un gène codant pour l'enzyme, l'introduction d'une mutation dans une région régulatrice du gène pour augmenter l'activité de l'enzyme, la substitution du gène chromosomique par un gène muté pour augmenter l'activité de l'enzyme, et l'introduction d'une mutation dans le gène chromosomique pour augmenter l'activité de l'enzyme.

14. Méthode selon la revendication 1, dans laquelle le micro-organisme recombinant est en outre modifié pour réduire l'activité d'au moins une enzyme sélectionnée parmi le groupe constitué par les isozymes GpmA, GpmI ou GpmB de la phosphoglycérate mutase.

15. Méthode selon la revendication 1, dans laquelle le micro-organisme recombinant est en outre modifié pour réduire l'activité de la L-sérine déshydratase I SdaA.

16. Méthode selon la revendication 1, dans laquelle le micro-organisme recombinant est en outre modifié pour réduire l'activité de la 2-amino-3-cétobutyrate coenzyme A ligase Kb1 ou d'un facteur de transcription Ic1R.

17. Méthode selon la revendication 1, dans laquelle le micro-organisme recombinant est en outre modifié pour augmenter l'activité d'au moins une enzyme sélectionnée parmi le groupe constitué par l'acétyl-CoA synthétase Acs, l'acide acétique kinase AckA-phosphotransacétylase Pta, la malate synthase G GlcB, la malate déshydrogénase MaeB, la glutamate déshydrogénase GdhA, la glyoxylate carboligase Glc, la tartronate semialdéhyde réductase 2 GlxR et la glycérate kinase II GlxK.

18. Méthode selon la revendication 17, dans laquelle le micro-organisme recombinant est amélioré en termes de consommation de sucre et de croissance en augmentant l'activité d'au moins une enzyme sélectionnée parmi le groupe constitué par Glc, GlxR, et GlxK.

19. Méthode selon la revendication 1, dans laquelle le micro-organisme recombinant est une bactérie *Escherichia sp.* ou *Coryneforme.*

20. Méthode selon la revendication 1, dans laquelle le sulfure de l'étape 2) est sélectionné parmi le groupe constitué par Na₂S, NaSH, (NH₄)₂S, H₂S, Na₂S₂O₃ et une combinaison de ceux-ci.

21. Méthode selon la revendication 1, dans laquelle le sulfure de l'étape 2) est utilisé à une concentration molaire de 0,1 à 3 fois celle de l'OPS utilisée dans la réaction de l'étape 2).

22. Méthode selon la revendication 1, dans laquelle l'OPSS de l'étape 2) provient d'au moins une espèce choisie dans le groupe constitué par *Aeropyrum pernix, Mycobacterium tuberculosis, Mycobacterium smegmatis* et *Trichomonas vaginalis.*

23. Méthode selon la revendication 22, dans laquelle l'OPSS est en outre modifiée pour accroître le taux de conversion de l'étape 2).

24. Méthode selon la revendication 1, dans laquelle la réaction de l'étape 2) est mise en oeuvre en présence d'un cofacteur sélectionné parmi 0,001∼2 mM de PLP (pyridoxal-5-phosphate), 0,001∼100 mM de DTT (dithiothréitol), et une combinaison de ceux-ci.

25. Méthode selon la revendication 1, comprenant en outre l'isolement et la purification de la cystéine produite dans la réaction de l'étape 2).

26. Micro-organisme recombinant dans lequel l'activité de la phosphosérine phosphatase endogène SerB est réduite et qui est en outre modifié pour augmenter l'activité de la phosphoglycérate déshydrogénase SerA ou de la phosphosérine aminotransférase SerC, dans lequel la SerA est résistante à la rétro-inhibition de la sérine.

27. Micro-organisme recombinant selon la revendication 26, qui est en outre modifié pour réduire l'activité d'au moins un sélectionné parmi PhnCDE, PhoA, et AphA.

28. Micro-organisme recombinant selon la revendication 26, qui est déposé sous le No. d'accès KCCM11103P.

29. Méthode de production de N-acétylcystéine ou de S-carboxy-méthylcystéine, comprenant :
1) la culture d'un micro-organisme recombinant chez lequel l'activité de la phosphosérine phosphatase endogène SerB est réduite, pour produire de la O-phosphosérine (OPS) ;
2) la réaction de l'OPS de l'étape 1) avec un sulfure en présence de O-phosphosérine sulfhydrylase (OPSS) ou avec un sulfure en présence d'un micro-organisme exprimant l'OPSS, pour produire de la cystéine, et
3) la synthèse de la N-acétylcystéine ou de la S-carboxyméthylcystéine à partir de la cystéine produite à l'étape 2),
dans laquelle le niveau d'activité de l'enzyme SerB est réduit en utilisant une technique choisie dans le groupe constitué par :
la délétion du gène chromosomique codant pour l'enzyme SerB,
l'introduction de mutation dans le gène chromosomique codant pour l'enzyme SerB pour réduire l'activité du gène endogène,
la substitution du gène chromosomique codant pour l'enzyme SerB par un gène muté pour réduire l'activité de l'enzyme endogène,
l'introduction de mutation dans une région régulatrice du gène codant pour l'enzyme SerB pour réduire l'activité du gène endogène, et
l'introduction d'un oligonucléotide antisens complémentaire d'un transcrit du gène codant pour l'enzyme SerB pour inhiber la traduction de l'ARNm.

30. Méthode selon la revendication 29, comprenant en outre l'isolement et la purification de ladite N-acétylcystéine ou S-carboxyméthylcystéine produite dans la réaction de l'étape 3).


<110> CJ CheilJedang Corporation
<120> MICROORGANISM PRODUCING O-PHOSPHOSERINE AND METHOD OF PRODUCING L-CYSTEINE OR DERIVATIVES THEREOF FROM O-PHOSPHOSERINE USING THE SAME
<130> T3048 EP S3
<150> KR10-2010-0102664
   <151> 2010-10-20
<150> KR10-2011-0086081
   <151> 2011-08-26
<160> 124
<170> KopatentIn 1.71
<210> 1
   <211> 446
   <212> PRT
   <213> Corynebacterium glutamicum 13032
<220>
   <221> PEPTIDE
   <222> (1)..(446)
   <223> phosphoserine phosphatase, SerB
<400> 1
   Met Ser Cys Ser Ala Leu Arg His Glu Thr Ile Val Ala Val Thr Glu
   1 5 10 15
   Leu Ile Gln Asn Glu Ser Gln Glu Ile Ala Glu Leu Glu Ala Gly Gln
   20 25 30
   Gln Val Ala Leu Arg Glu Gly Tyr Leu Pro Ala Val Ile Thr Val Ser
   35 40 45
   Gly Lys Asp Arg Pro Gly Val Thr Ala Ala Phe Phe Arg Val Leu Ser
   50 55 60
   Ala Asn Gln Val Gln Val Leu Asp Val Glu Gln Ser Met Phe Arg Gly
   65 70 75 80
   Phe Leu Asn Leu Ala Ala Phe Val Gly Ile Ala Pro Glu Arg Val Glu
   85 90 95
   Thr Val Thr Thr Gly Leu Thr Asp Thr Leu Lys Val His Gly Gln Ser
   100 105 110
   Val Val Val Glu Leu Gln Glu Thr Val Gln Ser Ser Arg Pro Arg Ser
   115 120 125
   Ser His Val Val Val Val Leu Gly Asp Pro Val Asp Ala Leu Asp Ile
   130 135 140
   Ser Arg Ile Gly Gln Thr Leu Ala Asp Tyr Asp Ala Asn Ile Asp Thr
   145 150 155 160
   Ile Arg Gly Ile Ser Asp Tyr Pro Val Thr Gly Leu Glu Leu Lys Val
   165 170 175
   Thr Val Pro Asp Val Ser Pro Gly Gly Gly Glu Ala Met Arg Lys Ala
   180 185 190
   Leu Ala Ala Leu Thr Ser Glu Leu Asn Val Asp Ile Ala Ile Glu Arg
   195 200 205
   Ser Gly Leu Leu Arg Arg Ser Lys Arg Leu Val Cys Phe Asp Cys Asp
   210 215 220
   Ser Thr Leu Ile Thr Gly Glu Val Ile Glu Met Leu Ala Ala His Ala
   225 230 235 240
   Gly Lys Glu Ala Glu Val Ala Ala Val Thr Glu Arg Ala Met Arg Gly
   245 250 255
   Glu Leu Asp Phe Glu Glu Ser Leu Arg Glu Arg Val Lys Ala Leu Ala
   260 265 270
   Gly Leu Asp Ala Ser Val Ile Asp Glu Val Ala Ala Ala Ile Glu Leu
   275 280 285
   Thr Pro Gly Ala Arg Thr Thr Ile Arg Thr Leu Asn Arg Met Gly Tyr
   290 295 300
   Gln Thr Ala Val Val Ser Gly Gly Phe Ile Gln Val Leu Glu Gly Leu
   305 310 315 320
   Ala Glu Glu Leu Glu Leu Asp Tyr Val Arg Ala Asn Thr Leu Glu Ile
   325 330 335
   Val Asp Gly Lys Leu Thr Gly Asn Val Thr Gly Lys Ile Val Asp Arg
   340 345 350
   Ala Ala Lys Ala Glu Phe Leu Arg Glu Phe Ala Ala Asp Ser Gly Leu
   355 360 365
   Lys Met Tyr Gln Thr Val Ala Val Gly Asp Gly Ala Asn Asp Ile Asp
   370 375 380
   Met Leu Ser Ala Ala Gly Leu Gly Val Ala Phe Asn Ala Lys Pro Ala
   385 390 395 400
   Leu Lys Glu Ile Ala Asp Thr Ser Val Asn His Pro Phe Leu Asp Glu
   405 410 415
   Val Leu His Ile Met Gly Ile Ser Arg Asp Glu Ile Asp Leu Ala Asp
   420 425 430
   Gln Glu Asp Gly Thr Phe His Arg Val Pro Leu Thr Asn Ala
   435 440 445
<210> 2
   <211> 322
   <212> PRT
   <213> Escherichia coli K12 W3110
<220>
   <221> PEPTIDE
   <222> (1)..(322)
   <223> phosphoserine phosphatase, SerB
<400> 2
<210> 3
   <211> 530
   <212> PRT
   <213> Artificial Sequence
<220>
   <223> D-3-phosphoglycerate dehydrogenase(E235K), SerA(E235K)
<400> 3
   Met Ser Gln Asn Gly Arg Pro Val Val Leu Ile Ala Asp Lys Leu Ala
    1               5                   10                  15
   Gln Ser Thr Val Asp Ala Leu Gly Asp Ala Val Glu Val Arg Trp Val
               20                  25                  30
   Asp Gly Pro Asn Arg Pro Glu Leu Leu Asp Ala Val Lys Glu Ala Asp
           35                  40                  45
   Ala Leu Leu Val Arg Ser Ala Thr Thr Val Asp Ala Glu Val Ile Ala
       50                  55                  60
   Ala Ala Pro Asn Leu Lys Ile Val Gly Arg Ala Gly Val Gly Leu Asp
   65                  70                  75                  80
   Asn Val Asp Ile Pro Ala Ala Thr Glu Ala Gly Val Met Val Ala Asn
                   85                  90                  95
   Ala Pro Thr Ser Asn Ile His Ser Ala Cys Glu His Ala Ile Ser Leu
               100                 105                 110
   Leu Leu Ser Thr Ala Arg Gln Ile Pro Ala Ala Asp Ala Thr Leu Arg
           115                 120                 125
   Glu Gly Glu Trp Lys Arg Ser Ser Phe Asn Gly Val Glu Ile Phe Gly
       130                 135                 140
   Lys Thr Val Gly Ile Val Gly Phe Gly His Ile Gly Gln Leu Phe Ala
   145                 150                 155                 160
   Gln Arg Leu Ala Ala Phe Glu Thr Thr Ile Val Ala Tyr Asp Pro Tyr
                   165                 170                 175
   Ala Asn Pro Ala Arg Ala Ala Gln Leu Asn Val Glu Leu Val Glu Leu
               180                 185                 190
   Asp Glu Leu Met Ser Arg Ser Asp Phe Val Thr Ile His Leu Pro Lys
           195                 200                 205
   Thr Lys Glu Thr Ala Gly Met Phe Asp Ala Gln Leu Leu Ala Lys Ser
       210                 215                 220
   Lys Lys Gly Gln Ile Ile Ile Asn Ala Ala Arg Gly Gly Leu Val Asp
   225                 230                 235                 240
   Glu Gln Ala Leu Ala Asp Ala Ile Glu Ser Gly His Ile Arg Gly Ala
                   245                 250                 255
   Gly Phe Asp Val Tyr Ser Thr Glu Pro Cys Thr Asp Ser Pro Leu Phe
               260                 265                 270
   Lys Leu Pro Gln Val Val Val Thr Pro His Leu Gly Ala Ser Thr Glu
           275                 280                 285
   Glu Ala Gln Asp Arg Ala Gly Thr Asp Val Ala Asp Ser Val Leu Lys
       290                 295                 300
   Ala Leu Ala Gly Glu Phe Val Ala Asp Ala Val Asn Val Ser Gly Gly
   305                 310                 315                 320
   Arg Val Gly Glu Lys Val Ala Val Trp Met Asp Leu Ala Arg Lys Leu
                   325                 330                 335
   Gly Leu Leu Ala Gly Lys Leu Val Asp Ala Ala Pro Val Ser Ile Glu
               340                 345                 350
   Val Glu Ala Arg Gly Glu Leu Ser Ser Glu Gln Val Asp Ala Leu Gly
           355                 360                 365
   Leu Ser Ala Val Arg Gly Leu Phe Ser Gly Ile Ile Glu Glu Ser Val
       370                 375                 380
   Thr Phe Val Asn Ala Pro Arg Ile Ala Glu Glu Arg Gly Leu Asp Ile
   385                 390                 395                 400
   Ser Val Lys Thr Asn Ser Glu Ser Val Thr His Arg Ser Val Leu Gln
                   405                 410                 415
   Val Lys Val Ile Thr Gly Ser Gly Ala Ser Ala Thr Val Val Gly Ala
               420                 425                 430
   Leu Thr Gly Leu Glu Arg Val Glu Lys Ile Thr Arg Ile Asn Gly Arg
           435                 440                 445
   Gly Leu Asp Leu Arg Ala Glu Gly Leu Asn Leu Phe Leu Gln Tyr Thr
       450                 455                 460
   Asp Ala Pro Gly Ala Leu Gly Thr Val Gly Thr Lys Leu Gly Ala Ala
   465                 470                 475                 480
   Gly Ile Asn Ile Glu Ala Ala Ala Leu Thr Gln Ala Glu Lys Gly Asp
                   485                 490                 495
   Gly Ala Val Leu Ile Leu Arg Val Glu Ser Ala Val Ser Glu Glu Leu
               500                 505                 510
   Glu Ala Glu Ile Asn Ala Glu Leu Gly Ala Thr Ser Phe Gln Val Asp
   515 520 525
   Leu Asp
   530
<210> 4
   <211> 333
   <212> PRT
   <213> Artificial Sequence
<220>
   <223> D-3-phosphoglycerate dehydrogenase(197 delta), SerA(197 delta)
<400> 4
   Met Ser Gln Asn Gly Arg Pro Val Val Leu Ile Ala Asp Lys Leu Ala
    1               5                  10                  15
   Gln Ser Thr Val Asp Ala Leu Gly Asp Ala Val Glu Val Arg Trp Val
               20                  25                  30
   Asp Gly Pro Asn Arg Pro Glu Leu Leu Asp Ala Val Lys Glu Ala Asp
           35                  40                  45
   Ala Leu Leu Val Arg Ser Ala Thr Thr Val Asp Ala Glu Val Ile Ala
       50                  55                  60
   Ala Ala Pro Asn Leu Lys Ile Val Gly Arg Ala Gly Val Gly Leu Asp
   65                  70                  75                  80
   Asn Val Asp Ile Pro Ala Ala Thr Glu Ala Gly Val Met Val Ala Asn
                   85                  90                  95
   Ala Pro Thr Ser Asn Ile His Ser Ala Cys Glu His Ala Ile Ser Leu
               100                 105                 110
   Leu Leu Ser Thr Ala Arg Gln Ile Pro Ala Ala Asp Ala Thr Leu Arg
           115                 120                 125
   Glu Gly Glu Trp Lys Arg Ser Ser Phe Asn Gly Val Glu Ile Phe Gly
       130                 135                 140
   Lys Thr Val Gly Ile Val Gly Phe Gly His Ile Gly Gln Leu Phe Ala
   145                 150                 155                 160
   Gln Arg Leu Ala Ala Phe Glu Thr Thr Ile Val Ala Tyr Asp Pro Tyr
                   165                 170                 175
   Ala Asn Pro Ala Arg Ala Ala Gln Leu Asn Val Glu Leu Val Glu Leu
               180                 185                 190
   Asp Glu Leu Met Ser Arg Ser Asp Phe Val Thr Ile His Leu Pro Lys
           195                 200                 205
   Thr Lys Glu Thr Ala Gly Met Phe Asp Ala Gln Leu Leu Ala Lys Ser
       210                 215                 220
   Lys Lys Gly Gln Ile Ile Ile Asn Ala Ala Arg Gly Gly Leu Val Asp
   225                 230                 235                 240
   Glu Gln Ala Leu Ala Asp Ala Ile Glu Ser Gly His Ile Arg Gly Ala
                   245                 250                 255
   Gly Phe Asp Val Tyr Ser Thr Glu Pro Cys Thr Asp Ser Pro Leu Phe
               260                 265                 270
   Lys Leu Pro Gln Val Val Val Thr Pro His Leu Gly Ala Ser Thr Glu
           275                 280                 285
   Glu Ala Gln Asp Arg Ala Gly Thr Asp Val Ala Asp Ser Val Leu Lys
       290                 295                 300
   Ala Leu Ala Gly Glu Phe Val Ala Asp Ala Val Asn Val Ser Gly Gly
   305                 310                 315                 320
   Arg Val Gly Glu Glu Val Ala Val Trp Met Asp Leu Ala
                   325                 330
<210> 5
   <211> 410
   <212> PRT
   <213> Artificial Sequence
<220>
   <223> D-3-phosphoglycerate dehydrogenase(G336V), SerA(G336V)
<400> 5
<210> 6
   <211> 410
   <212> PRT
   <213> Artificial Sequence
<220>
   <223> D-3-phosphoglycerate dehydrogenase(G336V, G337V), SerA(G336V, G337V)
<400> 6
   Met Ala Lys Val Ser Leu Glu Lys Asp Lys Ile Lys Phe Leu Leu Val
     1               5                  10                  15
   Glu Gly Val His Gln Lys Ala Leu Glu Ser Leu Arg Ala Ala Gly Tyr
                20                  25                  30
   Thr Asn Ile Glu Phe His Lys Gly Ala Leu Asp Asp Glu Gln Leu Lys
            35                  40                  45
   Glu Ser Ile Arg Asp Ala His Phe Ile Gly Leu Arg Ser Arg Thr His
        50                  55                  60
   Leu Thr Glu Asp Val Ile Asn Ala Ala Glu Lys Leu Val Ala Ile Gly
    65                  70                  75                  80
   Cys Phe Cys Ile Gly Thr Asn Gln Val Asp Leu Asp Ala Ala Ala Lys
                    85                  90                  95
   Arg Gly Ile Pro Val Phe Asn Ala Pro Phe Ser Asn Thr Arg Ser Val
               100                 105                 110
   Ala Glu Leu Val Ile Gly Glu Leu Leu Leu Leu Leu Arg Gly Val Pro
           115                 120                 125
   Glu Ala Asn Ala Lys Ala His Arg Gly Val Trp Asn Lys Leu Ala Ala
       130                 135                 140
   Gly Ser Phe Glu Ala Arg Gly Lys Lys Leu Gly Ile Ile Gly Tyr Gly
   145                 150                 155                 160
   His Ile Gly Thr Gln Leu Gly Ile Leu Ala Glu Ser Leu Gly Met Tyr
                   165                 170                 175
   Val Tyr Phe Tyr Asp Ile Glu Asn Lys Leu Pro Leu Gly Asn Ala Thr
               180                 185                 190
   Gln Val Gln His Leu Ser Asp Leu Leu Asn Met Ser Asp Val Val Ser
           195                 200                 205
   Leu His Val Pro Glu Asn Pro Ser Thr Lys Asn Met Met Gly Ala Lys
       210                 215                 220
   Glu Ile Ser Leu Met Lys Pro Gly Ser Leu Leu Ile Asn Ala Ser Arg
   225                 230                 235                 240
   Gly Thr Val Val Asp Ile Pro Ala Leu Cys Asp Ala Leu Ala Ser Lys
                   245                 250                 255
   His Leu Ala Gly Ala Ala Ile Asp Val Phe Pro Thr Glu Pro Ala Thr
               260                 265                 270
   Asn Ser Asp Pro Phe Thr Ser Pro Leu Cys Glu Phe Asp Asn Val Leu
           275                 280                 285
   Leu Thr Pro His Ile Gly Gly Ser Thr Gln Glu Ala Gln Glu Asn Ile
       290                 295                 300
   Gly Leu Glu Val Ala Gly Lys Leu Ile Lys Tyr Ser Asp Asn Gly Ser
   305                 310                 315                 320
   Thr Leu Ser Ala Val Asn Phe Pro Glu Val Ser Leu Pro Leu His Val
                   325                 330                 335
   Val Arg Arg Leu Met His Ile His Glu Asn Arg Pro Gly Val Leu Thr
               340                 345                 350
   Ala Leu Asn Lys Ile Phe Ala Glu Gln Gly Val Asn Ile Ala Ala Gln
           355                 360                 365
   Tyr Leu Gln Thr Ser Ala Gln Met Gly Tyr Val Val Ile Asp Ile Glu
       370                 375                 380
   Ala Asp Glu Asp Val Ala Glu Lys Ala Leu Gln Ala Met Lys Ala Ile
   385                 390                 395                 400
   Pro Gly Thr Ile Arg Ala Arg Leu Leu Tyr
                   405                 410
<210> 7
   <211> 410
   <212> PRT
   <213> Artificial Sequence
<220>
   <223> D-3-phosphoglycerate dehydrogenase(G336V, R338G), SerA(G336V, R338G)
<400> 7
   Met Ala Lys Val Ser Leu Glu Lys Asp Lys Ile Lys Phe Leu Leu Val
    1               5                  10                  15
   Glu Gly Val His Gln Lys Ala Leu Glu Ser Leu Arg Ala Ala Gly Tyr
               20                  25                  30
   Thr Asn Ile Glu Phe His Lys Gly Ala Leu Asp Asp Glu Gln Leu Lys
           35                  40                  45
   Glu Ser Ile Arg Asp Ala His Phe Ile Gly Leu Arg Ser Arg Thr His
       50                  55                  60
   Leu Thr Glu Asp Val Ile Asn Ala Ala Glu Lys Leu Val Ala Ile Gly
   65                  70                  75                  80
   Cys Phe Cys Ile Gly Thr Asn Gln Val Asp Leu Asp Ala Ala Ala Lys
                   85                  90                  95
   Arg Gly Ile Pro Val Phe Asn Ala Pro Phe Ser Asn Thr Arg Ser Val
               100                 105                 110
   Ala Glu Leu Val Ile Gly Glu Leu Leu Leu Leu Leu Arg Gly Val Pro
           115                 120                 125
   Glu Ala Asn Ala Lys Ala His Arg Gly Val Trp Asn Lys Leu Ala Ala
       130                 135                 140
   Gly Ser Phe Glu Ala Arg Gly Lys Lys Leu Gly Ile Ile Gly Tyr Gly
   145                 150                 155                 160
   His Ile Gly Thr Gln Leu Gly Ile Leu Ala Glu Ser Leu Gly Met Tyr
                   165                 170                 175
   Val Tyr Phe Tyr Asp Ile Glu Asn Lys Leu Pro Leu Gly Asn Ala Thr
               180                 185                 190
   Gln Val Gln His Leu Ser Asp Leu Leu Asn Met Ser Asp Val Val Ser
           195                 200                 205
   Leu His Val Pro Glu Asn Pro Ser Thr Lys Asn Met Met Gly Ala Lys
       210                 215                 220
   Glu Ile Ser Leu Met Lys Pro Gly Ser Leu Leu Ile Asn Ala Ser Arg
   225                 230                 235                 240
   Gly Thr Val Val Asp Ile Pro Ala Leu Cys Asp Ala Leu Ala Ser Lys
                   245                 250                 255
   His Leu Ala Gly Ala Ala Ile Asp Val Phe Pro Thr Glu Pro Ala Thr
               260                 265                 270
   Asn Ser Asp Pro Phe Thr Ser Pro Leu Cys Glu Phe Asp Asn Val Leu
           275                 280                 285
   Leu Thr Pro His Ile Gly Gly Ser Thr Gln Glu Ala Gln Glu Asn Ile
       290                 295                 300
   Gly Leu Glu Val Ala Gly Lys Leu Ile Lys Tyr Ser Asp Asn Gly Ser
   305                 310                 315                 320
   Thr Leu Ser Ala Val Asn Phe Pro Glu Val Ser Leu Pro Leu His Val
                   325                 330                 335
   Gly Gly Arg Leu Met His Ile His Glu Asn Arg Pro Gly Val Leu Thr
               340                 345                 350
   Ala Leu Asn Lys Ile Phe Ala Glu Gln Gly Val Asn Ile Ala Ala Gln
           355                 360                 365
   Tyr Leu Gln Thr Ser Ala Gln Met Gly Tyr Val Val Ile Asp Ile Glu
       370                 375                 380
   Ala Asp Glu Asp Val Ala Glu Lys Ala Leu Gln Ala Met Lys Ala Ile
   385                 390                 395                 400
   Pro Gly Thr Ile Arg Ala Arg Leu Leu Tyr
                   405                 410
<210> 8
   <211> 362
   <212> PRT
   <213> Escherichia coli K12 W3110
<220>
   <221> PEPTIDE
   <222> (1)..(362)
   <223> 3-phosphoserine/phosphohydroxythreonine aminotransferase, SerC
<400> 8
   Met Ala Gln Ile Phe Asn Phe Ser Ser Gly Pro Ala Met Leu Pro Ala
   1               5                   10                  15
   Glu Val Leu Lys Gln Ala Gln Gln Glu Leu Arg Asp Trp Asn Gly Leu
               20                  25                  30
   Gly Thr Ser Val Met Glu Val Ser His Arg Gly Lys Glu Phe Ile Gln
           35                  40                  45
   Val Ala Glu Glu Ala Glu Lys Asp Phe Arg Asp Leu Leu Asn Val Pro
       50                  55                  60
   Ser Asn Tyr Lys Val Leu Phe Cys His Gly Gly Gly Arg Gly Gln Phe
   65                  70                  75                  80
   Ala Ala Val Pro Leu Asn Ile Leu Gly Asp Lys Thr Thr Ala Asp Tyr
                   85                  90                  95
   Val Asp Ala Gly Tyr Trp Ala Ala Ser Ala Ile Lys Glu Ala Lys Lys
               100                 105                 110
   Tyr Cys Thr Pro Asn Val Phe Asp Ala Lys Val Thr Val Asp Gly Leu
           115                 120                 125
   Arg Ala Val Lys Pro Met Arg Glu Trp Gln Leu Ser Asp Asn Ala Ala
       130                 135                 140
   Tyr Met His Tyr Cys Pro Asn Glu Thr Ile Asp Gly Ile Ala Ile Asp
   145                 150                 155                 160
   Glu Thr Pro Asp Phe Gly Ala Asp Val Val Val Ala Ala Asp Phe Ser
                   165                 170                 175
   Ser Thr Ile Leu Ser Arg Pro Ile Asp Val Ser Arg Tyr Gly Val Ile
               180                 185                 190
   Tyr Ala Gly Ala Gln Lys Asn Ile Gly Pro Ala Gly Leu Thr Ile Val
           195                 200                 205
   Ile Val Arg Glu Asp Leu Leu Gly Lys Ala Asn Ile Ala Cys Pro Ser
       210                 215                 220
   Ile Leu Asp Tyr Ser Ile Leu Asn Asp Asn Gly Ser Met Phe Asn Thr
   225                 230                 235                 240
   Pro Pro Thr Phe Ala Trp Tyr Leu Ser Gly Leu Val Phe Lys Trp Leu
                   245                 250                 255
   Lys Ala Asn Gly Gly Val Ala Glu Met Asp Lys Ile Asn Gln Gln Lys
               260                 265                 270
   Ala Glu Leu Leu Tyr Gly Val Ile Asp Asn Ser Asp Phe Tyr Arg Asn
           275                 280                 285
   Asp Val Ala Lys Ala Asn Arg Ser Arg Met Asn Val Pro Phe Gln Leu
       290                 295                 300
   Ala Asp Ser Ala Leu Asp Lys Leu Phe Leu Glu Glu Ser Phe Ala Ala
   305                 310                 315                 320
   Gly Leu His Ala Leu Lys Gly His Arg Val Val Gly Gly Met Arg Ala
   325 330 335
   Ser Ile Tyr Asn Ala Met Pro Leu Glu Gly Val Lys Ala Leu Thr Asp
   340 345 350
   Phe Met Val Glu Phe Glu Arg Arg His Gly
   355 360
<210> 9
   <211> 323
   <212> PRT
   <213> Mycobacterium smegmatics str. MC2 155
<220>
   <221> PEPTIDE
   <222> (1)..(323)
   <223> Msm-OPSS
<400> 9
   Met Thr Arg Tyr Asp Ser Leu Leu Gln Ala Leu Gly Asn Thr Pro Leu
   1               5                   10                  15
   Val Gly Leu Gln Asn Leu Ser Pro Arg Trp Asp Asp Glu Asp Gly Lys
               20                  25                  30
   Pro His Val Arg Leu Trp Ala Lys Leu Glu Asp Arg Asn Pro Thr Gly
           35                  40                  45
   Ser Ile Lys Asp Arg Pro Ala Leu Arg Met Ile Glu Gln Ala Glu Arg
       50                  55                  60
   Asp Gly Leu Leu Gln Pro Gly Ala Thr Ile Leu Glu Pro Thr Ser Gly
   65                  70                  75                  80
   Asn Thr Gly Ile Ser Leu Ala Met Ala Ala Leu Leu Lys Gly Tyr Asn
                   85                  90                  95
   Met Ile Cys Val Met Pro Glu Asn Thr Ser Ile Glu Arg Arg Gln Ile
               100                 105                 110
   Leu Glu Leu Tyr Gly Ala Arg Ile Ile Phe Ser Pro Ala Glu Gly Gly
           115                 120                 125
   Ser Asn Thr Ala Val Ala Thr Ala Lys Glu Leu Ala Ala Gln Asn Pro
       130                 135                 140
   Ser Trp Val Met Leu Tyr Gln Tyr Gly Asn Pro Ala Asn Ser Asp Ala
   145                 150                 155                 160
   His Tyr Phe Gly Thr Gly Pro Glu Leu Leu Ala Asp Leu Pro Glu Ile
                   165                 170                 175
   Thr His Phe Val Ala Gly Leu Gly Thr Thr Gly Thr Leu Met Gly Thr
               180                 185                 190
   Gly Arg Phe Leu Arg Glu His Val Pro Gly Val Gln Ile Val Ala Ala
   195 200 205
   Glu Pro Arg Tyr Gly Glu Gly Val Tyr Ala Leu Arg Asn Ile Asp Glu
   210 215 220
   Gly Phe Ile Pro Glu Leu Tyr Asp Ala Asp Val Leu Thr Thr Arg Phe
   225 230 235 240
   Ser Val Gly Ser Phe Asp Ala Val Arg Arg Thr Arg Glu Leu Val Thr
   245 250 255
   Arg Glu Gly Ile Phe Ala Gly Ile Ser Thr Gly Ala Val Leu His Ala
   260 265 270
   Ala Leu Gly Met Ala Ala Lys Ala Val Lys Ala Gly Glu Arg Ala Asp
   275 280 285
   Ile Ala Phe Val Val Ala Asp Ala Gly Trp Lys Tyr Leu Ser Thr Gly
   290 295 300
   Ala Tyr Ala Gly Ser Leu Asp Asp Ala Glu Asp Ala Leu Glu Gly Gln
   305 310 315 320
   Leu Trp Ala
<210> 10
   <211> 318
   <212> PRT
   <213> Mycobacterium smegmatics str. MC2 155
<220>
   <221> PEPTIDE
   <222> (1)..(318)
   <223> Msm-T
<400> 10
   Met Thr Arg Tyr Asp Ser Leu Leu Gln Ala Leu Gly Asn Thr Pro Leu
   1               5                   10                  15
   Val Gly Leu Gln Asn Leu Ser Pro Arg Trp Asp Asp Glu Asp Gly Lys
               20                  25                  30
   Pro His Val Arg Leu Trp Ala Lys Leu Glu Asp Arg Asn Pro Thr Gly
           35                  40                  45
   Ser Ile Lys Asp Arg Pro Ala Leu Arg Met Ile Glu Gln Ala Glu Arg
       50                  55                  60
   Asp Gly Leu Leu Gln Pro Gly Ala Thr Ile Leu Glu Pro Thr Ser Gly
   65                  70                  75                  80
   Asn Thr Gly Ile Ser Leu Ala Met Ala Ala Leu Leu Lys Gly Tyr Asn
                   85                  90                  95
   Met Ile Cys Val Met Pro Glu Asn Thr Ser Ile Glu Arg Arg Gln Ile
               100                 105                 110
   Leu Glu Leu Tyr Gly Ala Arg Ile Ile Phe Ser Pro Ala Glu Gly Gly
   115 120 125
   Ser Asn Thr Ala Val Ala Thr Ala Lys Glu Leu Ala Ala Gln Asn Pro
   130 135 140
   Ser Trp Val Met Leu Tyr Gln Tyr Gly Asn Pro Ala Asn Ser Asp Ala
   145 150 155 160
   His Tyr Phe Gly Thr Gly Pro Glu Leu Leu Ala Asp Leu Pro Glu Ile
   165 170 175
   Thr His Phe Val Ala Gly Leu Gly Thr Thr Gly Thr Leu Met Gly Thr
   180 185 190
   Gly Arg Phe Leu Arg Glu His Val Pro Gly Val Gln Ile Val Ala Ala
   195 200 205
   Glu Pro Arg Tyr Gly Glu Gly Val Tyr Ala Leu Arg Asn Ile Asp Glu
   210 215 220
   Gly Phe Ile Pro Glu Leu Tyr Asp Ala Asp Val Leu Thr Thr Arg Phe
   225 230 235 240
   Ser Val Gly Ser Phe Asp Ala Val Arg Arg Thr Arg Glu Leu Val Thr
   245 250 255
   Arg Glu Gly Ile Phe Ala Gly Ile Ser Thr Gly Ala Val Leu His Ala
   260 265 270
   Ala Leu Gly Met Ala Ala Lys Ala Val Lys Ala Gly Glu Arg Ala Asp
   275 280 285
   Ile Ala Phe Val Val Ala Asp Ala Gly Trp Lys Tyr Leu Ser Thr Gly
   290 295 300
   Ala Tyr Ala Gly Ser Leu Asp Asp Ala Glu Asp Ala Leu Glu
   305 310 315
<210> 11
   <211> 318
   <212> PRT
   <213> Mycobacterium tuberculosis H37Rv
<220>
   <221> PEPTIDE
   <222> (1)..(318)
   <223> Mtb-T
<400> 11
   Met Thr Arg Tyr Asp Ser Leu Leu Gln Ala Leu Gly Asn Thr Pro Leu
    1               5                  10                  15
   Val Gly Leu Gln Arg Leu Ser Pro Arg Trp Asp Asp Gly Arg Asp Gly
               20                  25                  30
   Pro His Val Arg Leu Trp Ala Lys Leu Glu Asp Arg Asn Pro Thr Gly
           35                  40                  45
   Ser Ile Lys Asp Arg Pro Ala Val Arg Met Ile Glu Gln Ala Glu Ala
       50                  55                  60
   Asp Gly Leu Leu Arg Pro Gly Ala Thr Ile Leu Glu Pro Thr Ser Gly
   65                  70                  75                  80
   Asn Thr Gly Ile Ser Leu Ala Met Ala Ala Arg Leu Lys Gly Tyr Arg
                   85                  90                  95
   Leu Ile Cys Val Met Pro Glu Asn Thr Ser Val Glu Arg Arg Gln Leu
               100                 105                 110
   Leu Glu Leu Tyr Gly Ala Gln Ile Ile Phe Ser Ala Ala Glu Gly Gly
           115                 120                 125
   Ser Asn Thr Ala Val Ala Thr Ala Lys Glu Leu Ala Ala Thr Asn Pro
       130                 135                 140
   Ser Trp Val Met Leu Tyr Gln Tyr Gly Asn Pro Ala Asn Thr Asp Ser
   145                 150                 155                 160
   His Tyr Cys Gly Thr Gly Pro Glu Leu Leu Ala Asp Leu Pro Glu Ile
                   165                 170                 175
   Thr His Phe Val Ala Gly Leu Gly Thr Thr Gly Thr Leu Met Gly Thr
               180                 185                 190
   Gly Arg Phe Leu Arg Glu His Val Ala Asn Val Lys Ile Val Ala Ala
           195                 200                 205
   Glu Pro Arg Tyr Gly Glu Gly Val Tyr Ala Leu Arg Asn Met Asp Glu
       210                 215                 220
   Gly Phe Val Pro Glu Leu Tyr Asp Pro Glu Ile Leu Thr Ala Arg Tyr
   225                 230                 235                 240
   Ser Val Gly Ala Val Asp Ala Val Arg Arg Thr Arg Glu Leu Val His
                   245                 250                 255
   Thr Glu Gly Ile Phe Ala Gly Ile Ser Thr Gly Ala Val Leu His Ala
               260                 265                 270
   Ala Leu Gly Val Gly Ala Gly Ala Leu Ala Ala Gly Glu Arg Ala Asp
           275                 280                 285
   Ile Ala Leu Val Val Ala Asp Ala Gly Trp Lys Tyr Leu Ser Thr Gly
       290                 295                 300
   Ala Tyr Ala Gly Ser Leu Asp Asp Ala Glu Thr Ala Leu Glu
   305                 310                 315
<210> 12
   <211> 389
   <212> PRT
   <213> Artificial Sequence
<220>
   <223> Ape-OPSS
<400> 12
   Met Ala Leu Ala Asp Ile Ser Gly Tyr Leu Asp Val Leu Asp Ser Val
   1               5                   10                  15
   Arg Gly Phe Ser Tyr Leu Glu Asn Ala Arg Glu Val Leu Arg Ser Gly
               20                  25                  30
   Glu Ala Arg Cys Leu Gly Asn Pro Arg Ser Glu Pro Glu Tyr Val Lys
           35                  40                  45
   Ala Leu Tyr Val Ile Gly Ala Ser Arg Ile Pro Val Gly Asp Gly Cys
       50                  55                  60
   Ser His Thr Leu Glu Glu Leu Gly Val Phe Asp Ile Ser Val Pro Gly
   65                  70                  75                  80
   Glu Met Val Phe Pro Ser Pro Leu Asp Phe Phe Glu Arg Gly Lys Pro
                   85                  90                  95
   Thr Pro Leu Val Arg Ser Arg Leu Gln Leu Pro Asn Gly Val Arg Val
               100                 105                 110
   Trp Leu Lys Leu Glu Trp Tyr Asn Pro Phe Ser Leu Ser Val Lys Asp
           115                 120                 125
   Arg Pro Ala Val Glu Ile Ile Ser Arg Leu Ser Arg Arg Val Glu Lys
       130                 135                 140
   Gly Ser Leu Val Ala Asp Ala Thr Ser Ser Asn Phe Gly Val Ala Leu
   145                 150                 155                 160
   Ser Ala Val Ala Arg Leu Tyr Gly Tyr Arg Ala Arg Val Tyr Leu Pro
                   165                 170                 175
   Gly Ala Ala Glu Glu Phe Gly Lys Leu Leu Pro Arg Leu Leu Gly Ala
               180                 185                 190
   Gln Val Ile Val Asp Pro Glu Ala Pro Ser Thr Val His Leu Leu Pro
           195                 200                 205
   Arg Val Met Lys Asp Ser Lys Asn Glu Gly Phe Val His Val Asn Gln
       210                 215                 220
   Phe Tyr Asn Asp Ala Asn Phe Glu Ala His Met Arg Gly Thr Ala Arg
   225                 230                 235                 240
   Glu Ile Phe Val Gln Ser Arg Arg Gly Gly Leu Ala Leu Arg Gly Val
                   245                 250                 255
   Ala Gly Ser Leu Gly Thr Ser Gly His Met Ser Ala Ala Ala Phe Tyr
               260                 265                 270
   Leu Gln Ser Val Asp Pro Ser Ile Arg Ala Val Leu Val Gln Pro Ala
           275                 280                 285
   Gln Gly Asp Ser Ile Pro Gly Ile Arg Arg Val Glu Thr Gly Met Leu
       290                 295                 300
   Trp Ile Asn Met Leu Asp Ile Ser Tyr Thr Leu Ala Glu Val Thr Leu
   305 310 315 320
   Glu Glu Ala Met Glu Ala Val Val Glu Val Ala Arg Ser Asp Gly Leu
   325 330 335
   Val Ile Gly Pro Ser Gly Gly Ala Ala Val Lys Ala Leu Ala Lys Lys
   340 345 350
   Ala Ala Glu Gly Asp Leu Glu Pro Gly Asp Tyr Val Val Val Val Pro
   355 360 365
   Asp Thr Gly Phe Lys Tyr Leu Ser Leu Val Gln Asn Ala Leu Glu Gly
   370 375 380
   Ala Gly Asp Ser Val
   385
<210> 13
   <211> 1341
   <212> DNA
   <213> Corynebacterium glutamicum 13032
<220>
   <221> gene
   <222> (1)..(1341)
   <223> serB
<400> 13
   atgtcgtgtt ccgcgctcag acatgagaca attgttgccg tgactgaact catccagaat
   60
   gaatcccaag aaatcgctga gctggaagcc ggccagcagg ttgcattgcg tgaaggttat
   120
   cttcctgcgg tgatcacagt gagcggtaaa gaccgcccag gtgtgactgc cgcgttcttt
   180
   agggtcttgt ccgctaatca ggttcaggtc ttggacgttg agcagtcaat gttccgtggc
   240
   tttttgaact tggcggcgtt tgtgggtatc gcacctgagc gtgtcgagac cgtcaccaca
   300
   ggcctgactg acaccctcaa ggtgcatgga cagtccgtgg tggtggagct gcaggaaact
   360
   gtgcagtcgt cccgtcctcg ttcttcccat gttgttgtgg tgttgggtga tccggttgat
   420
   gcgttggata tttcccgcat tggtcagacc ctggcggatt acgatgccaa cattgacacc
   480
   attcgtggta tttcggatta ccctgtgacc ggcctggagc tgaaggtgac tgtgccggat
   540
   gtcagccctg gtggtggtga agcgatgcgt aaggcgcttg ctgctcttac ctctgagctg
   600
   aatgtggata ttgcgattga gcgttctggt ttgctgcgtc gttctaagcg tctggtgtgc 660
   ttcgattgtg attccacgtt gatcactggt gaggtcattg agatgctggc ggctcacgcg 720
   ggcaaggaag ctgaagttgc ggcagttact gagcgtgcga tgcgcggtga gctcgatttc 780
   gaggagtctc tgcgtgagcg tgtgaaggcg ttggctggtt tggatgcgtc ggtgatcgat 840
   gaggtcgctg ccgctattga gctgacccct ggtgcgcgca ccacgatccg tacgctgaac 900
   cgcatgggtt accagaccgc tgttgtttcc ggtggtttca tccaggtgtt ggaaggtttg 960
   gctgaggagt tggagttgga ttatgtccgc gccaacactt tggaaatcgt tgatggcaag 1020
   ctgaccggca acgtcaccgg aaagatcgtt gaccgcgctg cgaaggctga gttcctccgt 1080
   gagttcgctg cggattctgg cctgaagatg taccagactg tcgctgtcgg tgatggcgct 1140
   aatgacatcg atatgctctc cgctgcgggt ctgggtgttg ctttcaacgc gaagcctgcg 1200
   ctgaaggaga ttgcggatac ttccgtgaac cacccattcc tcgacgaggt tttgcacatc 1260
   atgggcattt cccgcgacga gatcgatctg gcggatcagg aagacggcac tttccaccgc 1320
   gttccattga ccaatgccta a 1341
<210> 14
   <211> 1593
   <212> DNA
   <213> Artificial Sequence
<220>
   <223> serA*(E235K)
<400> 14
   atgagccaga atggccgtcc ggtagtcctc atcgccgata agcttgcgca gtccactgtt 60
   gacgcgcttg gagatgcagt agaagtccgt tgggttgacg gacctaaccg cccagaactg 120
   cttgatgcag ttaaggaagc ggacgcactg ctcgtgcgtt ctgctaccac tgtcgatgct 180
   gaagtcatcg ccgctgcccc taacttgaag atcgtcggtc gtgccggcgt gggcttggac 240
   aacgttgaca tccctgctgc cactgaagct ggcgtcatgg ttgctaacgc accgacctct 300
   aatattcact ccgcttgtga gcacgcaatt tctttgctgc tgtctactgc tcgccagatc 360
   cctgctgctg atgcgacgct gcgtgagggc gagtggaagc ggtcttcttt caacggtgtg 420
   gaaattttcg gaaaaactgt cggtatcgtc ggttttggcc acattggtca gttgtttgct 480
   cagcgtcttg ctgcgtttga gaccaccatt gttgcttacg atccttacgc taaccctgct 540
   cgtgcggctc agctgaacgt tgagttggtt gagttggatg agctgatgag ccgttctgac 600
   tttgtcacca ttcaccttcc taagaccaag gaaactgctg gcatgtttga tgcgcagctc 660
   cttgctaagt ccaagaaggg ccagatcatc atcaacgctg ctcgtggtgg ccttgttgat 720
   gagcaggctt tggctgatgc gattgagtcc ggtcacattc gtggcgctgg tttcgatgtg 780
   tactccaccg agccttgcac tgattctcct ttgttcaagt tgcctcaggt tgttgtgact 840
   cctcacttgg gtgcttctac tgaagaggct caggatcgtg cgggtactga cgttgctgat 900
   tctgtgctca aggcgctggc tggcgagttc gtggcggatg ctgtgaacgt ttccggtggt 960
   cgcgtgggcg aaaaggttgc tgtgtggatg gatctggctc gcaagcttgg tcttcttgct 1020
   ggcaagcttg tcgacgccgc cccagtctcc attgaggttg aggctcgagg cgagctttct 1080
   tccgagcagg tcgatgcact tggtttgtcc gctgttcgtg gtttgttctc cggaattatc 1140
   gaagagtccg ttactttcgt caacgctcct cgcattgctg aagagcgtgg cctggacatc 1200
   tccgtgaaga ccaactctga gtctgttact caccgttccg tcctgcaggt caaggtcatt 1260
   actggcagcg gcgcgagcgc aactgttgtt ggtgccctga ctggtcttga gcgcgttgag 1320
   aagatcaccc gcatcaatgg ccgtggcctg gatctgcgcg cagagggtct gaacctcttc 1380
   ctgcagtaca ctgacgctcc tggtgcactg ggtaccgttg gtaccaagct gggtgctgct
   1440
   ggcatcaaca tcgaggctgc tgcgttgact caggctgaga agggtgacgg cgctgtcctg
   1500
   atcctgcgtg ttgagtccgc tgtctctgaa gagctggaag ctgaaatcaa cgctgagttg
   1560
   ggtgctactt ccttccaggt tgatcttgac taa
   1593
<210> 15
   <211> 1002
   <212> DNA
   <213> Artificial Sequence
<220>
   <223> serA*(197 delta)
<400> 15
   atgagccaga atggccgtcc ggtagtcctc atcgccgata agcttgcgca gtccactgtt 60
   gacgcgcttg gagatgcagt agaagtccgt tgggttgacg gacctaaccg cccagaactg 120
   cttgatgcag ttaaggaagc ggacgcactg ctcgtgcgtt ctgctaccac tgtcgatgct 180
   gaagtcatcg ccgctgcccc taacttgaag atcgtcggtc gtgccggcgt gggcttggac 240
   aacgttgaca tccctgctgc cactgaagct ggcgtcatgg ttgctaacgc accgacctct 300
   aatattcact ccgcttgtga gcacgcaatt tctttgctgc tgtctactgc tcgccagatc 360
   cctgctgctg atgcgacgct gcgtgagggc gagtggaagc ggtcttcttt caacggtgtg 420
   gaaattttcg gaaaaactgt cggtatcgtc ggttttggcc acattggtca gttgtttgct 480
   cagcgtcttg ctgcgtttga gaccaccatt gttgcttacg atccttacgc taaccctgct 540
   cgtgcggctc agctgaacgt tgagttggtt gagttggatg agctgatgag ccgttctgac 600
   tttgtcacca ttcaccttcc taagaccaag gaaactgctg gcatgtttga tgcgcagctc 660
   cttgctaagt ccaagaaggg ccagatcatc atcaacgctg ctcgtggtgg ccttgttgat 720
   gagcaggctt tggctgatgc gattgagtcc ggtcacattc gtggcgctgg tttcgatgtg 780
   tactccaccg agccttgcac tgattctcct ttgttcaagt tgcctcaggt tgttgtgact 840
   cctcacttgg gtgcttctac tgaagaggct caggatcgtg cgggtactga cgttgctgat 900
   tctgtgctca aggcgctggc tggcgagttc gtggcggatg ctgtgaacgt ttccggtggt 960
   cgcgtgggcg aagaggttgc tgtgtggatg gatctggctt aa 1002
<210> 16
   <211> 969
   <212> DNA
   <213> Escherichia coli K12 W3110
<220>
   <221> gene
   <222> (1)..(969)
   <223> serB
<400> 16
   atgcctaaca ttacctggtg cgacctgcct gaagatgtct ctttatggcc gggtctgcct 60
   ctttcattaa gtggtgatga agtgatgcca ctggattacc acgcaggtcg tagcggctgg 120
   ctgctgtatg gtcgtgggct ggataaacaa cgtctgaccc aataccagag caaactgggt 180
   gcggcgatgg tgattgttgc cgcctggtgc gtggaagatt atcaggtgat tcgtctggca 240
   ggttcactca ccgcacgggc tacacgcctg gcccacgaag cgcagctgga tgtcgccccg 300
   ctggggaaaa tcccgcacct gcgcacgccg ggtttgctgg tgatggatat ggactccacc 360
   gccatccaga ttgaatgtat tgatgaaatt gccaaactgg ccggaacggg cgagatggtg 420
   gcggaagtaa ccgaacgggc gatgcgcggc gaactcgatt ttaccgccag cctgcgcagc 480
   cgtgtggcga cgctgaaagg cgctgacgcc aatattctgc aacaggtgcg tgaaaatctg 540
   ccgctgatgc caggcttaac gcaactggtg ctcaagctgg aaacgctggg ctggaaagtg 600
   gcgattgcct ccggcggctt tactttcttt gctgaatacc tgcgcgacaa gctgcgcctg 660
   accgccgtgg tagccaatga actggagatc atggacggta aatttaccgg caatgtgatc 720
   ggcgacatcg tagacgcgca gtacaaagcg aaaactctga ctcgcctcgc gcaggagtat 780
   gaaatcccgc tggcgcagac cgtggcgatt ggcgatggag ccaatgacct gccgatgatc 840
   aaagcggcag ggctggggat tgcctaccat gccaagccaa aagtgaatga aaaggcggaa 900
   gtcaccatcc gtcacgctga cctgatgggg gtattctgca tcctctcagg cagcctgaat 960
   cagaagtaa 969
<210> 17
   <211> 1233
   <212> DNA
   <213> Escherichia coli K12 W3110
<220>
   <221> gene
   <222> (1)..(1233)
   <223> serA
<400> 17
   atggcaaagg tatcgctgga gaaagacaag attaagtttc tgctggtaga aggcgtgcac 60
   caaaaggcgc tggaaagcct tcgtgcagct ggttacacca acatcgaatt tcacaaaggc 120
   gcgctggatg atgaacaatt aaaagaatcc atccgcgatg cccacttcat cggcctgcga 180
   tcccgtaccc atctgactga agacgtgatc aacgccgcag aaaaactggt cgctattggc 240
   tgtttctgta tcggaacaaa ccaggttgat ctggatgcgg cggcaaagcg cgggatcccg 300
   gtatttaacg caccgttctc aaatacgcgc tctgttgcgg agctggtgat tggcgaactg 360
   ctgctgctat tgcgcggcgt gccggaagcc aatgctaaag cgcaccgtgg cgtgtggaac 420
   aaactggcgg cgggttcttt tgaagcgcgc ggcaaaaagc tgggtatcat cggctacggt 480
   catattggta cgcaattggg cattctggct gaatcgctgg gaatgtatgt ttacttttat 540
   gatattgaaa ataaactgcc gctgggcaac gccactcagg tacagcatct ttctgacctg 600
   ctgaatatga gcgatgtggt gagtctgcat gtaccagaga atccgtccac caaaaatatg 660
   atgggcgcga aagaaatttc actaatgaag cccggctcgc tgctgattaa tgcttcgcgc 720
   ggtactgtgg tggatattcc ggcgctgtgt gatgcgctgg cgagcaaaca tctggcgggg 780
   gcggcaatcg acgtattccc gacggaaccg gcgaccaata gcgatccatt tacctctccg 840
   ctgtgtgaat tcgacaacgt ccttctgacg ccacacattg gcggttcgac tcaggaagcg 900
   caggagaata tcggcctgga agttgcgggt aaattgatca agtattctga caatggctca 960
   acgctctctg cggtgaactt cccggaagtc tcgctgccac tgcacggtgg gcgtcgtctg 1020
   atgcacatcc acgaaaaccg tccgggcgtg ctaactgcgc tgaacaaaat cttcgccgag 1080
   caggcgtcaa acatcgccgc gcaatatctg caaacttccg cccagatggg ttatgtggtt 1140
   attgatattg aagccgacga agacgttgcc gaaaaagcgc tgcaggcaat gaaagctatt 1200
   ccgggtacca ttcgcgcccg tctgctgtac taa 1233
<210> 18
   <211> 1233
   <212> DNA
   <213> Artificial Sequence
<220>
   <223> serA*(G336V)
<400> 18
   atggcaaagg tatcgctgga gaaagacaag attaagtttc tgctggtaga aggcgtgcac 60
   caaaaggcgc tggaaagcct tcgtgcagct ggttacacca acatcgaatt tcacaaaggc 120
   gcgctggatg atgaacaatt aaaagaatcc atccgcgatg cccacttcat cggcctgcga 180
   tcccgtaccc atctgactga agacgtgatc aacgccgcag aaaaactggt cgctattggc 240
   tgtttctgta tcggaacaaa ccaggttgat ctggatgcgg cggcaaagcg cgggatcccg 300
   gtatttaacg caccgttctc aaatacgcgc tctgttgcgg agctggtgat tggcgaactg 360
   ctgctgctat tgcgcggcgt gccggaagcc aatgctaaag cgcaccgtgg cgtgtggaac 420
   aaactggcgg cgggttcttt tgaagcgcgc ggcaaaaagc tgggtatcat cggctacggt 480
   catattggta cgcaattggg cattctggct gaatcgctgg gaatgtatgt ttacttttat 540
   gatattgaaa ataaactgcc gctgggcaac gccactcagg tacagcatct ttctgacctg 600
   ctgaatatga gcgatgtggt gagtctgcat gtaccagaga atccgtccac caaaaatatg 660
   atgggcgcga aagaaatttc actaatgaag cccggctcgc tgctgattaa tgcttcgcgc 720
   ggtactgtgg tggatattcc ggcgctgtgt gatgcgctgg cgagcaaaca tctggcgggg 780
   gcggcaatcg acgtattccc gacggaaccg gcgaccaata gcgatccatt tacctctccg 840
   ctgtgtgaat tcgacaacgt ccttctgacg ccacacattg gcggttcgac tcaggaagcg 900
   caggagaata tcggcctgga agttgcgggt aaattgatca agtattctga caatggctca 960
   acgctctctg cggtgaactt cccggaagtc tcgctgccac tgcacgttgg gcgtcgtctg 1020
   atgcacatcc acgaaaaccg tccgggcgtg ctaactgcgc tgaacaaaat cttcgccgag 1080
   cagggcgtca acatcgccgc gcaatatctg caaacttccg cccagatggg ttatgtggtt 1140
   attgatattg aagccgacga agacgttgcc gaaaaagcgc tgcaggcaat gaaagctatt 1200
   ccgggtacca ttcgcgcccg tctgctgtac taa 1233
<210> 19
   <211> 1233
   <212> DNA
   <213> Artificial Sequence
<220>
   <223> serA*(G336V,G337V)
<400> 19
   atggcaaagg tatcgctgga gaaagacaag attaagtttc tgctggtaga aggcgtgcac
   60
   caaaaggcgc tggaaagcct tcgtgcagct ggttacacca acatcgaatt tcacaaaggc
   120
   gcgctggatg atgaacaatt aaaagaatcc atccgcgatg cccacttcat cggcctgcga
   180
   tcccgtaccc atctgactga agacgtgatc aacgccgcag aaaaactggt cgctattggc
   240
   tgtttctgta tcggaacaaa ccaggttgat ctggatgcgg cggcaaagcg cgggatcccg
   300
   gtatttaacg caccgttctc aaatacgcgc tctgttgcgg agctggtgat tggcgaactg
   360
   ctgctgctat tgcgcggcgt gccggaagcc aatgctaaag cgcaccgtgg cgtgtggaac
   420
   aaactggcgg cgggttcttt tgaagcgcgc ggcaaaaagc tgggtatcat cggctacggt
   480
   catattggta cgcaattggg cattctggct gaatcgctgg gaatgtatgt ttacttttat
   540
   gatattgaaa ataaactgcc gctgggcaac gccactcagg tacagcatct ttctgacctg
   600
   ctgaatatga gcgatgtggt gagtctgcat gtaccagaga atccgtccac caaaaatatg
   660
   atgggcgcga aagaaatttc actaatgaag cccggctcgc tgctgattaa tgcttcgcgc
   720
   ggtactgtgg tggatattcc ggcgctgtgt gatgcgctgg cgagcaaaca tctggcgggg
   780
   gcggcaatcg acgtattccc gacggaaccg gcgaccaata gcgatccatt tacctctccg
   840
   ctgtgtgaat tcgacaacgt ccttctgacg ccacacattg gcggttcgac tcaggaagcg
   900
   caggagaata tcggcctgga agttgcgggt aaattgatca agtattctga caatggctca
   960
   acgctctctg cggtgaactt cccggaagtc tcgctgccac tgcacgttgt gcgtcgtctg
   1020
   atgcacatcc acgaaaaccg tccgggcgtg ctaactgcgc tgaacaaaat cttcgccgag
   1080
   cagggcgtca acatcgccgc gcaatatctg caaacttccg cccagatggg ttatgtggtt
   1140
   attgatattg aagccgacga agacgttgcc gaaaaagcgc tgcaggcaat gaaagctatt
   1200
   ccgggtacca ttcgcgcccg tctgctgtac taa
   1233
<210> 20
   <211> 1233
   <212> DNA
   <213> Artificial Sequence
<220>
   <223> serA*(G336V,R338G)
<400> 20
   atggcaaagg tatcgctgga gaaagacaag attaagtttc tgctggtaga aggcgtgcac 60
   caaaaggcgc tggaaagcct tcgtgcagct ggttacacca acatcgaatt tcacaaaggc 120
   gcgctggatg atgaacaatt aaaagaatcc atccgcgatg cccacttcat cggcctgcga 180
   tcccgtaccc atctgactga agacgtgatc aacgccgcag aaaaactggt cgctattggc 240
   tgtttctgta tcggaacaaa ccaggttgat ctggatgcgg cggcaaagcg cgggatcccg 300
   gtatttaacg caccgttctc aaatacgcgc tctgttgcgg agctggtgat tggcgaactg 360
   ctgctgctat tgcgcggcgt gccggaagcc aatgctaaag cgcaccgtgg cgtgtggaac 420
   aaactggcgg cgggttcttt tgaagcgcgc ggcaaaaagc tgggtatcat cggctacggt 480
   catattggta cgcaattggg cattctggct gaatcgctgg gaatgtatgt ttacttttat 540
   gatattgaaa ataaactgcc gctgggcaac gccactcagg tacagcatct ttctgacctg 600
   ctgaatatga gcgatgtggt gagtctgcat gtaccagaga atccgtccac caaaaatatg 660
   atgggcgcga aagaaatttc actaatgaag cccggctcgc tgctgattaa tgcttcgcgc 720
   ggtactgtgg tggatattcc ggcgctgtgt gatgcgctgg cgagcaaaca tctggcgggg 780
   gcggcaatcg acgtattccc gacggaaccg gcgaccaata gcgatccatt tacctctccg 840
   ctgtgtgaat tcgacaacgt ccttctgacg ccacacattg gcggttcgac tcaggaagcg 900
   caggagaata tcggcctgga agttgcgggt aaattgatca agtattctga caatggctca 960
   acgctctctg cggtgaactt cccggaagtc tcgctgccac tgcacgttgg gggtcgtctg 1020
   atgcacatcc acgaaaaccg tccgggcgtg ctaactgcgc tgaacaaaat cttcgccgag 1080
   cagggcgtca acatcgccgc gcaatatctg caaacttccg cccagatggg ttatgtggtt 1140
   attgatattg aagccgacga agacgttgcc gaaaaagcgc tgcaggcaat gaaagctatt 1200
   ccgggtacca ttcgcgcccg tctgctgtac taa 1233
<210> 21
   <211> 1089
   <212> DNA
   <213> Escherichia coli K12 W3110
<220>
   <221> gene
   <222> (1)..(1089)
   <223> serC
<400> 21
   atggctcaaa tcttcaattt tagttctggt ccggcaatgc taccggcaga ggtgcttaaa 60
   caggctcaac aggaactgcg cgactggaac ggtcttggta cgtcggtgat ggaagtgagt 120
   caccgtggca aagagttcat tcaggttgca gaggaagccg agaaggattt tcgcgatctt 180
   cttaatgtcc cctccaacta caaggtatta ttctgccatg gcggtggtcg cggtcagttt 240
   gctgcggtac cgctgaatat tctcggtgat aaaaccaccg cagattatgt tgatgccggt 300
   tactgggcgg caagtgccat taaagaagcg aaaaaatact gcacgcctaa tgtctttgac 360
   gccaaagtga ctgttgatgg tctgcgcgcg gttaagccaa tgcgtgaatg gcaactctct 420
   gataatgctg cttatatgca ttattgcccg aatgaaacca tcgatggtat cgccatcgac 480
   gaaacgccag acttcggcgc agatgtggtg gtcgccgctg acttctcttc aaccattctt 540
   tcccgtccga ttgacgtcag ccgttatggt gtaatttacg ctggcgcgca gaaaaatatc 600
   ggcccggctg gcctgacaat cgtcatcgtt cgtgaagatt tgctgggcaa agcgaatatc
   660
   gcgtgtccgt cgattctgga ttattccatc ctcaacgata acggctccat gtttaacacg
   720
   ccgccgacat ttgcctggta tctatctggt ctggtcttta aatggctgaa agcgaacggc
   780
   ggtgtagctg aaatggataa aatcaatcag caaaaagcag aactgctata tggggtgatt
   840
   gataacagcg atttctaccg caatgacgtg gcgaaagcta accgttcgcg gatgaacgtg
   900
   ccgttccagt tggcggacag tgcgcttgac aaattgttcc ttgaagagtc ttttgctgct
   960
   ggccttcatg cactgaaagg tcaccgtgtg gtcggcggaa tgcgcgcttc tatttataac
   1020
   gccatgccgc tggaaggcgt taaagcgctg acagacttca tggttgagtt cgaacgccgt
   1080
   cacggttaa
   1089
<210> 22
   <211> 26
   <212> DNA
   <213> Artificial Sequence
<220>
   <223> primer for deletion cassette of serB
<400> 22
   gcgatatcat gaccttagaa tggtgg
   26
<210> 23
   <211> 23
   <212> DNA
   <213> Artificial Sequence
<220>
   <223> primer for deletion cassette of serB
<400> 23
   gctctagatc acgcatgcct cgc
   23
<210> 24
   <211> 30
   <212> DNA
   <213> Artificial Sequence
<220>
   <223> primer for deletion cassette of serB
<400> 24
   gcgatatcat gtcacccctg tgaaaatgac
   30
<210> 25
   <211> 29
   <212> DNA
   <213> Artificial Sequence
<220>
   <223> primer for deletion cassette of serB
<400> 25
   gctctagatc agttcgatac ctggggtat
   29
<210> 26
   <211> 24
   <212> DNA
   <213> Artificial Sequence
<220>
   <223> primer for deletion cassette of serB
<400> 26
   atcatgttac tggcaggcgc tatc
   24
<210> 27
   <211> 29
   <212> DNA
   <213> Artificial Sequence
<220>
   <223> primer for deletion cassette of serB
<400> 27
   gctctagatt acaaagtgaa agagagacg
   29
<210> 28
   <211> 26
   <212> DNA
   <213> Artificial Sequence
<220>
   <223> primer for construction of serA*
<400> 28
   acgatatcat gagccagaat ggccgt
   26
<210> 29
   <211> 28
   <212> DNA
   <213> Artificial Sequence
<220>
   <223> primer for construction of serA*
<400> 29
   cgtctagatt agtcaagatc aacctgga
   28
<210> 30
   <211> 41
   <212> DNA
   <213> Artificial Sequence
<220>
   <223> primer for construction of serA*(E325K)
<400> 30
   atccatccac acagcaacct tttcgcccac gcgaccaccg g
   41
<210> 31
   <211> 41
   <212> DNA
   <213> Artificial Sequence
<220>
   <223> primer for construction of serA*(E325K)
<400> 31
   ccggtggtcg cgtgggcgaa aaggttgctg tgtggatgga t
   41
<210> 32
   <211> 31
   <212> DNA
   <213> Artificial Sequence
<220>
   <223> primer for construction of serA* (197 delta)
<400> 32
   cgtctagatt aagccagatc catccacaca g
   31
<210> 33
   <211> 70
   <212> DNA
   <213> Artificial Sequence
<220>
   <223> primer for deletion cassette of serB
<400> 33
   atgcctaaca ttacctggtg cgacctgcct gaagatgtct ctttatggcc gtgtaggctg
   60
   gagctgcttc
   70
<210> 34
   <211> 70
   <212> DNA
   <213> Artificial Sequence
<220>
   <223> primer for deletion cassette of serB
<400> 34
   ggatggcggg ccaccaatta cttctgattc aggctgcctg agaggatgca catatgaata
   60
   tcctccttag
   70
<210> 35
   <211> 27
   <212> DNA
   <213> Artificial Sequence
<220>
   <223> primer for amplification of pself-serB to construct pBAC-pself-serB
<400> 35
   cccaagcttc ttccaccctt tgaaaat
   27
<210> 36
   <211> 27
   <212> DNA
   <213> Artificial Sequence
<220>
   <223> primer for amplification of pself-serB to construct pBAC-pself-serB
<400> 36
   cccaagcttt tacttctgat tcaggct
   27
<210> 37
   <211> 19
   <212> DNA
   <213> Artificial Sequence
<220>
   <223> primer for amplification of pself-CTG-serB to construct pBAC-pself-CTG-serB
<400> 37
   ggagccttac tgcctaaca
   19
<210> 38
   <211> 19
   <212> DNA
   <213> Artificial Sequence
<220>
   <223> primer for amplification of pself-CTG-serB to construct pBAC-pself-CTG-serB
<400> 38
   tgttaggcag taaggctcc
   19
<210> 39
   <211> 20
   <212> DNA
   <213> Artificial Sequence
<220>
   <223> primer for construction of serA*
<400> 39
   agggcgtggt gaccgataat
   20
<210> 40
   <211> 28
   <212> DNA
   <213> Artificial Sequence
<220>
   <223> primer for construction of serA*
<400> 40
   cctagagctc cattctggct gaatcgct
<210> 41
   <211> 26
   <212> DNA
   <213> Artificial Sequence
<220>
   <223> primer for construction of serA*
<400> 41
   acggatcccc cctgagactg actgtt
   26
<210> 42
   <211> 37
   <212> DNA
   <213> Artificial Sequence
<220>
   <223> primer for construction of serA*(G336V)
<400> 42
   tctcgctgcc actgcacgtt gggcgtcgtc tgatgca
   37
<210> 43
   <211> 37
   <212> DNA
   <213> Artificial Sequence
<220>
   <223> primer for construction of serA*(G336V)
<400> 43
   tgcatcagac gacgcccaac gtgcagtggc agcgaga
   37
<210> 44
   <211> 37
   <212> DNA
   <213> Artificial Sequence
<220>
   <223> primer for construction of serA*(G336V, G337V)
<400> 44
   cgctgccact gcacgttgtg cgtcgtctga tgcacat
   37
<210> 45
   <211> 37
   <212> DNA
   <213> Artificial Sequence
<220>
   <223> primer for construction of serA*(G336V, G337V)
<400> 45
   atgtgcatca gacgacgcac aacgtgcagt ggcagcg
   37
<210> 46
   <211> 37
   <212> DNA
   <213> Artificial Sequence
<220>
   <223> primer for construction of serA*(G336V, R338G)
<400> 46
   ctgccactgc acgttgtggg tcgtctgatg cacatcc
   37
<210> 47
   <211> 37
   <212> DNA
   <213> Artificial Sequence
<220>
   <223> primer for construction of serA*(G336V, R338G)
<400> 47
   ggatgtgcat cagacgaccc acaacgtgca gtggcag
   37
<210> 48
   <211> 28
   <212> DNA
   <213> Artificial Sequence
<220>
   <223> primer for amplification of serA to construct pCL-Prmf-serA
<400> 48
   gatatcatgg caaaggtatc gctggaga
   28
<210> 49
   <211> 26
   <212> DNA
   <213> Artificial Sequence
<220>
   <223> primer for amplification of serA to construct pCL-Prmf-serA
<400> 49
   aagcttttag tacagcagac gggcgc
   26
<210> 50
   <211> 24
   <212> DNA
   <213> Artificial Sequence
<220>
   <223> primer for amplification of serA to construct pCL-Prmf-serC
<400> 50
   gatatcatgg ctcaaatctt caat
   24
<210> 51
   <211> 27
   <212> DNA
   <213> Artificial Sequence
<220>
   <223> primer for amplification of serA to construct pCL-Prmf-serC
<400> 51
   cccaagcttt taaccgtgac ggcgttc
   27
<210> 52
   <211> 24
   <212> DNA
   <213> Artificial Sequence
<220>
   <223> primer for amplification of serA to construct pCL-Prmf-serA-(RBS)serC
<400> 52
   aagcttacgc aacgtggtga gggg
   24
<210> 53
   <211> 70
   <212> DNA
   <213> Artificial Sequence
<220>
   <223> primer for deletion cassette of phnCDE
<400> 53
   atgcaaacga ttatccgtgt cgagaagctc gccaaaacct tcaatcagca gtgtaggctg
   60
   gagctgcttc
   70
<210> 54
   <211> 70
   <212> DNA
   <213> Artificial Sequence
<220>
   <223> primer for deletion cassette of phnCDE
<400> 54
   tcagataaag tgcttacgca accgttgaga gaggaaatcc agcaggctga catatgaata
   60
   tcctccttag
   70
<210> 55
   <211> 70
   <212> DNA
   <213> Artificial Sequence
<220>
   <223> primer for deletion cassette of phoA
<400> 55
   gtgaaacaaa gcactattgc actggcactc ttaccgttac tgtttacccc gtgtaggctg
   60
   gagctgcttc
   70
<210> 56
   <211> 70
   <212> DNA
   <213> Artificial Sequence
<220>
   <223> primer for deletion cassette of phoA
<400> 56
   ttatttcagc cccagagcgg ctttcatggt gtagaagaga tcggtctggt catatgaata 60
   tcctccttag 70
<210> 57
   <211> 70
   <212> DNA
   <213> Artificial Sequence
<220>
   <223> primer for deletion cassette of aphA
<400> 57
   atgcgcaaga tcacacaggc aatcagtgcc gtttgcttat tgttcgctct gtgtaggctg 60
   gagctgcttc 70
<210> 58
   <211> 70
   <212> DNA
   <213> Artificial Sequence
<220>
   <223> primer for deletion cassette of aphA
<400> 58
   tcagtattct gaattgacga tcacctcttc accaaacgca cccgcttgtg catatgaata 60
   tcctccttag 70
<210> 59
   <211> 70
   <212> DNA
   <213> Artificial Sequence
<220>
   <223> primer for promoter replacement cassette of pntAB
<400> 59
   tatcacattc cttaagccaa ttttaatcct gctcaaatga ccgtctatgc aggtgacact 60
   atagaacgcg 70
<210> 60
   <211> 67
   <212> DNA
   <213> Artificial Sequence
<220>
   <223> primer for promoter replacement cassette of pntAB
<400> 60
   gcaacacggg tttcattggt taaccgttct cttggtatgc caattcgcat ggtctgtttc 60
   ctgtgtg 67
<210> 61
   <211> 28
   <212> DNA
   <213> Artificial Sequence
<220>
   <223> primer for amplification of gdhA to construct pcclBAC-P(native)-gdhA
<400> 61
   cgaagcttgt ctctgctact gataacgg 28
<210> 62
   <211> 26
   <212> DNA
   <213> Artificial Sequence
<220>
   <223> primer for amplification of gdhA to construct pcclBAC-P(native)-gdhA
<400> 62
   cgaagcttgg gagcatcatc cgttaa 26
<210> 63
   <211> 28
   <212> DNA
   <213> Artificial Sequence
<220>
   <223> primer for amplification of ydeD to construct pCL-Prmf-ydeD
<400> 63
   aagatatcat gtcgcgaaaa gatggggt
   28
<210> 64
   <211> 29
   <212> DNA
   <213> Artificial Sequence
<220>
   <223> primer for amplification of ydeD to construct pCL-Prmf-ydeD
<400> 64
   cccaagcttg tgccaatgtc gtggttgtt
   29
<210> 65
   <211> 28
   <212> DNA
   <213> Artificial Sequence
<220>
   <223> primer for amplification of yfiK to construct pCL-Prmf-yfiK
<400> 65
   aagatatcgt gacaccgacc cttttaag
   28
<210> 66
   <211> 29
   <212> DNA
   <213> Artificial Sequence
<220>
   <223> primer for amplification of yfiK to construct pCL-Prmf-yfiK
<400> 66
   cccaagctta ccatccgtgt atccggtta
   29
<210> 67
   <211> 26
   <212> DNA
   <213> Artificial Sequence
<220>
   <223> primer for amplification of rhtB to construct pCL-Prmf-rhtB
<400> 67
   gcgatatcat gaccttagaa tggtgg
   26
<210> 68
   <211> 23
   <212> DNA
   <213> Artificial Sequence
<220>
   <223> primer for amplification of rhtB to construct pCL-Prmf-rhtB
<400> 68
   gctctagatc acgcatgcct cgc
   23
<210> 69
   <211> 32
   <212> DNA
   <213> Artificial Sequence
<220>
   <223> primer for amplification of rhtC to construct pCL-Prmf-rhtC
<400> 69
   gcgatatcat gttgatgtta tttctcaccg tc
   32
<210> 70
   <211> 32
   <212> DNA
   <213> Artificial Sequence
<220>
   <223> primer for amplification of rhtC to construct pCL-Prmf-rhtC
<400> 70
   gctctagatc accgcgaaat aatcaaatga at
   32
<210> 71
   <211> 24
   <212> DNA
   <213> Artificial Sequence
<220>
   <223> primer for amplification of asrB to construct pCL-Prmf-asrB
<400> 71
   atcatgttac tggcaggcgc tatc
   24
<210> 72
   <211> 29
   <212> DNA
   <213> Artificial Sequence
<220>
   <223> primer for amplification of asrB to construct pCL-Prmf-asrB
<400> 72
   gctctagatt acaaagtgaa agagagacg
   29
<210> 73
   <211> 29
   <212> DNA
   <213> Artificial Sequence
<220>
   <223> primer for amplification of livHM to construct pCL-Prmf-livHM
<400> 73
   gcgatatcat gtctgagcag tttttgtat
   29
<210> 74
   <211> 29
   <212> DNA
   <213> Artificial Sequence
<220>
   <223> primer for amplification of livHM to construct pCL-Prmf-livHM
<400> 74
   gctctagatc atgcctgctc tcctttcgc
   29
<210> 75
   <211> 70
   <212> DNA
   <213> Artificial Sequence
<220>
   <223> primer for deletion cassette of gpmA
<400> 75
   gtgtaggctg gagctgcttc tataatgaga attattatca ttaaaagatg atttgaggag 60
   taagtatatg 70
<210> 76
   <211> 70
   <212> DNA
   <213> Artificial Sequence
<220>
   <223> primer for deletion cassette of gpmA
<400> 76
   catatgaata tcctccttag tcggctttct cattttaaac gaatgacgtt tacttcgctt 60
   taccctggtt 70
<210> 77
   <211> 26
   <212> DNA
   <213> Artificial Sequence
<220>
   <223> primer for amplification of gpmI to construct pSG76C-gpmI
<400> 77
   acggatcccg agcggtcgta tatttt 26
<210> 78
   <211> 43
   <212> DNA
   <213> Artificial Sequence
<220>
   <223> primer for amplification of gpmI to construct pSG76C-gpmI involved stop codon
<400> 78
   cagcgcggca aatttttctt actatttttt cagcgaggat tca 43
<210> 79
   <211> 43
   <212> DNA
   <213> Artificial Sequence
<220>
   <223> primer for amplification of gpmI to construct pSG76C-gpmI involved stop codon
<400> 79
   tgaatcctcg ctgaaaaaat agtaagaaaa atttgccgcg ctg
   43
<210> 80
   <211> 28
   <212> DNA
   <213> Artificial Sequence
<220>
   <223> primer for amplification of gpmI to construct pSG76C-gpmI
<400> 80
   cctagagctc aacgactctt ctacgcca
   28
<210> 81
   <211> 70
   <212> DNA
   <213> Artificial Sequence
<220>
   <223> primer for deletion cassette of gpmB
<400> 81
   gcatgttaca ggtataccta gtccgccacg gtgaaacgca gtggaacgcc gtgtaggctg
   60
   gagctgcttc
   70
<210> 82
   <211> 70
   <212> DNA
   <213> Artificial Sequence
<220>
   <223> primer for deletion cassette of gpmB
<400> 82
   tcccctgcgg tttcaacgac ccagccagac gccagccaca ggctttcctg catatgaata
   60
   tcctccttag
   70
<210> 83
   <211> 70
   <212> DNA
   <213> Artificial Sequence
<220>
   <223> primer for deletion cassette of kbl
<400> 83
   aagtttgggt aatatgtgct ggaatttgcc ctgtctggag aatcgcaatg gtgtaggctg 60
   gagctgcttc 70
<210> 84
   <211> 70
   <212> DNA
   <213> Artificial Sequence
<220>
   <223> primer for deletion cassette of kbl
<400> 84
   agtttggata acgctttcat ctcacatcct caggcgataa cgcccagttg catatgaata 60
   tcctccttag 70
<210> 85
   <211> 73
   <212> DNA
   <213> Artificial Sequence
<220>
   <223> primer for deletion cassette of sdaA
<400> 85
   gtgtattagt tcgttactgg aagtccagtc accttgtcag gagtattatc gtggtgtagg 60
   ctggagctgc ttc 73
<210> 86
   <211> 70
   <212> DNA
   <213> Artificial Sequence
<220>
   <223> primer for deletion cassette of sdaA
<400> 86
   atccgttgca gatgggcgag taagaagtat tagtcacact ggactttgat catatgaata 60
   tcctccttag 70
<210> 87
   <211> 70
   <212> DNA
   <213> Artificial Sequence
<220>
   <223> primer for deletion cassette of iclR
<400> 87
   atggtcgcac ccattcccgc gaaacgcggc agaaaacccg ccgttgccac gtgtaggctg 60
   gagctgcttc 70
<210> 88
   <211> 70
   <212> DNA
   <213> Artificial Sequence
<220>
   <223> primer for deletion cassette of iclR
<400> 88
   tcagcgcatt ccaccgtacg ccagcgtcac ttccttcgcc gctttaatca catatgaata 60
   tcctccttag 70
<210> 89
   <211> 21
   <212> DNA
   <213> Artificial Sequence
<220>
   <223> primer for amplification of acs to construct pCL-Prmf-acs
<400> 89
   actatgagcc aaattcacaa a 21
<210> 90
   <211> 27
   <212> DNA
   <213> Artificial Sequence
<220>
   <223> primer for amplification of acs to construct pCL-Prmf-acs
<400> 90
   cccaagcttt tacgatggca tcgcgat
   27
<210> 91
   <211> 24
   <212> DNA
   <213> Artificial Sequence
<220>
   <223> primer for amplification of poxB to construct pCL-Prmf-poxB
<400> 91
   actatgaaac aaacggttgc agct
   24
<210> 92
   <211> 30
   <212> DNA
   <213> Artificial Sequence
<220>
   <223> primer for amplification of poxB to construct pCL-Prmf-poxB
<400> 92
   cccaagcttt taccttagcc agtttgtttt
   30
<210> 93
   <211> 21
   <212> DNA
   <213> Artificial Sequence
<220>
   <223> primer for amplification of ackA-pta to construct pCL-Prmf-ackA-pta
<400> 93
   atcatgtcga gtaagttagt a
   21
<210> 94
   <211> 27
   <212> DNA
   <213> Artificial Sequence
<220>
   <223> primer for amplification of ackA-pta to construct pCL-Prmf-ackA-pta
<400> 94
   cccaagcttt tactgctgct gtgcaga
   27
<210> 95
   <211> 24
   <212> DNA
   <213> Artificial Sequence
<220>
   <223> primer for amplification of aceBA to construct pCL-Prmf-aceBA
<400> 95
   actatgactg aacaggcaac aaca
   24
<210> 96
   <211> 30
   <212> DNA
   <213> Artificial Sequence
<220>
   <223> primer for amplification of aceBA to construct pCL-Prmf-aceBA
<400> 96
   cccaagcttc tacgttcggc aacggctgta
   30
<210> 97
   <211> 24
   <212> DNA
   <213> Artificial Sequence
<220>
   <223> primer for amplification of pckA to construct pCL-Prmf-pckA
<400> 97
   actatgcgcg ttaacaatgg tttg
   24
<210> 98
   <211> 30
   <212> DNA
   <213> Artificial Sequence
<220>
   <223> primer for amplification of pckA to construct pCL-Prmf-pckA
<400> 98
   cccaagctta taaacgggag gcgaaggtgc
   30
<210> 99
   <211> 24
   <212> DNA
   <213> Artificial Sequence
<220>
   <223> primer for amplification of glcB to construct pCL-Prmf-glcB
<400> 99
   atcatgagtc aaaccataac ccag
   24
<210> 100
   <211> 30
   <212> DNA
   <213> Artificial Sequence
<220>
   <223> primer for amplification of glcB to construct pCL-Prmf-glcB
<400> 100
   cccaagcttt taatgacttt ctttttcgcg
   30
<210> 101
   <211> 21
   <212> DNA
   <213> Artificial Sequence
<220>
   <223> primer for amplification of maeB to construct pCL-Prmf-maeB
<400> 101
   atcatggatg accagttaaa a
   21
<210> 102
   <211> 27
   <212> DNA
   <213> Artificial Sequence
<220>
   <223> primer for amplification of maeB to construct pCL-Prmf-maeB
<400> 102
   cccaagcttt tacagcggtt gggtttg
   27
<210> 103
   <211> 21
   <212> DNA
   <213> Artificial Sequence
<220>
   <223> primer for construction of gcl
<400> 103
   atcatggcaa aaatgagagc c
   21
<210> 104
   <211> 23
   <212> DNA
   <213> Artificial Sequence
<220>
   <223> primer for construction of gcl
<400> 104
   gcgcaagctt ttattcatag tgc
   23
<210> 105
   <211> 22
   <212> DNA
   <213> Artificial Sequence
<220>
   <223> primer for construction of glxR-glxK
<400> 105
   gcgcgatatc atgaaactgg ga
   22
<210> 106
   <211> 34
   <212> DNA
   <213> Artificial Sequence
<220>
   <223> primer for construction of glxR-glxK
<400> 106
   ataaactggc ctgatctaga tttgttgaaa aagg
   34
<210> 107
   <211> 34
   <212> DNA
   <213> Artificial Sequence
<220>
   <223> primer for construction of glxR-glxK
<400> 107
   cctttttcaa caaatctaga tcaggccagt ttat
   34
<210> 108
   <211> 32
   <212> DNA
   <213> Artificial Sequence
<220>
   <223> primer for construction of glxR-glxK
<400> 108
   ggcaagctta ttgcggccgc ttagttttta at
   32
<210> 109
   <211> 29
   <212> DNA
   <213> Artificial Sequence
<220>
   <223> forward primer for Ape-OPSS
<400> 109
   gtcatatgat ggctctggct gacatctct
   29
<210> 110
   <211> 29
   <212> DNA
   <213> Artificial Sequence
<220>
   <223> reverse primer for Ape-OPSS
<400> 110
   gtaagctttt aaacagagtc accagcacc
   29
<210> 111
   <211> 29
   <212> DNA
   <213> Artificial Sequence
<220>
   <223> forward primer for Mtb-OPSS
<400> 111
   gtcatatgat gacacgatac gactcgctg
   29
<210> 112
   <211> 29
   <212> DNA
   <213> Artificial Sequence
<220>
   <223> reverse primer for Mtb-OPSS
<400> 112
   gtaagctttc atgcccatag ttgcccttc
   29
<210> 113
   <211> 29
   <212> DNA
   <213> Artificial Sequence
<220>
   <223> forward primer for Msm-OPSS
<400> 113
   ataagctttc atgcccatag ctgcccttc
   29
<210> 114
   <211> 29
   <212> DNA
   <213> Artificial Sequence
<220>
   <223> reverse primer for Msm-OPSS
<400> 114
   ataagctttc attccagcgc gtcctcggc
   29
<210> 115
   <211> 29
   <212> DNA
   <213> Artificial Sequence
<220>
   <223> forward primer for Rjo-OPSS
<400> 115
   gtcatatgat ggcgcggttc gattcgctg
   29
<210> 116
   <211> 31
   <212> DNA
   <213> Artificial Sequence
<220>
   <223> reverse primer for Rjo-OPSS
<400> 116
   tagcggccgc tcatgcccac aactgccctt c
   31
<210> 117
   <211> 29
   <212> DNA
   <213> Artificial Sequence
<220>
   <223> forward primer for Nfa-OPSS
<400> 117
   gtcatatgat ggcacgctac gaatcgctg
   29
<210> 118
   <211> 28
   <212> DNA
   <213> Artificial Sequence
<220>
   <223> reverse primer for Nfa-OPSS
<400> 118
   gtaagctttc aggcccagag ctggcctt
   28
<210> 119
   <211> 29
   <212> DNA
   <213> Artificial Sequence
<220>
   <223> forward primer for Mtb-T
<400> 119
   gtcatatgat gacacgatac gactcgctg
   29
<210> 120
   <211> 29
   <212> DNA
   <213> Artificial Sequence
<220>
   <223> reverse primer for Mtb-T
<400> 120
   gtaagctttc attccagagc ggtctcggc
   29
<210> 121
   <211> 29
   <212> DNA
   <213> Artificial Sequence
<220>
   <223> forward primer of Msm-T
<400> 121
   gtcatatgat gacgcgctac gactccctg
   29
<210> 122
   <211> 29
   <212> DNA
   <213> Artificial Sequence
<220>
   <223> reverse primer of Msm-T
<400> 122
   ataagctttc attccagcgc gtcctcggc
   29
<210> 123
   <211> 21
   <212> DNA
   <213> Artificial Sequence
<220>
   <223> forward primer of pCL-P(CJ1)-Msm-T
<400> 123
   gatatcgcag cagccatcat c
   21
<210> 124
   <211> 28
   <212> DNA
   <213> Artificial Sequence
<220>
   <223> reverse primer of pCL-P(CJ1)-Msm-T
<400> 124
   cccaagcttt cattccagcg cgtcctcg
   28